# EUROPEAN PATENT APPLICATION

(11) **EP 3 932 940 A1**
(43) Date of publication of application: **05.01.2022**
(21) Application number: 20183852.1
(22) Date of filing: 03.07.2020
(51) Int. Cl.: C07K 14/435, C07K 14/47, C07K 14/705, C07K 14/72, G01N 33/68

(54) **POLYPEPTIDES FOR LIGHT INDUCED CA2 RELEASE AND CONSTRUCTS CONTAINING THE SAME**

(71) Applicant: Rheinische Friedrich-Wilhelms-Universität Bonn, 53113 Bonn (DE)
(72) Inventor: SAßE, Phillip, 50825 Köln (DE); JANGSANGTHONG, Wanchana, 50931 Köln (DE); GOTTSCHALK, Alexander, 61118 Bad Vilbel (DE); LEHNART, Stephan, 37075 Göttingen (DE); DURA, Miroslav, 37075 Göttingen (DE)
(74) Representative: Gramm, Lins & Partner Patent- und Rechtsanwälte PartGmbB

(57) **Abstract**

The present invention relates in a first aspect to a polypeptide comprising a channelrhodopsin moiety, a linker moiety and a moiety of a Ca²⁺ sensitive protein. In particular, the polypeptide is a polypeptide composed of the channelrhodopsin-2 of *Chlamydomonas reinhardtii*, a linker and RyR2 whereby the linker is designed to establish a distance of from 100 to 200 Angstrom between the channelrhodopsin moiety and the RyR2 moiety when present in a cellular membrane of eukaryotes. In addition, nucleic acid molecules encoding the polypeptide according to the present invention as well as vector or vector systems containing said nucleic acid molecule. Further, nucleic acid constructs comprising the nucleic acid encoding the polypeptide of the channelrhodopsin as well as the linker is provided. In addition, isolated cells, cell lines or host cells containing the vector or vector system according to the present invention or expressing the polypeptide according to the present invention are provided. Further, the *C. elegans* strains containing the vector according to the present invention or expressing the polypeptide according to the present invention are provided. Method for determining the activity of potential principal actives on Ca²⁺ sensitive proteins are provided based on determining the activity of these molecules on said Ca²⁺ sensitive protein.

SEQUENCE LISTING

<110>  Rheinische Friedrich-Wilhelms-Universitaet Bonn

<120>  Polypeptides fo light induced Ca2+ release and constructs
       containing the same

<130>  4464-001 EP-1

<160>  10

<170>  PatentIn version 3.5

<210>  1
<211>  4967
<212>  PRT
<213>  Homo Sapiens

<400>  1

Met Ala Asp Gly Gly Glu Gly Glu Asp Glu Ile Gln Phe Leu Arg Thr
1               5                   10                  15

Asp Asp Glu Val Val Leu Gln Cys Thr Ala Thr Ile His Lys Glu Gln
            20                  25                  30

Gln Lys Leu Cys Leu Ala Ala Glu Gly Phe Gly Asn Arg Leu Cys Phe
        35                  40                  45

Leu Glu Ser Thr Ser Asn Ser Lys Asn Val Pro Pro Asp Leu Ser Ile
    50                  55                  60

Cys Thr Phe Val Leu Glu Gln Ser Leu Ser Val Arg Ala Leu Gln Glu
65                  70                  75                  80

Met Leu Ala Asn Thr Val Glu Lys Ser Glu Gly Gln Val Asp Val Glu
                85                  90                  95

Lys Trp Lys Phe Met Met Lys Thr Ala Gln Gly Gly Gly His Arg Thr
            100                 105                 110

Leu Leu Tyr Gly His Ala Ile Leu Leu Arg His Ser Tyr Ser Gly Met
        115                 120                 125

Tyr Leu Cys Cys Leu Ser Thr Ser Arg Ser Ser Thr Asp Lys Leu Ala
    130                 135                 140

Phe Asp Val Gly Leu Gln Glu Asp Thr Thr Gly Glu Ala Cys Trp Trp
145                 150                 155                 160

Thr Ile His Pro Ala Ser Lys Gln Arg Ser Glu Gly Glu Lys Val Arg
                165                 170                 175

Val Gly Asp Asp Leu Ile Leu Val Ser Val Ser Ser Glu Arg Tyr Leu
        180                 185                 190

His Leu Ser Tyr Gly Asn Gly Ser Leu His Val Asp Ala Ala Phe Gln
    195                 200                 205

Gln Thr Leu Trp Ser Val Ala Pro Ile Ser Ser Gly Ser Glu Ala Ala
    210                 215                 220

Gln Gly Tyr Leu Ile Gly Gly Asp Val Leu Arg Leu Leu His Gly His
225                 230                 235                 240

Met Asp Glu Cys Leu Thr Val Pro Ser Gly Glu His Gly Glu Glu Gln
                245                 250                 255

Arg Arg Thr Val His Tyr Glu Gly Gly Ala Val Ser Val His Ala Arg
            260                 265                 270

Ser Leu Trp Arg Leu Glu Thr Leu Arg Val Ala Trp Ser Gly Ser His
        275                 280                 285

Ile Arg Trp Gly Gln Pro Phe Arg Leu Arg His Val Thr Thr Gly Lys
    290                 295                 300

Tyr Leu Ser Leu Met Glu Asp Lys Asn Leu Leu Leu Met Asp Lys Glu
305                 310                 315                 320

Lys Ala Asp Val Lys Ser Thr Ala Phe Thr Phe Arg Ser Ser Lys Glu
                325                 330                 335

Lys Leu Asp Val Gly Val Arg Lys Glu Val Asp Gly Met Gly Thr Ser
            340                 345                 350

Glu Ile Lys Tyr Gly Asp Ser Val Cys Tyr Ile Gln His Val Asp Thr
        355                 360                 365

Gly Leu Trp Leu Thr Tyr Gln Ser Val Asp Val Lys Ser Val Arg Met
    370                 375                 380

Gly Ser Ile Gln Arg Lys Ala Ile Met His His Glu Gly His Met Asp
385                 390                 395                 400

Asp Gly Ile Ser Leu Ser Arg Ser Gln His Glu Glu Ser Arg Thr Ala
                405                 410                 415

Arg Val Ile Arg Ser Thr Val Phe Leu Phe Asn Arg Phe Ile Arg Gly
            420                 425                 430

Leu Asp Ala Leu Ser Lys Lys Ala Lys Ala Ser Thr Val Asp Leu Pro
        435                 440                 445

Ile Glu Ser Val Ser Leu Ser Leu Gln Asp Leu Ile Gly Tyr Phe His
    450                 455                 460

Pro Pro Asp Glu His Leu Glu His Glu Asp Lys Gln Asn Arg Leu Arg
465                 470                 475                 480

Ala Leu Lys Asn Arg Gln Asn Leu Phe Gln Glu Glu Gly Met Ile Asn
                485                 490                 495

Leu Val Leu Glu Cys Ile Asp Arg Leu His Val Tyr Ser Ser Ala Ala
            500                 505                 510

His Phe Ala Asp Val Ala Gly Arg Glu Ala Gly Glu Ser Trp Lys Ser
        515                 520                 525

Ile Leu Asn Ser Leu Tyr Glu Leu Leu Ala Ala Leu Ile Arg Gly Asn
    530                 535                 540

Arg Lys Asn Cys Ala Gln Phe Ser Gly Ser Leu Asp Trp Leu Ile Ser
545                 550                 555                 560

Arg Leu Glu Arg Leu Glu Ala Ser Ser Gly Ile Leu Glu Val Leu His
                565                 570                 575

Cys Val Leu Val Glu Ser Pro Glu Ala Leu Asn Ile Ile Lys Glu Gly
            580                 585                 590

His Ile Lys Ser Ile Ile Ser Leu Leu Asp Lys His Gly Arg Asn His
        595                 600                 605

Lys Val Leu Asp Val Leu Cys Ser Leu Cys Val Cys His Gly Val Ala
    610                 615                 620

Val Arg Ser Asn Gln His Leu Ile Cys Asp Asn Leu Leu Pro Gly Arg
625                 630                 635                 640

Asp Leu Leu Leu Gln Thr Arg Leu Val Asn His Val Ser Ser Met Arg
                645                 650                 655

Pro Asn Ile Phe Leu Gly Val Ser Glu Gly Ser Ala Gln Tyr Lys Lys
            660                 665                 670

Trp Tyr Tyr Glu Leu Met Val Asp His Thr Glu Pro Phe Val Thr Ala

        675                 680                 685

Glu Ala Thr His Leu Arg Val Gly Trp Ala Ser Thr Glu Gly Tyr Ser
    690                 695                 700

Pro Tyr Pro Gly Gly Gly Glu Glu Trp Gly Gly Asn Gly Val Gly Asp
705                 710                 715                 720

Asp Leu Phe Ser Tyr Gly Phe Asp Gly Leu His Leu Trp Ser Gly Cys
                725                 730                 735

Ile Ala Arg Thr Val Ser Ser Pro Asn Gln His Leu Leu Arg Thr Asp
            740                 745                 750

Asp Val Ile Ser Cys Cys Leu Asp Leu Ser Ala Pro Ser Ile Ser Phe
        755                 760                 765

Arg Ile Asn Gly Gln Pro Val Gln Gly Met Phe Glu Asn Phe Asn Ile
    770                 775                 780

Asp Gly Leu Phe Phe Pro Val Val Ser Phe Ser Ala Gly Ile Lys Val
785                 790                 795                 800

Arg Phe Leu Leu Gly Gly Arg His Gly Glu Phe Lys Phe Leu Pro Pro
                805                 810                 815

Pro Gly Tyr Ala Pro Cys Tyr Glu Ala Val Leu Pro Lys Glu Lys Leu
            820                 825                 830

Lys Val Glu His Ser Arg Glu Tyr Lys Gln Glu Arg Thr Tyr Thr Arg
        835                 840                 845

Asp Leu Leu Gly Pro Thr Val Ser Leu Thr Gln Ala Ala Phe Thr Pro
    850                 855                 860

Ile Pro Val Asp Thr Ser Gln Ile Val Leu Pro Pro His Leu Glu Arg
865                 870                 875                 880

Ile Arg Glu Lys Leu Ala Glu Asn Ile His Glu Leu Trp Val Met Asn
                885                 890                 895

Lys Ile Glu Leu Gly Trp Gln Tyr Gly Pro Val Arg Asp Asp Asn Lys
            900                 905                 910

Arg Gln His Pro Cys Leu Val Glu Phe Ser Lys Leu Pro Glu Gln Glu
        915                 920                 925

Arg Asn Tyr Asn Leu Gln Met Ser Leu Glu Thr Leu Lys Thr Leu Leu
    930                 935                 940

Ala Leu Gly Cys His Val Gly Ile Ser Asp Glu His Ala Glu Asp Lys
945                 950                 955                 960

Val Lys Lys Met Lys Leu Pro Lys Asn Tyr Gln Leu Thr Ser Gly Tyr
                965                 970                 975

Lys Pro Ala Pro Met Asp Leu Ser Phe Ile Lys Leu Thr Pro Ser Gln
            980                 985                 990

Glu Ala Met Val Asp Lys Leu Ala  Glu Asn Ala His Asn  Val Trp Ala
      995                 1000                 1005

Arg Asp  Arg Ile Arg Gln Gly  Trp Thr Tyr Gly Ile  Gln Gln Asp
    1010                 1015                 1020

Val Lys  Asn Arg Arg Asn Pro  Arg Leu Val Pro Tyr  Thr Leu Leu
    1025                 1030                 1035

Asp Asp  Arg Thr Lys Lys Ser  Asn Lys Asp Ser Leu  Arg Glu Ala
    1040                 1045                 1050

Val Arg  Thr Leu Leu Gly Tyr  Gly Tyr Asn Leu Glu  Ala Pro Asp
    1055                 1060                 1065

Gln Asp  His Ala Ala Arg Ala  Glu Val Cys Ser Gly  Thr Gly Glu
    1070                 1075                 1080

Arg Phe  Arg Ile Phe Arg Ala  Glu Lys Thr Tyr Ala  Val Lys Ala
    1085                 1090                 1095

Gly Arg  Trp Tyr Phe Glu Phe  Glu Thr Val Thr Ala  Gly Asp Met
    1100                 1105                 1110

Arg Val  Gly Trp Ser Arg Pro  Gly Cys Gln Pro Asp  Gln Glu Leu
    1115                 1120                 1125

Gly Ser  Asp Glu Arg Ala Phe  Ala Phe Asp Gly Phe  Lys Ala Gln
    1130                 1135                 1140

Arg Trp  His Gln Gly Asn Glu  His Tyr Gly Arg Ser  Trp Gln Ala
    1145                 1150                 1155

Gly Asp  Val Val Gly Cys Met  Val Asp Met Asn Glu  His Thr Met
    1160                 1165                 1170

Met Phe Thr Leu Asn Gly Glu Ile Leu Leu Asp Asp Ser Gly Ser
    1175                1180                1185

Glu Leu Ala Phe Lys Asp Phe Asp Val Gly Asp Gly Phe Ile Pro
    1190                1195                1200

Val Cys Ser Leu Gly Val Ala Gln Val Gly Arg Met Asn Phe Gly
    1205                1210                1215

Lys Asp Val Ser Thr Leu Lys Tyr Phe Thr Ile Cys Gly Leu Gln
    1220                1225                1230

Glu Gly Tyr Glu Pro Phe Ala Val Asn Thr Asn Arg Asp Ile Thr
    1235                1240                1245

Met Trp Leu Ser Lys Arg Leu Pro Gln Phe Leu Gln Val Pro Ser
    1250                1255                1260

Asn His Glu His Ile Glu Val Thr Arg Ile Asp Gly Thr Ile Asp
    1265                1270                1275

Ser Ser Pro Cys Leu Lys Val Thr Gln Lys Ser Phe Gly Ser Gln
    1280                1285                1290

Asn Ser Asn Thr Asp Ile Met Phe Tyr Arg Leu Ser Met Pro Ile
    1295                1300                1305

Glu Cys Ala Glu Val Phe Ser Lys Thr Val Ala Gly Gly Leu Pro
    1310                1315                1320

Gly Ala Gly Leu Phe Gly Pro Lys Asn Asp Leu Glu Asp Tyr Asp
    1325                1330                1335

Ala Asp Ser Asp Phe Glu Val Leu Met Lys Thr Ala His Gly His
    1340                1345                1350

Leu Val Pro Asp Arg Val Asp Lys Asp Lys Glu Ala Thr Lys Pro
    1355                1360                1365

Glu Phe Asn Asn His Lys Asp Tyr Ala Gln Glu Lys Pro Ser Arg
    1370                1375                1380

Leu Lys Gln Arg Phe Leu Leu Arg Arg Thr Lys Pro Asp Tyr Ser
    1385                1390                1395

Thr Ser His Ser Ala Arg Leu Thr Glu Asp Val Leu Ala Asp Asp
    1400                1405                1410

Arg Asp Asp Tyr Asp Phe Leu Met Gln Thr Ser Thr Tyr Tyr Tyr
    1415                1420                1425

Ser Val Arg Ile Phe Pro Gly Gln Glu Pro Ala Asn Val Trp Val
    1430                1435                1440

Gly Trp Ile Thr Ser Asp Phe His Gln Tyr Asp Thr Gly Phe Asp
    1445                1450                1455

Leu Asp Arg Val Arg Thr Val Thr Val Thr Leu Gly Asp Glu Lys
    1460                1465                1470

Gly Lys Val His Glu Ser Ile Lys Arg Ser Asn Cys Tyr Met Val
    1475                1480                1485

Cys Ala Gly Glu Ser Met Ser Pro Gly Gln Gly Arg Asn Asn Asn
    1490                1495                1500

Gly Leu Glu Ile Gly Cys Val Val Asp Ala Ala Ser Gly Leu Leu
    1505                1510                1515

Thr Phe Ile Ala Asn Gly Lys Glu Leu Ser Thr Tyr Tyr Gln Val
    1520                1525                1530

Glu Pro Ser Thr Lys Leu Phe Pro Ala Val Phe Ala Gln Ala Thr
    1535                1540                1545

Ser Pro Asn Val Phe Gln Phe Glu Leu Gly Arg Ile Lys Asn Val
    1550                1555                1560

Met Pro Leu Ser Ala Gly Leu Phe Lys Ser Glu His Lys Asn Pro
    1565                1570                1575

Val Pro Gln Cys Pro Pro Arg Leu His Val Gln Phe Leu Ser His
    1580                1585                1590

Val Leu Trp Ser Arg Met Pro Asn Gln Phe Leu Lys Val Asp Val
    1595                1600                1605

Ser Arg Ile Ser Glu Arg Gln Gly Trp Leu Val Gln Cys Leu Asp
    1610                1615                1620

Pro Leu Gln Phe Met Ser Leu His Ile Pro Glu Glu Asn Arg Ser
    1625                1630                1635

Val Asp Ile Leu Glu Leu Thr Glu Gln Glu Glu Leu Leu Lys Phe

1640                1645                1650

His Tyr His Thr Leu Arg Leu Tyr Ser Ala Val Cys Ala Leu Gly
    1655                1660                1665

Asn His Arg Val Ala His Ala Leu Cys Ser His Val Asp Glu Pro
    1670                1675                1680

Gln Leu Leu Tyr Ala Ile Glu Asn Lys Tyr Met Pro Gly Leu Leu
    1685                1690                1695

Arg Ala Gly Tyr Tyr Asp Leu Leu Ile Asp Ile His Leu Ser Ser
    1700                1705                1710

Tyr Ala Thr Ala Arg Leu Met Met Asn Asn Glu Tyr Ile Val Pro
    1715                1720                1725

Met Thr Glu Glu Thr Lys Ser Ile Thr Leu Phe Pro Asp Glu Asn
    1730                1735                1740

Lys Lys His Gly Leu Pro Gly Ile Gly Leu Ser Thr Ser Leu Arg
    1745                1750                1755

Pro Arg Met Gln Phe Ser Ser Pro Ser Phe Val Ser Ile Ser Asn
    1760                1765                1770

Glu Cys Tyr Gln Tyr Ser Pro Glu Phe Pro Leu Asp Ile Leu Lys
    1775                1780                1785

Ser Lys Thr Ile Gln Met Leu Thr Glu Ala Val Lys Glu Gly Ser
    1790                1795                1800

Leu His Ala Arg Asp Pro Val Gly Gly Thr Thr Glu Phe Leu Phe
    1805                1810                1815

Val Pro Leu Ile Lys Leu Phe Tyr Thr Leu Leu Ile Met Gly Ile
    1820                1825                1830

Phe His Asn Glu Asp Leu Lys His Ile Leu Gln Leu Ile Glu Pro
    1835                1840                1845

Ser Val Phe Lys Glu Ala Ala Thr Pro Glu Glu Glu Ser Asp Thr
    1850                1855                1860

Leu Glu Lys Glu Leu Ser Val Asp Asp Ala Lys Leu Gln Gly Ala
    1865                1870                1875

Gly Glu Glu Glu Ala Lys Gly Gly Lys Arg Pro Lys Glu Gly Leu
    1880                1885                1890

Leu Gln Met Lys Leu Pro Glu Pro Val Lys Leu Gln Met Cys Leu
    1895                1900                1905

Leu Leu Gln Tyr Leu Cys Asp Cys Gln Val Arg His Arg Ile Glu
    1910                1915                1920

Ala Ile Val Ala Phe Ser Asp Asp Phe Val Ala Lys Leu Gln Asp
    1925                1930                1935

Asn Gln Arg Phe Arg Tyr Asn Glu Val Met Gln Ala Leu Asn Met
    1940                1945                1950

Ser Ala Ala Leu Thr Ala Arg Lys Thr Lys Glu Phe Arg Ser Pro
    1955                1960                1965

Pro Gln Glu Gln Ile Asn Met Leu Leu Asn Phe Lys Asp Asp Lys
    1970                1975                1980

Ser Glu Cys Pro Cys Pro Glu Glu Ile Arg Asp Gln Leu Leu Asp
    1985                1990                1995

Phe His Glu Asp Leu Met Thr His Cys Gly Ile Glu Leu Asp Glu
    2000                2005                2010

Asp Gly Ser Leu Asp Gly Asn Ser Asp Leu Thr Ile Arg Gly Arg
    2015                2020                2025

Leu Leu Ser Leu Val Glu Lys Val Thr Tyr Leu Lys Lys Lys Gln
    2030                2035                2040

Ala Glu Lys Pro Val Glu Ser Asp Ser Lys Lys Ser Ser Thr Leu
    2045                2050                2055

Gln Gln Leu Ile Ser Glu Thr Met Val Arg Trp Ala Gln Glu Ser
    2060                2065                2070

Val Ile Glu Asp Pro Glu Leu Val Arg Ala Met Phe Val Leu Leu
    2075                2080                2085

His Arg Gln Tyr Asp Gly Ile Gly Gly Leu Val Arg Ala Leu Pro
    2090                2095                2100

Lys Thr Tyr Thr Ile Asn Gly Val Ser Val Glu Asp Thr Ile Asn
    2105                2110                2115

Leu Leu Ala Ser Leu Gly Gln Ile Arg Ser Leu Leu Ser Val Arg
    2120                2125                2130

Met Gly Lys Glu Glu Glu Lys Leu Met Ile Arg Gly Leu Gly Asp
    2135                2140                2145

Ile Met Asn Asn Lys Val Phe Tyr Gln His Pro Asn Leu Met Arg
    2150                2155                2160

Ala Leu Gly Met His Glu Thr Val Met Glu Val Met Val Asn Val
    2165                2170                2175

Leu Gly Gly Gly Glu Ser Lys Glu Ile Thr Phe Pro Lys Met Val
    2180                2185                2190

Ala Asn Cys Cys Arg Phe Leu Cys Tyr Phe Cys Arg Ile Ser Arg
    2195                2200                2205

Gln Asn Gln Lys Ala Met Phe Asp His Leu Ser Tyr Leu Leu Glu
    2210                2215                2220

Asn Ser Ser Val Gly Leu Ala Ser Pro Ala Met Arg Gly Ser Thr
    2225                2230                2235

Pro Leu Asp Val Ala Ala Ala Ser Val Met Asp Asn Asn Glu Leu
    2240                2245                2250

Ala Leu Ala Leu Arg Glu Pro Asp Leu Glu Lys Val Val Arg Tyr
    2255                2260                2265

Leu Ala Gly Cys Gly Leu Gln Ser Cys Gln Met Leu Val Ser Lys
    2270                2275                2280

Gly Tyr Pro Asp Ile Gly Trp Asn Pro Val Glu Gly Glu Arg Tyr
    2285                2290                2295

Leu Asp Phe Leu Arg Phe Ala Val Phe Cys Asn Gly Glu Ser Val
    2300                2305                2310

Glu Glu Asn Ala Asn Val Val Val Arg Leu Leu Ile Arg Arg Pro
    2315                2320                2325

Glu Cys Phe Gly Pro Ala Leu Arg Gly Glu Gly Gly Asn Gly Leu
    2330                2335                2340

Leu Ala Ala Met Glu Glu Ala Ile Lys Ile Ala Glu Asp Pro Ser
    2345                2350                2355

Arg Asp Gly Pro Ser Pro Asn Ser Gly Ser Ser Lys Thr Leu Asp
    2360                2365                2370

Thr Glu Glu Glu Glu Asp Asp Thr Ile His Met Gly Asn Ala Ile
    2375                2380                2385

Met Thr Phe Tyr Ser Ala Leu Ile Asp Leu Leu Gly Arg Cys Ala
    2390                2395                2400

Pro Glu Met His Leu Ile His Ala Gly Lys Gly Glu Ala Ile Arg
    2405                2410                2415

Ile Arg Ser Ile Leu Arg Ser Leu Ile Pro Leu Gly Asp Leu Val
    2420                2425                2430

Gly Val Ile Ser Ile Ala Phe Gln Met Pro Thr Ile Ala Lys Asp
    2435                2440                2445

Gly Asn Val Val Glu Pro Asp Met Ser Ala Gly Phe Cys Pro Asp
    2450                2455                2460

His Lys Ala Ala Met Val Leu Phe Leu Asp Arg Val Tyr Gly Ile
    2465                2470                2475

Glu Val Gln Asp Phe Leu Leu His Leu Leu Glu Val Gly Phe Leu
    2480                2485                2490

Pro Asp Leu Arg Ala Ala Ala Ser Leu Asp Thr Ala Ala Leu Ser
    2495                2500                2505

Ala Thr Asp Met Ala Leu Ala Leu Asn Arg Tyr Leu Cys Thr Ala
    2510                2515                2520

Val Leu Pro Leu Leu Thr Arg Cys Ala Pro Leu Phe Ala Gly Thr
    2525                2530                2535

Glu His His Ala Ser Leu Ile Asp Ser Leu Leu His Thr Val Tyr
    2540                2545                2550

Arg Leu Ser Lys Gly Cys Ser Leu Thr Lys Ala Gln Arg Asp Ser
    2555                2560                2565

Ile Glu Val Cys Leu Leu Ser Ile Cys Gly Gln Leu Arg Pro Ser
    2570                2575                2580

Met Met Gln His Leu Leu Arg Arg Leu Val Phe Asp Val Pro Leu

2585                2590                2595

Leu Asn Glu His Ala Lys Met Pro Leu Lys Leu Leu Thr Asn His
    2600                2605                2610

Tyr Glu Arg Cys Trp Lys Tyr Tyr Cys Leu Pro Gly Gly Trp Gly
    2615                2620                2625

Asn Phe Gly Ala Ala Ser Glu Glu Glu Leu His Leu Ser Arg Lys
    2630                2635                2640

Leu Phe Trp Gly Ile Phe Asp Ala Leu Ser Gln Lys Lys Tyr Glu
    2645                2650                2655

Gln Glu Leu Phe Lys Leu Ala Leu Pro Cys Leu Ser Ala Val Ala
    2660                2665                2670

Gly Ala Leu Pro Pro Asp Tyr Met Glu Ser Asn Tyr Val Ser Met
    2675                2680                2685

Met Glu Lys Gln Ser Ser Met Asp Ser Glu Gly Asn Phe Asn Pro
    2690                2695                2700

Gln Pro Val Asp Thr Ser Asn Ile Thr Ile Pro Glu Lys Leu Glu
    2705                2710                2715

Tyr Phe Ile Asn Lys Tyr Ala Glu His Ser His Asp Lys Trp Ser
    2720                2725                2730

Met Asp Lys Leu Ala Asn Gly Trp Ile Tyr Gly Glu Ile Tyr Ser
    2735                2740                2745

Asp Ser Ser Lys Val Gln Pro Leu Met Lys Pro Tyr Lys Leu Leu
    2750                2755                2760

Ser Glu Lys Glu Lys Glu Ile Tyr Arg Trp Pro Ile Lys Glu Ser
    2765                2770                2775

Leu Lys Thr Met Leu Ala Trp Gly Trp Arg Ile Glu Arg Thr Arg
    2780                2785                2790

Glu Gly Asp Ser Met Ala Leu Tyr Asn Arg Thr Arg Arg Ile Ser
    2795                2800                2805

Gln Thr Ser Gln Val Ser Val Asp Ala Ala His Gly Tyr Ser Pro
    2810                2815                2820

Arg Ala Ile Asp Met Ser Asn Val Thr Leu Ser Arg Asp Leu His
    2825                2830                2835

Ala Met Ala Glu Met Met Ala Glu Asn Tyr His Asn Ile Trp Ala
    2840                2845                2850

Lys Lys Lys Lys Met Glu Leu Glu Ser Lys Gly Gly Gly Asn His
    2855                2860                2865

Pro Leu Leu Val Pro Tyr Asp Thr Leu Thr Ala Lys Glu Lys Ala
    2870                2875                2880

Lys Asp Arg Glu Lys Ala Gln Asp Ile Leu Lys Phe Leu Gln Ile
    2885                2890                2895

Asn Gly Tyr Ala Val Ser Arg Gly Phe Lys Asp Leu Glu Leu Asp
    2900                2905                2910

Thr Pro Ser Ile Glu Lys Arg Phe Ala Tyr Ser Phe Leu Gln Gln
    2915                2920                2925

Leu Ile Arg Tyr Val Asp Glu Ala His Gln Tyr Ile Leu Glu Phe
    2930                2935                2940

Asp Gly Gly Ser Arg Gly Lys Gly Glu His Phe Pro Tyr Glu Gln
    2945                2950                2955

Glu Ile Lys Phe Phe Ala Lys Val Val Leu Pro Leu Ile Asp Gln
    2960                2965                2970

Tyr Phe Lys Asn His Arg Leu Tyr Phe Leu Ser Ala Ala Ser Arg
    2975                2980                2985

Pro Leu Cys Ser Gly Gly His Ala Ser Asn Lys Glu Lys Glu Met
    2990                2995                3000

Val Thr Ser Leu Phe Cys Lys Leu Gly Val Leu Val Arg His Arg
    3005                3010                3015

Ile Ser Leu Phe Gly Asn Asp Ala Thr Ser Ile Val Asn Cys Leu
    3020                3025                3030

His Ile Leu Gly Gln Thr Leu Asp Ala Arg Thr Val Met Lys Thr
    3035                3040                3045

Gly Leu Glu Ser Val Lys Ser Ala Leu Arg Ala Phe Leu Asp Asn
    3050                3055                3060

Ala Ala Glu Asp Leu Glu Lys Thr Met Glu Asn Leu Lys Gln Gly
    3065                3070                3075

Gln Phe Thr His Thr Arg Asn Gln Pro Lys Gly Val Thr Gln Ile
    3080                3085                3090

Ile Asn Tyr Thr Thr Val Ala Leu Leu Pro Met Leu Ser Ser Leu
    3095                3100                3105

Phe Glu His Ile Gly Gln His Gln Phe Gly Glu Asp Leu Ile Leu
    3110                3115                3120

Glu Asp Val Gln Val Ser Cys Tyr Arg Ile Leu Thr Ser Leu Tyr
    3125                3130                3135

Ala Leu Gly Thr Ser Lys Ser Ile Tyr Val Glu Arg Gln Arg Ser
    3140                3145                3150

Ala Leu Gly Glu Cys Leu Ala Ala Phe Ala Gly Ala Phe Pro Val
    3155                3160                3165

Ala Phe Leu Glu Thr His Leu Asp Lys His Asn Ile Tyr Ser Ile
    3170                3175                3180

Tyr Asn Thr Lys Ser Ser Arg Glu Arg Ala Ala Leu Ser Leu Pro
    3185                3190                3195

Thr Asn Val Glu Asp Val Cys Pro Asn Ile Pro Ser Leu Glu Lys
    3200                3205                3210

Leu Met Glu Glu Ile Val Glu Leu Ala Glu Ser Gly Ile Arg Tyr
    3215                3220                3225

Thr Gln Met Pro His Val Met Glu Val Ile Leu Pro Met Leu Cys
    3230                3235                3240

Ser Tyr Met Ser Arg Trp Trp Glu His Gly Pro Glu Asn Asn Pro
    3245                3250                3255

Glu Arg Ala Glu Met Cys Cys Thr Ala Leu Asn Ser Glu His Met
    3260                3265                3270

Asn Thr Leu Leu Gly Asn Ile Leu Lys Ile Ile Tyr Asn Asn Leu
    3275                3280                3285

Gly Ile Asp Glu Gly Ala Trp Met Lys Arg Leu Ala Val Phe Ser
    3290                3295                3300

Gln Pro Ile Ile Asn Lys Val Lys Pro Gln Leu Leu Lys Thr His
    3305                3310                3315

Phe Leu Pro Leu Met Glu Lys Leu Lys Lys Lys Ala Ala Thr Val
    3320                3325                3330

Val Ser Glu Glu Asp His Leu Lys Ala Glu Ala Arg Gly Asp Met
    3335                3340                3345

Ser Glu Ala Glu Leu Leu Ile Leu Asp Glu Phe Thr Thr Leu Ala
    3350                3355                3360

Arg Asp Leu Tyr Ala Phe Tyr Pro Leu Leu Ile Arg Phe Val Asp
    3365                3370                3375

Tyr Asn Arg Ala Lys Trp Leu Lys Glu Pro Asn Pro Glu Ala Glu
    3380                3385                3390

Glu Leu Phe Arg Met Val Ala Glu Val Phe Ile Tyr Trp Ser Lys
    3395                3400                3405

Ser His Asn Phe Lys Arg Glu Glu Gln Asn Phe Val Val Gln Asn
    3410                3415                3420

Glu Ile Asn Asn Met Ser Phe Leu Ile Thr Asp Thr Lys Ser Lys
    3425                3430                3435

Met Ser Lys Ala Ala Val Ser Asp Gln Glu Arg Lys Lys Met Lys
    3440                3445                3450

Arg Lys Gly Asp Arg Tyr Ser Met Gln Thr Ser Leu Ile Val Ala
    3455                3460                3465

Ala Leu Lys Arg Leu Leu Pro Ile Gly Leu Asn Ile Cys Ala Pro
    3470                3475                3480

Gly Asp Gln Glu Leu Ile Ala Leu Ala Lys Asn Arg Phe Ser Leu
    3485                3490                3495

Lys Asp Thr Glu Asp Glu Val Arg Asp Ile Ile Arg Ser Asn Ile
    3500                3505                3510

His Leu Gln Gly Lys Leu Glu Asp Pro Ala Ile Arg Trp Gln Met
    3515                3520                3525

Ala Leu Tyr Lys Asp Leu Pro Asn Arg Thr Asp Asp Thr Ser Asp

3530                3535                3540

Pro Glu Lys Thr Val Glu Arg Val Leu Asp Ile Ala Asn Val Leu
    3545                3550                3555

Phe His Leu Glu Gln Lys Ser Lys Arg Val Gly Arg Arg His Tyr
    3560                3565                3570

Cys Leu Val Glu His Pro Gln Arg Ser Lys Lys Ala Val Trp His
    3575                3580                3585

Lys Leu Leu Ser Lys Gln Arg Lys Arg Ala Val Val Ala Cys Phe
    3590                3595                3600

Arg Met Ala Pro Leu Tyr Asn Leu Pro Arg His Arg Ala Val Asn
    3605                3610                3615

Leu Phe Leu Gln Gly Tyr Glu Lys Ser Trp Ile Glu Thr Glu Glu
    3620                3625                3630

His Tyr Phe Glu Asp Lys Leu Ile Glu Asp Leu Ala Lys Pro Gly
    3635                3640                3645

Ala Glu Pro Pro Glu Glu Asp Glu Gly Thr Lys Arg Val Asp Pro
    3650                3655                3660

Leu His Gln Leu Ile Leu Leu Phe Ser Arg Thr Ala Leu Thr Glu
    3665                3670                3675

Lys Cys Lys Leu Glu Glu Asp Phe Leu Tyr Met Ala Tyr Ala Asp
    3680                3685                3690

Ile Met Ala Lys Ser Cys His Asp Glu Glu Asp Asp Asp Gly Glu
    3695                3700                3705

Glu Glu Val Lys Ser Phe Glu Glu Lys Glu Met Glu Lys Gln Lys
    3710                3715                3720

Leu Leu Tyr Gln Gln Ala Arg Leu His Asp Arg Gly Ala Ala Glu
    3725                3730                3735

Met Val Leu Gln Thr Ile Ser Ala Ser Lys Gly Glu Thr Gly Pro
    3740                3745                3750

Met Val Ala Ala Thr Leu Lys Leu Gly Ile Ala Ile Leu Asn Gly
    3755                3760                3765

Gly Asn Ser Thr Val Gln Gln Lys Met Leu Asp Tyr Leu Lys Glu
    3770                3775                3780

Lys Lys Asp Val Gly Phe Phe Gln Ser Leu Ala Gly Leu Met Gln
    3785                3790                3795

Ser Cys Ser Val Leu Asp Leu Asn Ala Phe Glu Arg Gln Asn Lys
    3800                3805                3810

Ala Glu Gly Leu Gly Met Val Thr Glu Glu Gly Ser Gly Glu Lys
    3815                3820                3825

Val Leu Gln Asp Asp Glu Phe Thr Cys Asp Leu Phe Arg Phe Leu
    3830                3835                3840

Gln Leu Leu Cys Glu Gly His Asn Ser Asp Phe Gln Asn Tyr Leu
    3845                3850                3855

Arg Thr Gln Thr Gly Asn Asn Thr Thr Val Asn Ile Ile Ile Ser
    3860                3865                3870

Thr Val Asp Tyr Leu Leu Arg Val Gln Glu Ser Ile Ser Asp Phe
    3875                3880                3885

Tyr Trp Tyr Tyr Ser Gly Lys Asp Val Ile Asp Glu Gln Gly Gln
    3890                3895                3900

Arg Asn Phe Ser Lys Ala Ile Gln Val Ala Lys Gln Val Phe Asn
    3905                3910                3915

Thr Leu Thr Glu Tyr Ile Gln Gly Pro Cys Thr Gly Asn Gln Gln
    3920                3925                3930

Ser Leu Ala His Ser Arg Leu Trp Asp Ala Val Val Gly Phe Leu
    3935                3940                3945

His Val Phe Ala His Met Gln Met Lys Leu Ser Gln Asp Ser Ser
    3950                3955                3960

Gln Ile Glu Leu Leu Lys Glu Leu Met Asp Leu Gln Lys Asp Met
    3965                3970                3975

Val Val Met Leu Leu Ser Met Leu Glu Gly Asn Val Val Asn Gly
    3980                3985                3990

Thr Ile Gly Lys Gln Met Val Asp Met Leu Val Glu Ser Ser Asn
    3995                4000                4005

Asn Val Glu Met Ile Leu Lys Phe Phe Asp Met Phe Leu Lys Leu
    4010                4015                4020

Lys Asp Leu Thr Ser Ser Asp Thr Phe Lys Glu Tyr Asp Pro Asp
    4025                4030                4035

Gly Lys Gly Val Ile Ser Lys Arg Asp Phe His Lys Ala Met Glu
    4040                4045                4050

Ser His Lys His Tyr Thr Gln Ser Glu Thr Glu Phe Leu Leu Ser
    4055                4060                4065

Cys Ala Glu Thr Asp Glu Asn Glu Thr Leu Asp Tyr Glu Glu Phe
    4070                4075                4080

Val Lys Arg Phe His Glu Pro Ala Lys Asp Ile Gly Phe Asn Val
    4085                4090                4095

Ala Val Leu Leu Thr Asn Leu Ser Glu His Met Pro Asn Asp Thr
    4100                4105                4110

Arg Leu Gln Thr Phe Leu Glu Leu Ala Glu Ser Val Leu Asn Tyr
    4115                4120                4125

Phe Gln Pro Phe Leu Gly Arg Ile Glu Ile Met Gly Ser Ala Lys
    4130                4135                4140

Arg Ile Glu Arg Val Tyr Phe Glu Ile Ser Glu Ser Ser Arg Thr
    4145                4150                4155

Gln Trp Glu Lys Pro Gln Val Lys Glu Ser Lys Arg Gln Phe Ile
    4160                4165                4170

Phe Asp Val Val Asn Glu Gly Gly Glu Lys Glu Lys Met Glu Leu
    4175                4180                4185

Phe Val Asn Phe Cys Glu Asp Thr Ile Phe Glu Met Gln Leu Ala
    4190                4195                4200

Ala Gln Ile Ser Glu Ser Asp Leu Asn Glu Arg Ser Ala Asn Lys
    4205                4210                4215

Glu Glu Ser Glu Lys Glu Arg Pro Glu Glu Gln Gly Pro Arg Met
    4220                4225                4230

Ala Phe Phe Ser Ile Leu Thr Val Arg Ser Ala Leu Phe Ala Leu
    4235                4240                4245

Arg Tyr Asn Ile Leu Thr Leu Met Arg Met Leu Ser Leu Lys Ser
    4250                4255                4260

Leu Lys Lys Gln Met Lys Lys Val Lys Lys Met Thr Val Lys Asp
    4265                4270                4275

Met Val Thr Ala Phe Phe Ser Ser Tyr Trp Ser Ile Phe Met Thr
    4280                4285                4290

Leu Leu His Phe Val Ala Ser Val Phe Arg Gly Phe Phe Arg Ile
    4295                4300                4305

Ile Cys Ser Leu Leu Leu Gly Gly Ser Leu Val Glu Gly Ala Lys
    4310                4315                4320

Lys Ile Lys Val Ala Glu Leu Leu Ala Asn Met Pro Asp Pro Thr
    4325                4330                4335

Gln Asp Glu Val Arg Gly Asp Gly Glu Glu Gly Glu Arg Lys Pro
    4340                4345                4350

Leu Glu Ala Ala Leu Pro Ser Glu Asp Leu Thr Asp Leu Lys Glu
    4355                4360                4365

Leu Thr Glu Glu Ser Asp Leu Leu Ser Asp Ile Phe Gly Leu Asp
    4370                4375                4380

Leu Lys Arg Glu Gly Gly Gln Tyr Lys Leu Ile Pro His Asn Pro
    4385                4390                4395

Asn Ala Gly Leu Ser Asp Leu Met Ser Asn Pro Val Pro Met Pro
    4400                4405                4410

Glu Val Gln Glu Lys Phe Gln Glu Gln Lys Ala Lys Glu Glu Glu
    4415                4420                4425

Lys Glu Glu Lys Glu Glu Thr Lys Ser Glu Pro Glu Lys Ala Glu
    4430                4435                4440

Gly Glu Asp Gly Glu Lys Glu Glu Lys Ala Lys Glu Asp Lys Gly
    4445                4450                4455

Lys Gln Lys Leu Arg Gln Leu His Thr His Arg Tyr Gly Glu Pro
    4460                4465                4470

Glu Val Pro Glu Ser Ala Phe Trp Lys Lys Ile Ile Ala Tyr Gln

4475                4480                4485

Gln Lys Leu Leu Asn Tyr Phe Ala Arg Asn Phe Tyr Asn Met Arg
    4490                4495                4500

Met Leu Ala Leu Phe Val Ala Phe Ala Ile Asn Phe Ile Leu Leu
    4505                4510                4515

Phe Tyr Lys Val Ser Thr Ser Ser Val Val Glu Gly Lys Glu Leu
    4520                4525                4530

Pro Thr Arg Ser Ser Ser Glu Asn Ala Lys Val Thr Ser Leu Asp
    4535                4540                4545

Ser Ser Ser His Arg Ile Ile Ala Val His Tyr Val Leu Glu Glu
    4550                4555                4560

Ser Ser Gly Tyr Met Glu Pro Thr Leu Arg Ile Leu Ala Ile Leu
    4565                4570                4575

His Thr Val Ile Ser Phe Phe Cys Ile Ile Gly Tyr Tyr Cys Leu
    4580                4585                4590

Lys Val Pro Leu Val Ile Phe Lys Arg Glu Lys Glu Val Ala Arg
    4595                4600                4605

Lys Leu Glu Phe Asp Gly Leu Tyr Ile Thr Glu Gln Pro Ser Glu
    4610                4615                4620

Asp Asp Ile Lys Gly Gln Trp Asp Arg Leu Val Ile Asn Thr Gln
    4625                4630                4635

Ser Phe Pro Asn Asn Tyr Trp Asp Lys Phe Val Lys Arg Lys Val
    4640                4645                4650

Met Asp Lys Tyr Gly Glu Phe Tyr Gly Arg Asp Arg Ile Ser Glu
    4655                4660                4665

Leu Leu Gly Met Asp Lys Ala Ala Leu Asp Phe Ser Asp Ala Arg
    4670                4675                4680

Glu Lys Lys Lys Pro Lys Lys Asp Ser Ser Leu Ser Ala Val Leu
    4685                4690                4695

Asn Ser Ile Asp Val Lys Tyr Gln Met Trp Lys Leu Gly Val Val
    4700                4705                4710

Phe Thr Asp Asn Ser Phe Leu Tyr Leu Ala Trp Tyr Met Thr Met
    4715                4720                4725

Ser Val Leu Gly His Tyr Asn Asn Phe Phe Phe Ala Ala His Leu
    4730                4735                4740

Leu Asp Ile Ala Met Gly Phe Lys Thr Leu Arg Thr Ile Leu Ser
    4745                4750                4755

Ser Val Thr His Asn Gly Lys Gln Leu Val Leu Thr Val Gly Leu
    4760                4765                4770

Leu Ala Val Val Val Tyr Leu Tyr Thr Val Val Ala Phe Asn Phe
    4775                4780                4785

Phe Arg Lys Phe Tyr Asn Lys Ser Glu Asp Gly Asp Thr Pro Asp
    4790                4795                4800

Met Lys Cys Asp Asp Met Leu Thr Cys Tyr Met Phe His Met Tyr
    4805                4810                4815

Val Gly Val Arg Ala Gly Gly Gly Ile Gly Asp Glu Ile Glu Asp
    4820                4825                4830

Pro Ala Gly Asp Glu Tyr Glu Ile Tyr Arg Ile Ile Phe Asp Ile
    4835                4840                4845

Thr Phe Phe Phe Phe Val Ile Val Ile Leu Leu Ala Ile Ile Gln
    4850                4855                4860

Gly Leu Ile Ile Asp Ala Phe Gly Glu Leu Arg Asp Gln Gln Glu
    4865                4870                4875

Gln Val Lys Glu Asp Met Glu Thr Lys Cys Phe Ile Cys Gly Ile
    4880                4885                4890

Gly Asn Asp Tyr Phe Asp Thr Val Pro His Gly Phe Glu Thr His
    4895                4900                4905

Thr Leu Gln Glu His Asn Leu Ala Asn Tyr Leu Phe Phe Leu Met
    4910                4915                4920

Tyr Leu Ile Asn Lys Asp Glu Thr Glu His Thr Gly Gln Glu Ser
    4925                4930                4935

Tyr Val Trp Lys Met Tyr Gln Glu Arg Cys Trp Glu Phe Phe Pro
    4940                4945                4950

Ala Gly Asp Cys Phe Arg Lys Gln Tyr Glu Asp Gln Leu Asn
    4955                4960                4965

<210> 2
<211> 310
<212> PRT
<213> Caenorhabditis elagans

<400> 2

Met Asp Tyr Gly Gly Ala Leu Ser Ala Val Gly Arg Glu Leu Leu Phe
1               5                   10                  15

Val Thr Asn Pro Val Val Val Asn Gly Ser Val Leu Val Pro Glu Asp
            20                  25                  30

Gln Cys Tyr Cys Ala Gly Trp Ile Glu Ser Arg Gly Thr Asn Gly Ala
        35                  40                  45

Gln Thr Ala Ser Asn Val Leu Gln Trp Leu Ala Ala Gly Phe Ser Ile
    50                  55                  60

Leu Leu Leu Met Phe Tyr Ala Tyr Gln Thr Trp Lys Ser Thr Cys Gly
65                  70                  75                  80

Trp Glu Glu Ile Tyr Val Cys Ala Ile Glu Met Val Lys Val Ile Leu
                85                  90                  95

Glu Phe Phe Phe Glu Phe Lys Asn Pro Ser Met Leu Tyr Leu Ala Thr
            100                 105                 110

Gly His Arg Val Gln Trp Leu Arg Tyr Ala Glu Trp Leu Leu Thr Cys
        115                 120                 125

Pro Val Ile Leu Ile His Leu Ser Asn Leu Thr Gly Leu Ser Asn Asp
    130                 135                 140

Tyr Ser Arg Arg Thr Met Gly Leu Leu Val Ser Asp Ile Gly Thr Ile
145                 150                 155                 160

Val Trp Gly Ala Thr Ser Ala Met Ala Thr Gly Tyr Val Lys Val Ile
                165                 170                 175

Phe Phe Cys Leu Gly Leu Cys Tyr Gly Ala Asn Thr Phe Phe His Ala
            180                 185                 190

Ala Lys Ala Tyr Ile Glu Gly Tyr His Thr Val Pro Lys Gly Arg Cys
        195                 200                 205

Arg Gln Val Val Thr Gly Met Ala Trp Leu Phe Phe Val Ser Trp Gly
    210                 215                 220

Met Phe Pro Ile Leu Phe Ile Leu Gly Pro Glu Gly Phe Gly Val Leu
225                 230                 235                 240

Ser Val Tyr Gly Ser Thr Val Gly His Thr Ile Ile Asp Leu Met Ser
                245                 250                 255

Lys Asn Cys Trp Gly Leu Leu Gly His Tyr Leu Arg Val Leu Ile His
            260                 265                 270

Glu His Ile Leu Ile His Gly Asp Ile Arg Lys Thr Thr Lys Leu Asn
        275                 280                 285

Ile Gly Gly Thr Glu Ile Glu Val Glu Thr Leu Val Glu Asp Glu Ala
    290                 295                 300

Glu Ala Gly Ala Val Pro
305                 310

<210>  3
<211>  310
<212>  PRT
<213>  artificial

<220>
<223>  mutated ChR,

<400>  3

Met Asp Tyr Gly Gly Ala Leu Ser Ala Val Gly Arg Glu Leu Leu Phe
1               5                   10                  15

Val Thr Asn Pro Val Val Val Asn Gly Ser Val Leu Val Pro Glu Asp
            20                  25                  30

Gln Cys Tyr Cys Ala Gly Trp Ile Glu Ser Arg Gly Thr Asn Gly Ala
        35                  40                  45

Gln Thr Ala Ser Asn Val Leu Gln Trp Leu Ala Ala Gly Phe Ser Ile
    50                  55                  60

Leu Leu Leu Met Phe Tyr Ala Tyr Gln Thr Trp Lys Ser Thr Cys Gly
65                  70                  75                  80

Trp Glu Glu Ile Tyr Val Cys Ala Ile Glu Met Val Lys Val Ile Leu
                85                  90                  95

Glu Phe Phe Phe Glu Phe Lys Asn Pro Ser Met Leu Tyr Leu Ala Thr
                100                 105                 110

Gly His Arg Val Gln Trp Leu Arg Tyr Ala Glu Trp Leu Leu Thr Cys
            115                 120                 125

Pro Val Ile Cys Ile Arg Leu Ser Asn Leu Thr Gly Leu Ser Asn Asp
    130                 135                 140

Tyr Ser Arg Arg Thr Met Gly Leu Leu Val Ser Asp Ile Gly Cys Ile
145                 150                 155                 160

Val Trp Gly Ala Thr Ser Ala Met Ala Thr Gly Tyr Val Lys Val Ile
                165                 170                 175

Phe Phe Cys Leu Gly Leu Cys Tyr Gly Ala Asn Thr Phe Phe His Ala
                180                 185                 190

Ala Lys Ala Tyr Ile Glu Gly Tyr His Thr Val Pro Lys Gly Arg Cys
            195                 200                 205

Arg Gln Val Val Thr Gly Met Ala Trp Leu Phe Phe Val Ser Trp Gly
    210                 215                 220

Met Phe Pro Ile Leu Phe Ile Leu Gly Pro Glu Gly Phe Gly Val Leu
225                 230                 235                 240

Ser Val Tyr Gly Ser Thr Val Gly His Thr Ile Ile Asp Leu Met Ser
                245                 250                 255

Lys Asn Cys Trp Gly Leu Leu Gly His Tyr Leu Arg Val Leu Ile His
                260                 265                 270

Glu His Ile Leu Ile His Gly Asp Ile Arg Lys Thr Thr Lys Leu Asn
            275                 280                 285

Ile Gly Gly Thr Glu Ile Glu Val Glu Thr Leu Val Glu Asp Glu Ala
    290                 295                 300

Glu Ala Gly Ala Val Pro
305                 310

<210>  4
<211>  192
<212>  PRT
<213>  chicken

<400>  4

Met Pro Asn Trp Gly Gly Gly Lys Lys Cys Gly Val Cys Gln Lys Ala
1               5                   10                  15

Val Tyr Phe Ala Glu Glu Val Gln Cys Glu Gly Ser Ser Phe His Lys
            20                  25                  30

Ser Cys Phe Leu Cys Met Val Cys Lys Lys Asn Leu Asp Ser Thr Thr
        35                  40                  45

Val Ala Val His Gly Asp Glu Ile Tyr Cys Lys Ser Cys Tyr Gly Lys
    50                  55                  60

Lys Tyr Gly Pro Lys Gly Lys Gly Lys Gly Met Gly Ala Gly Thr Leu
65                  70                  75                  80

Ser Thr Asp Lys Gly Glu Ser Leu Gly Ile Lys Tyr Glu Glu Gly Gln
                85                  90                  95

Ser His Arg Pro Thr Asn Pro Asn Ala Ser Arg Met Ala Gln Lys Val
            100                 105                 110

Gly Gly Ser Asp Gly Cys Pro Arg Cys Gly Gln Ala Val Tyr Ala Ala
        115                 120                 125

Glu Lys Val Ile Gly Ala Gly Lys Ser Trp His Lys Ser Cys Phe Arg
    130                 135                 140

Cys Ala Lys Cys Gly Lys Ser Leu Glu Ser Thr Thr Leu Ala Asp Lys
145                 150                 155                 160

Asp Gly Glu Ile Tyr Cys Lys Gly Cys Tyr Ala Lys Asn Phe Gly Pro
                165                 170                 175

Lys Gly Phe Gly Phe Gly Gln Gly Ala Gly Ala Leu Ile His Ser Gln
            180                 185                 190

<210>  5
<211>  5722
<212>  PRT
<213>  artificial

<220>
<223>  construct of chr, egf, linker and RyR2

<400>  5

Met Asp Tyr Gly Gly Ala Leu Ser Ala Val Gly Arg Glu Leu Leu Phe
1               5                   10                  15

Val Thr Asn Pro Val Val Val Asn Gly Ser Val Leu Val Pro Glu Asp

20                  25                  30

Gln Cys Tyr Cys Ala Gly Trp Ile Glu Ser Arg Gly Thr Asn Gly Ala
        35                  40                  45

Gln Thr Ala Ser Asn Val Leu Gln Trp Leu Ala Ala Gly Phe Ser Ile
    50                  55                  60

Leu Leu Leu Met Phe Tyr Ala Tyr Gln Thr Trp Lys Ser Thr Cys Gly
65                  70                  75                  80

Trp Glu Glu Ile Tyr Val Cys Ala Ile Glu Met Val Lys Val Ile Leu
                85                  90                  95

Glu Phe Phe Phe Glu Phe Lys Asn Pro Ser Met Leu Tyr Leu Ala Thr
            100                 105                 110

Gly His Arg Val Gln Trp Leu Arg Tyr Ala Glu Trp Leu Leu Thr Cys
        115                 120                 125

Pro Val Ile Cys Ile Arg Leu Ser Asn Leu Thr Gly Leu Ser Asn Asp
    130                 135                 140

Tyr Ser Arg Arg Thr Met Gly Leu Leu Val Ser Asp Ile Gly Cys Ile
145                 150                 155                 160

Val Trp Gly Ala Thr Ser Ala Met Ala Thr Gly Tyr Val Lys Val Ile
                165                 170                 175

Phe Phe Cys Leu Gly Leu Cys Tyr Gly Ala Asn Thr Phe Phe His Ala
            180                 185                 190

Ala Lys Ala Tyr Ile Glu Gly Tyr His Thr Val Pro Lys Gly Arg Cys
        195                 200                 205

Arg Gln Val Val Thr Gly Met Ala Trp Leu Phe Phe Val Ser Trp Gly
    210                 215                 220

Met Phe Pro Ile Leu Phe Ile Leu Gly Pro Glu Gly Phe Gly Val Leu
225                 230                 235                 240

Ser Val Tyr Gly Ser Thr Val Gly His Thr Ile Ile Asp Leu Met Ser
                245                 250                 255

Lys Asn Cys Trp Gly Leu Leu Gly His Tyr Leu Arg Val Leu Ile His
            260                 265                 270

Glu His Ile Leu Ile His Gly Asp Ile Arg Lys Thr Thr Lys Leu Asn
        275                 280                 285

Ile Gly Gly Thr Glu Ile Glu Val Glu Thr Leu Val Glu Asp Glu Ala
    290                 295                 300

Glu Ala Gly Ala Val Pro Ala Ala Ala Thr Met Val Ser Lys Gly Glu
305                 310                 315                 320

Glu Leu Phe Thr Gly Val Val Pro Ile Leu Val Glu Leu Asp Gly Asp
                325                 330                 335

Val Asn Gly His Lys Phe Ser Val Ser Gly Glu Gly Glu Gly Asp Ala
            340                 345                 350

Thr Tyr Gly Lys Leu Thr Leu Lys Phe Ile Cys Thr Thr Gly Lys Leu
        355                 360                 365

Pro Val Pro Trp Pro Thr Leu Val Thr Thr Phe Gly Tyr Gly Leu Gln
    370                 375                 380

Cys Phe Ala Arg Tyr Pro Asp His Met Lys Gln His Asp Phe Phe Lys
385                 390                 395                 400

Ser Ala Met Pro Glu Gly Tyr Val Gln Glu Arg Thr Ile Phe Phe Lys
                405                 410                 415

Asp Asp Gly Asn Tyr Lys Thr Arg Ala Glu Val Lys Phe Glu Gly Asp
            420                 425                 430

Thr Leu Val Asn Arg Ile Glu Leu Lys Gly Ile Asp Phe Lys Glu Asp
        435                 440                 445

Gly Asn Ile Leu Gly His Lys Leu Glu Tyr Asn Tyr Asn Ser His Asn
    450                 455                 460

Val Tyr Ile Met Ala Asp Lys Gln Lys Asn Gly Ile Lys Val Asn Phe
465                 470                 475                 480

Lys Ile Arg His Asn Ile Glu Asp Gly Ser Val Gln Leu Ala Asp His
                485                 490                 495

Tyr Gln Gln Asn Thr Pro Ile Gly Asp Gly Pro Val Leu Leu Pro Asp
            500                 505                 510

Asn His Tyr Leu Ser Tyr Gln Ser Ala Leu Ser Lys Asp Pro Asn Glu
        515                 520                 525

Lys Arg Asp His Met Val Leu Leu Glu Phe Val Thr Ala Ala Gly Ile
    530                 535                 540

Thr Leu Gly Met Asp Glu Leu Tyr Lys Met Pro Asn Trp Gly Gly Gly
545                 550                 555                 560

Lys Lys Cys Gly Val Cys Gln Lys Ala Val Tyr Phe Ala Glu Glu Val
                565                 570                 575

Gln Cys Glu Gly Ser Ser Phe His Lys Ser Cys Phe Leu Cys Met Val
            580                 585                 590

Cys Lys Lys Asn Leu Asp Ser Thr Thr Val Ala Val His Gly Asp Glu
        595                 600                 605

Ile Tyr Cys Lys Ser Cys Tyr Gly Lys Lys Tyr Gly Pro Lys Gly Lys
    610                 615                 620

Gly Lys Gly Met Gly Ala Gly Thr Leu Ser Thr Asp Lys Gly Glu Ser
625                 630                 635                 640

Leu Gly Ile Lys Tyr Glu Glu Gly Gln Ser His Arg Pro Thr Asn Pro
                645                 650                 655

Asn Ala Ser Arg Met Ala Gln Lys Val Gly Gly Ser Asp Gly Cys Pro
            660                 665                 670

Arg Cys Gly Gln Ala Val Tyr Ala Ala Glu Lys Val Ile Gly Ala Gly
        675                 680                 685

Lys Ser Trp His Lys Ser Cys Phe Arg Cys Ala Lys Cys Gly Lys Ser
    690                 695                 700

Leu Glu Ser Thr Thr Leu Ala Asp Lys Asp Gly Glu Ile Tyr Cys Lys
705                 710                 715                 720

Gly Cys Tyr Ala Lys Asn Phe Gly Pro Lys Gly Phe Gly Phe Gly Gln
                725                 730                 735

Gly Ala Gly Ala Leu Ile His Ser Gln Ser Ser Gly Arg Ser Arg Thr
            740                 745                 750

Ser Gly Thr Met Ala Asp Gly Gly Glu Gly Glu Asp Glu Ile Gln Phe
        755                 760                 765

Leu Arg Thr Asp Asp Glu Val Val Leu Gln Cys Thr Ala Thr Ile His
    770                 775                 780

Lys Glu Gln Gln Lys Leu Cys Leu Ala Ala Glu Gly Phe Gly Asn Arg
785                 790                 795                 800

Leu Cys Phe Leu Glu Ser Thr Ser Asn Ser Lys Asn Val Pro Pro Asp
                805                 810                 815

Leu Ser Ile Cys Thr Phe Val Leu Glu Gln Ser Leu Ser Val Arg Ala
            820                 825                 830

Leu Gln Glu Met Leu Ala Asn Thr Val Glu Lys Ser Glu Gly Gln Val
        835                 840                 845

Asp Val Glu Lys Trp Lys Phe Met Met Lys Thr Ala Gln Gly Gly Gly
    850                 855                 860

His Arg Thr Leu Leu Tyr Gly His Ala Ile Leu Leu Arg His Ser Tyr
865                 870                 875                 880

Ser Gly Met Tyr Leu Cys Cys Leu Ser Thr Ser Arg Ser Ser Thr Asp
                885                 890                 895

Lys Leu Ala Phe Asp Val Gly Leu Gln Glu Asp Thr Thr Gly Glu Ala
            900                 905                 910

Cys Trp Trp Thr Ile His Pro Ala Ser Lys Gln Arg Ser Glu Gly Glu
        915                 920                 925

Lys Val Arg Val Gly Asp Asp Leu Ile Leu Val Ser Val Ser Ser Glu
    930                 935                 940

Arg Tyr Leu His Leu Ser Tyr Gly Asn Gly Ser Leu His Val Asp Ala
945                 950                 955                 960

Ala Phe Gln Gln Thr Leu Trp Ser Val Ala Pro Ile Ser Ser Gly Ser
                965                 970                 975

Glu Ala Ala Gln Gly Tyr Leu Ile Gly Gly Asp Val Leu Arg Leu Leu
            980                 985                 990

His Gly His Met Asp Glu Cys Leu  Thr Val Pro Ser Gly  Glu His Gly
        995                 1000                1005

Glu Glu  Gln Arg Arg Thr Val  His Tyr Glu Gly Gly  Ala Val Ser
    1010                1015                1020

Val His  Ala Arg Ser Leu Trp  Arg Leu Glu Thr Leu  Arg Val Ala

1025                1030                1035

Trp Ser Gly Ser His Ile Arg Trp Gly Gln Pro Phe Arg Leu Arg
    1040                1045                1050

His Val Thr Thr Gly Lys Tyr Leu Ser Leu Met Glu Asp Lys Asn
    1055                1060                1065

Leu Leu Leu Met Asp Lys Glu Lys Ala Asp Val Lys Ser Thr Ala
    1070                1075                1080

Phe Thr Phe Arg Ser Ser Lys Glu Lys Leu Asp Val Gly Val Arg
    1085                1090                1095

Lys Glu Val Asp Gly Met Gly Thr Ser Glu Ile Lys Tyr Gly Asp
    1100                1105                1110

Ser Val Cys Tyr Ile Gln His Val Asp Thr Gly Leu Trp Leu Thr
    1115                1120                1125

Tyr Gln Ser Val Asp Val Lys Ser Val Arg Met Gly Ser Ile Gln
    1130                1135                1140

Arg Lys Ala Ile Met His His Glu Gly His Met Asp Asp Gly Ile
    1145                1150                1155

Ser Leu Ser Arg Ser Gln His Glu Glu Ser Arg Thr Ala Arg Val
    1160                1165                1170

Ile Arg Ser Thr Val Phe Leu Phe Asn Arg Phe Ile Arg Gly Leu
    1175                1180                1185

Asp Ala Leu Ser Lys Lys Ala Lys Ala Ser Thr Val Asp Leu Pro
    1190                1195                1200

Ile Glu Ser Val Ser Leu Ser Leu Gln Asp Leu Ile Gly Tyr Phe
    1205                1210                1215

His Pro Pro Asp Glu His Leu Glu His Glu Asp Lys Gln Asn Arg
    1220                1225                1230

Leu Arg Ala Leu Lys Asn Arg Gln Asn Leu Phe Gln Glu Glu Gly
    1235                1240                1245

Met Ile Asn Leu Val Leu Glu Cys Ile Asp Arg Leu His Val Tyr
    1250                1255                1260

Ser Ser Ala Ala His Phe Ala Asp Val Ala Gly Arg Glu Ala Gly
    1265                1270                1275

Glu Ser Trp Lys Ser Ile Leu Asn Ser Leu Tyr Glu Leu Leu Ala
    1280                1285                1290

Ala Leu Ile Arg Gly Asn Arg Lys Asn Cys Ala Gln Phe Ser Gly
    1295                1300                1305

Ser Leu Asp Trp Leu Ile Ser Arg Leu Glu Arg Leu Glu Ala Ser
    1310                1315                1320

Ser Gly Ile Leu Glu Val Leu His Cys Val Leu Val Glu Ser Pro
    1325                1330                1335

Glu Ala Leu Asn Ile Ile Lys Glu Gly His Ile Lys Ser Ile Ile
    1340                1345                1350

Ser Leu Leu Asp Lys His Gly Arg Asn His Lys Val Leu Asp Val
    1355                1360                1365

Leu Cys Ser Leu Cys Val Cys His Gly Val Ala Val Arg Ser Asn
    1370                1375                1380

Gln His Leu Ile Cys Asp Asn Leu Leu Pro Gly Arg Asp Leu Leu
    1385                1390                1395

Leu Gln Thr Arg Leu Val Asn His Val Ser Ser Met Arg Pro Asn
    1400                1405                1410

Ile Phe Leu Gly Val Ser Glu Gly Ser Ala Gln Tyr Lys Lys Trp
    1415                1420                1425

Tyr Tyr Glu Leu Met Val Asp His Thr Glu Pro Phe Val Thr Ala
    1430                1435                1440

Glu Ala Thr His Leu Arg Val Gly Trp Ala Ser Thr Glu Gly Tyr
    1445                1450                1455

Ser Pro Tyr Pro Gly Gly Gly Glu Glu Trp Gly Gly Asn Gly Val
    1460                1465                1470

Gly Asp Asp Leu Phe Ser Tyr Gly Phe Asp Gly Leu His Leu Trp
    1475                1480                1485

Ser Gly Cys Ile Ala Arg Thr Val Ser Ser Pro Asn Gln His Leu
    1490                1495                1500

Leu Arg Thr Asp Asp Val Ile Ser Cys Cys Leu Asp Leu Ser Ala
    1505                1510                1515

Pro Ser Ile Ser Phe Arg Ile Asn Gly Gln Pro Val Gln Gly Met
    1520                1525                1530

Phe Glu Asn Phe Asn Ile Asp Gly Leu Phe Phe Pro Val Val Ser
    1535                1540                1545

Phe Ser Ala Gly Ile Lys Val Arg Phe Leu Leu Gly Gly Arg His
    1550                1555                1560

Gly Glu Phe Lys Phe Leu Pro Pro Pro Gly Tyr Ala Pro Cys Tyr
    1565                1570                1575

Glu Ala Val Leu Pro Lys Glu Lys Leu Lys Val Glu His Ser Arg
    1580                1585                1590

Glu Tyr Lys Gln Glu Arg Thr Tyr Thr Arg Asp Leu Leu Gly Pro
    1595                1600                1605

Thr Val Ser Leu Thr Gln Ala Ala Phe Thr Pro Ile Pro Val Asp
    1610                1615                1620

Thr Ser Gln Ile Val Leu Pro Pro His Leu Glu Arg Ile Arg Glu
    1625                1630                1635

Lys Leu Ala Glu Asn Ile His Glu Leu Trp Val Met Asn Lys Ile
    1640                1645                1650

Glu Leu Gly Trp Gln Tyr Gly Pro Val Arg Asp Asp Asn Lys Arg
    1655                1660                1665

Gln His Pro Cys Leu Val Glu Phe Ser Lys Leu Pro Glu Gln Glu
    1670                1675                1680

Arg Asn Tyr Asn Leu Gln Met Ser Leu Glu Thr Leu Lys Thr Leu
    1685                1690                1695

Leu Ala Leu Gly Cys His Val Gly Ile Ser Asp Glu His Ala Glu
    1700                1705                1710

Asp Lys Val Lys Lys Met Lys Leu Pro Lys Asn Tyr Gln Leu Thr
    1715                1720                1725

Ser Gly Tyr Lys Pro Ala Pro Met Asp Leu Ser Phe Ile Lys Leu
    1730                1735                1740

Thr Pro Ser Gln Glu Ala Met Val Asp Lys Leu Ala Glu Asn Ala
    1745                1750                1755

His Asn Val Trp Ala Arg Asp Arg Ile Arg Gln Gly Trp Thr Tyr
    1760                1765                1770

Gly Ile Gln Gln Asp Val Lys Asn Arg Arg Asn Pro Arg Leu Val
    1775                1780                1785

Pro Tyr Thr Leu Leu Asp Asp Arg Thr Lys Lys Ser Asn Lys Asp
    1790                1795                1800

Ser Leu Arg Glu Ala Val Arg Thr Leu Leu Gly Tyr Gly Tyr Asn
    1805                1810                1815

Leu Glu Ala Pro Asp Gln Asp His Ala Ala Arg Ala Glu Val Cys
    1820                1825                1830

Ser Gly Thr Gly Glu Arg Phe Arg Ile Phe Arg Ala Glu Lys Thr
    1835                1840                1845

Tyr Ala Val Lys Ala Gly Arg Trp Tyr Phe Glu Phe Glu Thr Val
    1850                1855                1860

Thr Ala Gly Asp Met Arg Val Gly Trp Ser Arg Pro Gly Cys Gln
    1865                1870                1875

Pro Asp Gln Glu Leu Gly Ser Asp Glu Arg Ala Phe Ala Phe Asp
    1880                1885                1890

Gly Phe Lys Ala Gln Arg Trp His Gln Gly Asn Glu His Tyr Gly
    1895                1900                1905

Arg Ser Trp Gln Ala Gly Asp Val Val Gly Cys Met Val Asp Met
    1910                1915                1920

Asn Glu His Thr Met Met Phe Thr Leu Asn Gly Glu Ile Leu Leu
    1925                1930                1935

Asp Asp Ser Gly Ser Glu Leu Ala Phe Lys Asp Phe Asp Val Gly
    1940                1945                1950

Asp Gly Phe Ile Pro Val Cys Ser Leu Gly Val Ala Gln Val Gly
    1955                1960                1965

Arg Met Asn Phe Gly Lys Asp Val Ser Thr Leu Lys Tyr Phe Thr

1970                1975                1980

Ile Cys Gly Leu Gln Glu Gly Tyr Glu Pro Phe Ala Val Asn Thr
    1985                1990                1995

Asn Arg Asp Ile Thr Met Trp Leu Ser Lys Arg Leu Pro Gln Phe
    2000                2005                2010

Leu Gln Val Pro Ser Asn His Glu His Ile Glu Val Thr Arg Ile
    2015                2020                2025

Asp Gly Thr Ile Asp Ser Ser Pro Cys Leu Lys Val Thr Gln Lys
    2030                2035                2040

Ser Phe Gly Ser Gln Asn Ser Asn Thr Asp Ile Met Phe Tyr Arg
    2045                2050                2055

Leu Ser Met Pro Ile Glu Cys Ala Glu Val Phe Ser Lys Thr Val
    2060                2065                2070

Ala Gly Gly Leu Pro Gly Ala Gly Leu Phe Gly Pro Lys Asn Asp
    2075                2080                2085

Leu Glu Asp Tyr Asp Ala Asp Ser Asp Phe Glu Val Leu Met Lys
    2090                2095                2100

Thr Ala His Gly His Leu Val Pro Asp Arg Val Asp Lys Asp Lys
    2105                2110                2115

Glu Ala Thr Lys Pro Glu Phe Asn Asn His Lys Asp Tyr Ala Gln
    2120                2125                2130

Glu Lys Pro Ser Arg Leu Lys Gln Arg Phe Leu Leu Arg Arg Thr
    2135                2140                2145

Lys Pro Asp Tyr Ser Thr Ser His Ser Ala Arg Leu Thr Glu Asp
    2150                2155                2160

Val Leu Ala Asp Asp Arg Asp Asp Tyr Asp Phe Leu Met Gln Thr
    2165                2170                2175

Ser Thr Tyr Tyr Tyr Ser Val Arg Ile Phe Pro Gly Gln Glu Pro
    2180                2185                2190

Ala Asn Val Trp Val Gly Trp Ile Thr Ser Asp Phe His Gln Tyr
    2195                2200                2205

Asp Thr Gly Phe Asp Leu Asp Arg Val Arg Thr Val Thr Val Thr
    2210                2215                2220

Leu Gly Asp Glu Lys Gly Lys Val His Glu Ser Ile Lys Arg Ser
    2225                2230                2235

Asn Cys Tyr Met Val Cys Ala Gly Glu Ser Met Ser Pro Gly Gln
    2240                2245                2250

Gly Arg Asn Asn Asn Gly Leu Glu Ile Gly Cys Val Val Asp Ala
    2255                2260                2265

Ala Ser Gly Leu Leu Thr Phe Ile Ala Asn Gly Lys Glu Leu Ser
    2270                2275                2280

Thr Tyr Tyr Gln Val Glu Pro Ser Thr Lys Leu Phe Pro Ala Val
    2285                2290                2295

Phe Ala Gln Ala Thr Ser Pro Asn Val Phe Gln Phe Glu Leu Gly
    2300                2305                2310

Arg Ile Lys Asn Val Met Pro Leu Ser Ala Gly Leu Phe Lys Ser
    2315                2320                2325

Glu His Lys Asn Pro Val Pro Gln Cys Pro Pro Arg Leu His Val
    2330                2335                2340

Gln Phe Leu Ser His Val Leu Trp Ser Arg Met Pro Asn Gln Phe
    2345                2350                2355

Leu Lys Val Asp Val Ser Arg Ile Ser Glu Arg Gln Gly Trp Leu
    2360                2365                2370

Val Gln Cys Leu Asp Pro Leu Gln Phe Met Ser Leu His Ile Pro
    2375                2380                2385

Glu Glu Asn Arg Ser Val Asp Ile Leu Glu Leu Thr Glu Gln Glu
    2390                2395                2400

Glu Leu Leu Lys Phe His Tyr His Thr Leu Arg Leu Tyr Ser Ala
    2405                2410                2415

Val Cys Ala Leu Gly Asn His Arg Val Ala His Ala Leu Cys Ser
    2420                2425                2430

His Val Asp Glu Pro Gln Leu Leu Tyr Ala Ile Glu Asn Lys Tyr
    2435                2440                2445

Met Pro Gly Leu Leu Arg Ala Gly Tyr Tyr Asp Leu Leu Ile Asp
    2450                2455                2460

Ile His Leu Ser Ser Tyr Ala Thr Ala Arg Leu Met Met Asn Asn
    2465                2470                2475

Glu Tyr Ile Val Pro Met Thr Glu Glu Thr Lys Ser Ile Thr Leu
    2480                2485                2490

Phe Pro Asp Glu Asn Lys Lys His Gly Leu Pro Gly Ile Gly Leu
    2495                2500                2505

Ser Thr Ser Leu Arg Pro Arg Met Gln Phe Ser Ser Pro Ser Phe
    2510                2515                2520

Val Ser Ile Ser Asn Glu Cys Tyr Gln Tyr Ser Pro Glu Phe Pro
    2525                2530                2535

Leu Asp Ile Leu Lys Ser Lys Thr Ile Gln Met Leu Thr Glu Ala
    2540                2545                2550

Val Lys Glu Gly Ser Leu His Ala Arg Asp Pro Val Gly Gly Thr
    2555                2560                2565

Thr Glu Phe Leu Phe Val Pro Leu Ile Lys Leu Phe Tyr Thr Leu
    2570                2575                2580

Leu Ile Met Gly Ile Phe His Asn Glu Asp Leu Lys His Ile Leu
    2585                2590                2595

Gln Leu Ile Glu Pro Ser Val Phe Lys Glu Ala Ala Thr Pro Glu
    2600                2605                2610

Glu Glu Ser Asp Thr Leu Glu Lys Glu Leu Ser Val Asp Asp Ala
    2615                2620                2625

Lys Leu Gln Gly Ala Gly Glu Glu Glu Ala Lys Gly Gly Lys Arg
    2630                2635                2640

Pro Lys Glu Gly Leu Leu Gln Met Lys Leu Pro Glu Pro Val Lys
    2645                2650                2655

Leu Gln Met Cys Leu Leu Leu Gln Tyr Leu Cys Asp Cys Gln Val
    2660                2665                2670

Arg His Arg Ile Glu Ala Ile Val Ala Phe Ser Asp Asp Phe Val
    2675                2680                2685

Ala Lys Leu Gln Asp Asn Gln Arg Phe Arg Tyr Asn Glu Val Met
    2690                2695                2700

Gln Ala Leu Asn Met Ser Ala Ala Leu Thr Ala Arg Lys Thr Lys
    2705                2710                2715

Glu Phe Arg Ser Pro Pro Gln Glu Gln Ile Asn Met Leu Leu Asn
    2720                2725                2730

Phe Lys Asp Asp Lys Ser Glu Cys Pro Cys Pro Glu Glu Ile Arg
    2735                2740                2745

Asp Gln Leu Leu Asp Phe His Glu Asp Leu Met Thr His Cys Gly
    2750                2755                2760

Ile Glu Leu Asp Glu Asp Gly Ser Leu Asp Gly Asn Ser Asp Leu
    2765                2770                2775

Thr Ile Arg Gly Arg Leu Leu Ser Leu Val Glu Lys Val Thr Tyr
    2780                2785                2790

Leu Lys Lys Lys Gln Ala Glu Lys Pro Val Glu Ser Asp Ser Lys
    2795                2800                2805

Lys Ser Ser Thr Leu Gln Gln Leu Ile Ser Glu Thr Met Val Arg
    2810                2815                2820

Trp Ala Gln Glu Ser Val Ile Glu Asp Pro Glu Leu Val Arg Ala
    2825                2830                2835

Met Phe Val Leu Leu His Arg Gln Tyr Asp Gly Ile Gly Gly Leu
    2840                2845                2850

Val Arg Ala Leu Pro Lys Thr Tyr Thr Ile Asn Gly Val Ser Val
    2855                2860                2865

Glu Asp Thr Ile Asn Leu Leu Ala Ser Leu Gly Gln Ile Arg Ser
    2870                2875                2880

Leu Leu Ser Val Arg Met Gly Lys Glu Glu Glu Lys Leu Met Ile
    2885                2890                2895

Arg Gly Leu Gly Asp Ile Met Asn Asn Lys Val Phe Tyr Gln His
    2900                2905                2910

Pro Asn Leu Met Arg Ala Leu Gly Met His Glu Thr Val Met Glu

2915                2920                2925

Val Met Val Asn Val Leu Gly Gly Gly Glu Ser Lys Glu Ile Thr
    2930                2935                2940

Phe Pro Lys Met Val Ala Asn Cys Cys Arg Phe Leu Cys Tyr Phe
    2945                2950                2955

Cys Arg Ile Ser Arg Gln Asn Gln Lys Ala Met Phe Asp His Leu
    2960                2965                2970

Ser Tyr Leu Leu Glu Asn Ser Ser Val Gly Leu Ala Ser Pro Ala
    2975                2980                2985

Met Arg Gly Ser Thr Pro Leu Asp Val Ala Ala Ala Ser Val Met
    2990                2995                3000

Asp Asn Asn Glu Leu Ala Leu Ala Leu Arg Glu Pro Asp Leu Glu
    3005                3010                3015

Lys Val Val Arg Tyr Leu Ala Gly Cys Gly Leu Gln Ser Cys Gln
    3020                3025                3030

Met Leu Val Ser Lys Gly Tyr Pro Asp Ile Gly Trp Asn Pro Val
    3035                3040                3045

Glu Gly Glu Arg Tyr Leu Asp Phe Leu Arg Phe Ala Val Phe Cys
    3050                3055                3060

Asn Gly Glu Ser Val Glu Glu Asn Ala Asn Val Val Val Arg Leu
    3065                3070                3075

Leu Ile Arg Arg Pro Glu Cys Phe Gly Pro Ala Leu Arg Gly Glu
    3080                3085                3090

Gly Gly Asn Gly Leu Leu Ala Ala Met Glu Glu Ala Ile Lys Ile
    3095                3100                3105

Ala Glu Asp Pro Ser Arg Asp Gly Pro Ser Pro Asn Ser Gly Ser
    3110                3115                3120

Ser Lys Thr Leu Asp Thr Glu Glu Glu Glu Asp Asp Thr Ile His
    3125                3130                3135

Met Gly Asn Ala Ile Met Thr Phe Tyr Ser Ala Leu Ile Asp Leu
    3140                3145                3150

Leu Gly Arg Cys Ala Pro Glu Met His Leu Ile His Ala Gly Lys
    3155                3160                3165

Gly Glu Ala Ile Arg Ile Arg Ser Ile Leu Arg Ser Leu Ile Pro
    3170                3175                3180

Leu Gly Asp Leu Val Gly Val Ile Ser Ile Ala Phe Gln Met Pro
    3185                3190                3195

Thr Ile Ala Lys Asp Gly Asn Val Val Glu Pro Asp Met Ser Ala
    3200                3205                3210

Gly Phe Cys Pro Asp His Lys Ala Ala Met Val Leu Phe Leu Asp
    3215                3220                3225

Arg Val Tyr Gly Ile Glu Val Gln Asp Phe Leu Leu His Leu Leu
    3230                3235                3240

Glu Val Gly Phe Leu Pro Asp Leu Arg Ala Ala Ala Ser Leu Asp
    3245                3250                3255

Thr Ala Ala Leu Ser Ala Thr Asp Met Ala Leu Ala Leu Asn Arg
    3260                3265                3270

Tyr Leu Cys Thr Ala Val Leu Pro Leu Leu Thr Arg Cys Ala Pro
    3275                3280                3285

Leu Phe Ala Gly Thr Glu His His Ala Ser Leu Ile Asp Ser Leu
    3290                3295                3300

Leu His Thr Val Tyr Arg Leu Ser Lys Gly Cys Ser Leu Thr Lys
    3305                3310                3315

Ala Gln Arg Asp Ser Ile Glu Val Cys Leu Leu Ser Ile Cys Gly
    3320                3325                3330

Gln Leu Arg Pro Ser Met Met Gln His Leu Leu Arg Arg Leu Val
    3335                3340                3345

Phe Asp Val Pro Leu Leu Asn Glu His Ala Lys Met Pro Leu Lys
    3350                3355                3360

Leu Leu Thr Asn His Tyr Glu Arg Cys Trp Lys Tyr Tyr Cys Leu
    3365                3370                3375

Pro Gly Gly Trp Gly Asn Phe Gly Ala Ala Ser Glu Glu Glu Leu
    3380                3385                3390

His Leu Ser Arg Lys Leu Phe Trp Gly Ile Phe Asp Ala Leu Ser
    3395                3400                3405

Gln Lys Lys Tyr Glu Gln Glu Leu Phe Lys Leu Ala Leu Pro Cys
    3410                3415                3420

Leu Ser Ala Val Ala Gly Ala Leu Pro Pro Asp Tyr Met Glu Ser
    3425                3430                3435

Asn Tyr Val Ser Met Met Glu Lys Gln Ser Ser Met Asp Ser Glu
    3440                3445                3450

Gly Asn Phe Asn Pro Gln Pro Val Asp Thr Ser Asn Ile Thr Ile
    3455                3460                3465

Pro Glu Lys Leu Glu Tyr Phe Ile Asn Lys Tyr Ala Glu His Ser
    3470                3475                3480

His Asp Lys Trp Ser Met Asp Lys Leu Ala Asn Gly Trp Ile Tyr
    3485                3490                3495

Gly Glu Ile Tyr Ser Asp Ser Ser Lys Val Gln Pro Leu Met Lys
    3500                3505                3510

Pro Tyr Lys Leu Leu Ser Glu Lys Glu Lys Glu Ile Tyr Arg Trp
    3515                3520                3525

Pro Ile Lys Glu Ser Leu Lys Thr Met Leu Ala Trp Gly Trp Arg
    3530                3535                3540

Ile Glu Arg Thr Arg Glu Gly Asp Ser Met Ala Leu Tyr Asn Arg
    3545                3550                3555

Thr Arg Arg Ile Ser Gln Thr Ser Gln Val Ser Val Asp Ala Ala
    3560                3565                3570

His Gly Tyr Ser Pro Arg Ala Ile Asp Met Ser Asn Val Thr Leu
    3575                3580                3585

Ser Arg Asp Leu His Ala Met Ala Glu Met Met Ala Glu Asn Tyr
    3590                3595                3600

His Asn Ile Trp Ala Lys Lys Lys Lys Met Glu Leu Glu Ser Lys
    3605                3610                3615

Gly Gly Gly Asn His Pro Leu Leu Val Pro Tyr Asp Thr Leu Thr
    3620                3625                3630

Ala Lys Glu Lys Ala Lys Asp Arg Glu Lys Ala Gln Asp Ile Leu
    3635                3640                3645

Lys Phe Leu Gln Ile Asn Gly Tyr Ala Val Ser Arg Gly Phe Lys
    3650                3655                3660

Asp Leu Glu Leu Asp Thr Pro Ser Ile Glu Lys Arg Phe Ala Tyr
    3665                3670                3675

Ser Phe Leu Gln Gln Leu Ile Arg Tyr Val Asp Glu Ala His Gln
    3680                3685                3690

Tyr Ile Leu Glu Phe Asp Gly Gly Ser Arg Gly Lys Gly Glu His
    3695                3700                3705

Phe Pro Tyr Glu Gln Glu Ile Lys Phe Phe Ala Lys Val Val Leu
    3710                3715                3720

Pro Leu Ile Asp Gln Tyr Phe Lys Asn His Arg Leu Tyr Phe Leu
    3725                3730                3735

Ser Ala Ala Ser Arg Pro Leu Cys Ser Gly Gly His Ala Ser Asn
    3740                3745                3750

Lys Glu Lys Glu Met Val Thr Ser Leu Phe Cys Lys Leu Gly Val
    3755                3760                3765

Leu Val Arg His Arg Ile Ser Leu Phe Gly Asn Asp Ala Thr Ser
    3770                3775                3780

Ile Val Asn Cys Leu His Ile Leu Gly Gln Thr Leu Asp Ala Arg
    3785                3790                3795

Thr Val Met Lys Thr Gly Leu Glu Ser Val Lys Ser Ala Leu Arg
    3800                3805                3810

Ala Phe Leu Asp Asn Ala Ala Glu Asp Leu Glu Lys Thr Met Glu
    3815                3820                3825

Asn Leu Lys Gln Gly Gln Phe Thr His Thr Arg Asn Gln Pro Lys
    3830                3835                3840

Gly Val Thr Gln Ile Ile Asn Tyr Thr Thr Val Ala Leu Leu Pro
    3845                3850                3855

Met Leu Ser Ser Leu Phe Glu His Ile Gly Gln His Gln Phe Gly

3860                3865                3870

Glu Asp Leu Ile Leu Glu Asp Val Gln Val Ser Cys Tyr Arg Ile
    3875                3880                3885

Leu Thr Ser Leu Tyr Ala Leu Gly Thr Ser Lys Ser Ile Tyr Val
    3890                3895                3900

Glu Arg Gln Arg Ser Ala Leu Gly Glu Cys Leu Ala Ala Phe Ala
    3905                3910                3915

Gly Ala Phe Pro Val Ala Phe Leu Glu Thr His Leu Asp Lys His
    3920                3925                3930

Asn Ile Tyr Ser Ile Tyr Asn Thr Lys Ser Ser Arg Glu Arg Ala
    3935                3940                3945

Ala Leu Ser Leu Pro Thr Asn Val Glu Asp Val Cys Pro Asn Ile
    3950                3955                3960

Pro Ser Leu Glu Lys Leu Met Glu Glu Ile Val Glu Leu Ala Glu
    3965                3970                3975

Ser Gly Ile Arg Tyr Thr Gln Met Pro His Val Met Glu Val Ile
    3980                3985                3990

Leu Pro Met Leu Cys Ser Tyr Met Ser Arg Trp Trp Glu His Gly
    3995                4000                4005

Pro Glu Asn Asn Pro Glu Arg Ala Glu Met Cys Cys Thr Ala Leu
    4010                4015                4020

Asn Ser Glu His Met Asn Thr Leu Leu Gly Asn Ile Leu Lys Ile
    4025                4030                4035

Ile Tyr Asn Asn Leu Gly Ile Asp Glu Gly Ala Trp Met Lys Arg
    4040                4045                4050

Leu Ala Val Phe Ser Gln Pro Ile Ile Asn Lys Val Lys Pro Gln
    4055                4060                4065

Leu Leu Lys Thr His Phe Leu Pro Leu Met Glu Lys Leu Lys Lys
    4070                4075                4080

Lys Ala Ala Thr Val Val Ser Glu Glu Asp His Leu Lys Ala Glu
    4085                4090                4095

Ala Arg Gly Asp Met Ser Glu Ala Glu Leu Leu Ile Leu Asp Glu
    4100                4105                4110

Phe Thr Thr Leu Ala Arg Asp Leu Tyr Ala Phe Tyr Pro Leu Leu
    4115                4120                4125

Ile Arg Phe Val Asp Tyr Asn Arg Ala Lys Trp Leu Lys Glu Pro
    4130                4135                4140

Asn Pro Glu Ala Glu Glu Leu Phe Arg Met Val Ala Glu Val Phe
    4145                4150                4155

Ile Tyr Trp Ser Lys Ser His Asn Phe Lys Arg Glu Glu Gln Asn
    4160                4165                4170

Phe Val Val Gln Asn Glu Ile Asn Asn Met Ser Phe Leu Ile Thr
    4175                4180                4185

Asp Thr Lys Ser Lys Met Ser Lys Ala Ala Val Ser Asp Gln Glu
    4190                4195                4200

Arg Lys Lys Met Lys Arg Lys Gly Asp Arg Tyr Ser Met Gln Thr
    4205                4210                4215

Ser Leu Ile Val Ala Ala Leu Lys Arg Leu Leu Pro Ile Gly Leu
    4220                4225                4230

Asn Ile Cys Ala Pro Gly Asp Gln Glu Leu Ile Ala Leu Ala Lys
    4235                4240                4245

Asn Arg Phe Ser Leu Lys Asp Thr Glu Asp Glu Val Arg Asp Ile
    4250                4255                4260

Ile Arg Ser Asn Ile His Leu Gln Gly Lys Leu Glu Asp Pro Ala
    4265                4270                4275

Ile Arg Trp Gln Met Ala Leu Tyr Lys Asp Leu Pro Asn Arg Thr
    4280                4285                4290

Asp Asp Thr Ser Asp Pro Glu Lys Thr Val Glu Arg Val Leu Asp
    4295                4300                4305

Ile Ala Asn Val Leu Phe His Leu Glu Gln Lys Ser Lys Arg Val
    4310                4315                4320

Gly Arg Arg His Tyr Cys Leu Val Glu His Pro Gln Arg Ser Lys
    4325                4330                4335

Lys Ala Val Trp His Lys Leu Leu Ser Lys Gln Arg Lys Arg Ala
    4340                4345                4350

Val Val Ala Cys Phe Arg Met Ala Pro Leu Tyr Asn Leu Pro Arg
    4355                4360                4365

His Arg Ala Val Asn Leu Phe Leu Gln Gly Tyr Glu Lys Ser Trp
    4370                4375                4380

Ile Glu Thr Glu Glu His Tyr Phe Glu Asp Lys Leu Ile Glu Asp
    4385                4390                4395

Leu Ala Lys Pro Gly Ala Glu Pro Pro Glu Glu Asp Glu Gly Thr
    4400                4405                4410

Lys Arg Val Asp Pro Leu His Gln Leu Ile Leu Leu Phe Ser Arg
    4415                4420                4425

Thr Ala Leu Thr Glu Lys Cys Lys Leu Glu Glu Asp Phe Leu Tyr
    4430                4435                4440

Met Ala Tyr Ala Asp Ile Met Ala Lys Ser Cys His Asp Glu Glu
    4445                4450                4455

Asp Asp Asp Gly Glu Glu Glu Val Lys Ser Phe Glu Glu Lys Glu
    4460                4465                4470

Met Glu Lys Gln Lys Leu Leu Tyr Gln Gln Ala Arg Leu His Asp
    4475                4480                4485

Arg Gly Ala Ala Glu Met Val Leu Gln Thr Ile Ser Ala Ser Lys
    4490                4495                4500

Gly Glu Thr Gly Pro Met Val Ala Ala Thr Leu Lys Leu Gly Ile
    4505                4510                4515

Ala Ile Leu Asn Gly Gly Asn Ser Thr Val Gln Gln Lys Met Leu
    4520                4525                4530

Asp Tyr Leu Lys Glu Lys Lys Asp Val Gly Phe Phe Gln Ser Leu
    4535                4540                4545

Ala Gly Leu Met Gln Ser Cys Ser Val Leu Asp Leu Asn Ala Phe
    4550                4555                4560

Glu Arg Gln Asn Lys Ala Glu Gly Leu Gly Met Val Thr Glu Glu
    4565                4570                4575

Gly Ser Gly Glu Lys Val Leu Gln Asp Asp Glu Phe Thr Cys Asp
    4580                4585                4590

Leu Phe Arg Phe Leu Gln Leu Leu Cys Glu Gly His Asn Ser Asp
    4595                4600                4605

Phe Gln Asn Tyr Leu Arg Thr Gln Thr Gly Asn Asn Thr Thr Val
    4610                4615                4620

Asn Ile Ile Ile Ser Thr Val Asp Tyr Leu Leu Arg Val Gln Glu
    4625                4630                4635

Ser Ile Ser Asp Phe Tyr Trp Tyr Tyr Ser Gly Lys Asp Val Ile
    4640                4645                4650

Asp Glu Gln Gly Gln Arg Asn Phe Ser Lys Ala Ile Gln Val Ala
    4655                4660                4665

Lys Gln Val Phe Asn Thr Leu Thr Glu Tyr Ile Gln Gly Pro Cys
    4670                4675                4680

Thr Gly Asn Gln Gln Ser Leu Ala His Ser Arg Leu Trp Asp Ala
    4685                4690                4695

Val Val Gly Phe Leu His Val Phe Ala His Met Gln Met Lys Leu
    4700                4705                4710

Ser Gln Asp Ser Ser Gln Ile Glu Leu Leu Lys Glu Leu Met Asp
    4715                4720                4725

Leu Gln Lys Asp Met Val Val Met Leu Leu Ser Met Leu Glu Gly
    4730                4735                4740

Asn Val Val Asn Gly Thr Ile Gly Lys Gln Met Val Asp Met Leu
    4745                4750                4755

Val Glu Ser Ser Asn Asn Val Glu Met Ile Leu Lys Phe Phe Asp
    4760                4765                4770

Met Phe Leu Lys Leu Lys Asp Leu Thr Ser Ser Asp Thr Phe Lys
    4775                4780                4785

Glu Tyr Asp Pro Asp Gly Lys Gly Val Ile Ser Lys Arg Asp Phe
    4790                4795                4800

His Lys Ala Met Glu Ser His Lys His Tyr Thr Gln Ser Glu Thr

4805                4810                4815

Glu Phe Leu Leu Ser Cys Ala Glu Thr Asp Glu Asn Glu Thr Leu
    4820                4825                4830

Asp Tyr Glu Glu Phe Val Lys Arg Phe His Glu Pro Ala Lys Asp
    4835                4840                4845

Ile Gly Phe Asn Val Ala Val Leu Leu Thr Asn Leu Ser Glu His
    4850                4855                4860

Met Pro Asn Asp Thr Arg Leu Gln Thr Phe Leu Glu Leu Ala Glu
    4865                4870                4875

Ser Val Leu Asn Tyr Phe Gln Pro Phe Leu Gly Arg Ile Glu Ile
    4880                4885                4890

Met Gly Ser Ala Lys Arg Ile Glu Arg Val Tyr Phe Glu Ile Ser
    4895                4900                4905

Glu Ser Ser Arg Thr Gln Trp Glu Lys Pro Gln Val Lys Glu Ser
    4910                4915                4920

Lys Arg Gln Phe Ile Phe Asp Val Val Asn Glu Gly Gly Glu Lys
    4925                4930                4935

Glu Lys Met Glu Leu Phe Val Asn Phe Cys Glu Asp Thr Ile Phe
    4940                4945                4950

Glu Met Gln Leu Ala Ala Gln Ile Ser Glu Ser Asp Leu Asn Glu
    4955                4960                4965

Arg Ser Ala Asn Lys Glu Glu Ser Glu Lys Glu Arg Pro Glu Glu
    4970                4975                4980

Gln Gly Pro Arg Met Ala Phe Phe Ser Ile Leu Thr Val Arg Ser
    4985                4990                4995

Ala Leu Phe Ala Leu Arg Tyr Asn Ile Leu Thr Leu Met Arg Met
    5000                5005                5010

Leu Ser Leu Lys Ser Leu Lys Lys Gln Met Lys Lys Val Lys Lys
    5015                5020                5025

Met Thr Val Lys Asp Met Val Thr Ala Phe Phe Ser Ser Tyr Trp
    5030                5035                5040

Ser Ile Phe Met Thr Leu Leu His Phe Val Ala Ser Val Phe Arg
    5045                5050                5055

Gly Phe Phe Arg Ile Ile Cys Ser Leu Leu Leu Gly Gly Ser Leu
    5060                5065                5070

Val Glu Gly Ala Lys Lys Ile Lys Val Ala Glu Leu Leu Ala Asn
    5075                5080                5085

Met Pro Asp Pro Thr Gln Asp Glu Val Arg Gly Asp Gly Glu Glu
    5090                5095                5100

Gly Glu Arg Lys Pro Leu Glu Ala Ala Leu Pro Ser Glu Asp Leu
    5105                5110                5115

Thr Asp Leu Lys Glu Leu Thr Glu Glu Ser Asp Leu Leu Ser Asp
    5120                5125                5130

Ile Phe Gly Leu Asp Leu Lys Arg Glu Gly Gly Gln Tyr Lys Leu
    5135                5140                5145

Ile Pro His Asn Pro Asn Ala Gly Leu Ser Asp Leu Met Ser Asn
    5150                5155                5160

Pro Val Pro Met Pro Glu Val Gln Glu Lys Phe Gln Glu Gln Lys
    5165                5170                5175

Ala Lys Glu Glu Glu Lys Glu Glu Lys Glu Glu Thr Lys Ser Glu
    5180                5185                5190

Pro Glu Lys Ala Glu Gly Glu Asp Gly Glu Lys Glu Glu Lys Ala
    5195                5200                5205

Lys Glu Asp Lys Gly Lys Gln Lys Leu Arg Gln Leu His Thr His
    5210                5215                5220

Arg Tyr Gly Glu Pro Glu Val Pro Glu Ser Ala Phe Trp Lys Lys
    5225                5230                5235

Ile Ile Ala Tyr Gln Gln Lys Leu Leu Asn Tyr Phe Ala Arg Asn
    5240                5245                5250

Phe Tyr Asn Met Arg Met Leu Ala Leu Phe Val Ala Phe Ala Ile
    5255                5260                5265

Asn Phe Ile Leu Leu Phe Tyr Lys Val Ser Thr Ser Ser Val Val
    5270                5275                5280

Glu Gly Lys Glu Leu Pro Thr Arg Ser Ser Ser Glu Asn Ala Lys
    5285                5290                5295

Val Thr Ser Leu Asp Ser Ser Ser His Arg Ile Ile Ala Val His
    5300                5305                5310

Tyr Val Leu Glu Glu Ser Ser Gly Tyr Met Glu Pro Thr Leu Arg
    5315                5320                5325

Ile Leu Ala Ile Leu His Thr Val Ile Ser Phe Phe Cys Ile Ile
    5330                5335                5340

Gly Tyr Tyr Cys Leu Lys Val Pro Leu Val Ile Phe Lys Arg Glu
    5345                5350                5355

Lys Glu Val Ala Arg Lys Leu Glu Phe Asp Gly Leu Tyr Ile Thr
    5360                5365                5370

Glu Gln Pro Ser Glu Asp Asp Ile Lys Gly Gln Trp Asp Arg Leu
    5375                5380                5385

Val Ile Asn Thr Gln Ser Phe Pro Asn Asn Tyr Trp Asp Lys Phe
    5390                5395                5400

Val Lys Arg Lys Val Met Asp Lys Tyr Gly Glu Phe Tyr Gly Arg
    5405                5410                5415

Asp Arg Ile Ser Glu Leu Leu Gly Met Asp Lys Ala Ala Leu Asp
    5420                5425                5430

Phe Ser Asp Ala Arg Glu Lys Lys Lys Pro Lys Lys Asp Ser Ser
    5435                5440                5445

Leu Ser Ala Val Leu Asn Ser Ile Asp Val Lys Tyr Gln Met Trp
    5450                5455                5460

Lys Leu Gly Val Val Phe Thr Asp Asn Ser Phe Leu Tyr Leu Ala
    5465                5470                5475

Trp Tyr Met Thr Met Ser Val Leu Gly His Tyr Asn Asn Phe Phe
    5480                5485                5490

Phe Ala Ala His Leu Leu Asp Ile Ala Met Gly Phe Lys Thr Leu
    5495                5500                5505

Arg Thr Ile Leu Ser Ser Val Thr His Asn Gly Lys Gln Leu Val
    5510                5515                5520

Leu Thr Val Gly Leu Leu Ala Val Val Val Tyr Leu Tyr Thr Val
    5525                5530                5535

Val Ala Phe Asn Phe Phe Arg Lys Phe Tyr Asn Lys Ser Glu Asp
    5540                5545                5550

Gly Asp Thr Pro Asp Met Lys Cys Asp Asp Met Leu Thr Cys Tyr
    5555                5560                5565

Met Phe His Met Tyr Val Gly Val Arg Ala Gly Gly Gly Ile Gly
    5570                5575                5580

Asp Glu Ile Glu Asp Pro Ala Gly Asp Glu Tyr Glu Ile Tyr Arg
    5585                5590                5595

Ile Ile Phe Asp Ile Thr Phe Phe Phe Phe Val Ile Val Ile Leu
    5600                5605                5610

Leu Ala Ile Ile Gln Gly Leu Ile Ile Asp Ala Phe Gly Glu Leu
    5615                5620                5625

Arg Asp Gln Gln Glu Gln Val Lys Glu Asp Met Glu Thr Lys Cys
    5630                5635                5640

Phe Ile Cys Gly Ile Gly Asn Asp Tyr Phe Asp Thr Val Pro His
    5645                5650                5655

Gly Phe Glu Thr His Thr Leu Gln Glu His Asn Leu Ala Asn Tyr
    5660                5665                5670

Leu Phe Phe Leu Met Tyr Leu Ile Asn Lys Asp Glu Thr Glu His
    5675                5680                5685

Thr Gly Gln Glu Ser Tyr Val Trp Lys Met Tyr Gln Glu Arg Cys
    5690                5695                5700

Trp Glu Phe Phe Pro Ala Gly Asp Cys Phe Arg Lys Gln Tyr Glu
    5705                5710                5715

Asp Gln Leu Asn
    5720

<210> 6
<211> 17169
<212> DNA
<213> artificial

<220>
<223>  nucleic acid of construct

<400>  6
atggattatg gaggcgccct gagtgccgtt gggcgcgagc tgctatttgt aacgaaccca      60

gtagtcgtca atggctctgt acttgtgcct gaggaccagt gttactgcgc gggctggatt     120

gagtcgcgtg gcacaaacgg tgcccaaacg gcgtcgaacg tgctgcaatg gcttgctgct     180

ggcttctcca tcctactgct tatgttttac gcctaccaaa catggaagtc aacctgcggc     240

tgggaggaga tctatgtgtg cgctatcgag atggtcaagg tgattctcga gttcttcttc     300

gagtttaaga acccgtccat gctgtatcta gccacaggcc accgcgtcca gtggttgcgt     360

tacgccgagt ggcttctcac ctgcccggtc atttgcattc gcctgtcaaa cctgacgggc     420

ttgtccaacg actacagcag gcgcaccatg ggtctgcttg tgtctgatat tggctgtatt     480

gtgtggggcg ccacttccgc catggccacc ggatacgtca aggtcatctt cttctgcctg     540

ggtctgtgtt atggtgctaa cacgttcttt cacgctgcca aggcctacat cgagggttac     600

cacaccgtgc cgaagggccg gtgtcgccag gtggtgactg gcatggcttg gctcttcttc     660

gtatcatggg gtatgttccc catcctgttc atcctcggcc ccgagggctt cggcgtcctg     720

agcgtgtacg gctccaccgt cggccacacc atcattgacc tgatgtcgaa gaactgctgg     780

ggtctgctcg gccactacct gcgcgtgctg atccacgagc atatcctcat ccacggcgac     840

attcgcaaga ccaccaaatt gaacattggt ggcactgaga ttgaggtcga gacgctggtg     900

gaggacgagg ccgaggctgg cgcggtaccc gcggccgcca ccatggtgag caagggcgag     960

gagctgttca ccggggtggt gcccatcctg gtcgagctgg acggcgacgt aaacggccac    1020

aagttcagcg tgtccggcga gggcgagggc gatgccacct acggcaagct gaccctgaag    1080

ttcatctgca ccaccggcaa gctgcccgtg ccctggccca ccctcgtgac caccttcggc    1140

tacggcctgc agtgcttcgc ccgctacccc gaccacatga agcagcacga cttcttcaag    1200

tccgccatgc ccgaaggcta cgtccaggag cgcaccatct tcttcaagga cgacggcaac    1260

tacaagaccc gcgccgaggt gaagttcgag ggcgacaccc tggtgaaccg catcgagctg    1320

aagggcatcg acttcaagga ggacggcaac atcctggggc acaagctgga gtacaactac    1380

aacagccaca acgtctatat catggccgac aagcagaaga acggcatcaa ggtgaacttc    1440

aagatccgcc acaacatcga ggacggcagc gtgcagctcg ccgaccacta ccagcagaac    1500

acccccatcg gcgacggccc cgtgctgctg cccgacaacc actacctgag ctaccagtcc    1560

gccctgagca aagaccccaa cgagaagcgc gatcacatgg tcctgctgga gttcgtgacc    1620

gccgccggga tcactctcgg catggacgag ctgtacaaga tgcccaactg gggcggaggc     1680

aagaaatgcg gcgtgtgtca gaaagccgtg tacttcgccg aagaggtgca gtgcgagggc    1740

agcagcttcc acaagagctg ctttctgtgc atggtctgca agaagaacct ggacagcacc    1800

accgtggcag tgcacggcga cgagatctac tgcaagtcct gctacggcaa gaagtacggc 1860

cccaaaggca aaggcaaagg catgggcgct ggcaccctga gcacagataa gggcgaaagc 1920

ctgggcatta agtacgagga aggccagagc cacagaccta caaaccccaa cgccagcaga 1980

atggcccaga aagtcggcgg ctctgacggc tgtcctagat gtggacaggc tgtgtacgcc 2040

gccgagaaag tgatcggcgc tggaaagagc tggcacaagt cttgcttcag atgcgccaag 2100

tgcggcaaga gcctggaaag cacaacactg gccgacaagg acggcgaaat ctattgcaag 2160

ggctgttacg ccaagaactt cggccctaaa ggcttcggct ttggccaagg tgctggcgcc 2220

ctgatccatt ctcagtcgag cggccgctct agaactagtg gaaccatggc cgatgggggc 2280

gagggcgaag acgagatcca gttcctgcga actgatgatg aagtggttct gcagtgcacc 2340

gcaaccatcc acaaagaaca acagaagcta tgcttggcag cagaaggatt tggcaacaga 2400

ctttgtttct tggagtccac ttccaattcc aagaatgtgc ccccagacct ctccatctgc 2460

acctttgtgc tggagcagtc cctctctgtc cgggcgctgc aggagatgct ggctaacacc 2520

gtggagaaat cagaaggcaa agttgatgtg gaaaaatgga aattcatgat gaagactgct 2580

caaggtggtg gtcatcgaac actcctctac ggacatgcca tattgctgcg ccattcctat 2640

agtggcatgt atctgtgctg cctgtccacc tcccggtctt caactgataa gctggctttt 2700

gatgttggct tgcaagagga caccacaggg gaggcttgtt ggtggaccat acaccctgcc 2760

tctaagcagc gatcagaagg agaaaaagta cgagttggag atgacctcat cttagttagc 2820

gtgtcctctg aaaggtactt gcacttgtct tatggcaacg gcagcttaca cgtggatgcc 2880

gctttccagc agactctctg gagcgtggcc ccaatcagct caggaagtga ggcagcccaa 2940

gggtatctca ttggtggtga tgtcctcagg ttgctgcatg gacacatgga cgagtgtctc 3000

actgtccctt caggagaaca tggtgaagag cagcggagaa ctgttcatta tgaaggtggc 3060

gctgtgtctg ttcatgcacg ttccctttgg agactagaga cgctaagagt tgcgtggagt 3120

ggaagccaca taagatgggg acagccattc cgactacgcc atgtcacaac aggaaaatac 3180

ttgagtctca tggaagacaa aaaccttcta ctcatggaca aagagaaagc tgatgtaaaa 3240

tcaacagcat ttaccttccg gtcttccaag gaaaaattgg atgtaggggt gagaaaagaa 3300

gtagatggca tgggaacatc tgaaataaaa tacggtgact cagtatgcta tatacaacat 3360

gtagacacag gcctatggct tacttaccag tctgtggacg tgaaatccgt gagaatggga 3420

tctatacaac gtaaggctat tatgcatcat gaaggccaca tggatgatgg cataagtttg 3480

tcgagatccc agcatgaaga atcacgcaca gcccgagtta tccggagcac agtcttcctt 3540

ttcaatagat ttataagggg ccttgatgct ctcagcaaga aagcgaaggc ttccacagtc 3600

gatttgccta tagagtccgt aagcctaagt ctgcaggatc tcattggcta cttccacccc 3660

ccagatgagc atttagagca tgaagacaaa cagaacagac tacgagccct gaagaatcgg 3720

caaaatctct tccaggaaga gggaatgatc aacctcgtgc ttgagtgcat agaccgtttg 3780

cacgtctaca gcagtgcagc acactttgct gatgttgctg ggcgagaagc aggagagtct 3840

tggaaatcca ttctgaattc tctgtatgag ttgctggcgg ctctaattag aggaaatcgt 3900

aaaaactgtg ctcaattttc tggctccctc gactggttga tcagcagatt ggaaagactg 3960

gaagcttctt caggcattct ggaagtttta cactgtgttt tagtagaaag tccagaagct 4020

ctaaatatta ttaaagaagg acatattaaa tctattatct cacttttaga caaacatgga 4080

agaaatcaca aggttctgga tgtcttgtgc tcactctgtg tttgccacgg ggttgcagtc 4140

cgttctaacc agcatctcat ctgtgacaat ctcctaccag gaagagactt gttattgcag 4200

acacgtcttg tgaaccatgt cagcagcatg agacccaata tttttctggg cgtcagtgaa 4260

ggttctgctc agtataagaa atggtactat gaattgatgg tggaccacac agagcccttt 4320

gtgacagctg aagcaactca cctgcgagtg ggctgggctt ccactgaagg atattctccc 4380

taccctggag ggggcgaaga gtggggtgga aatggtgttg gagatgatct cttctcctat 4440

ggatttgatg gccttcatct ctggtcaggt tgtattgctc gtactgtaag ctcaccaaac 4500

caacatctgt taagaactga tgatgtcatc agttgctgtt tagatctgag tgccccaagc 4560

atctcgttcc gaattaatgg acaacctgtt caaggaatgt ttgagaattt caacatcgat 4620

ggcctcttct ttccagtcgt tagtttctct gcaggaataa aagtacgctt tctgcttgga 4680

gggcgacatg gagaattcaa atttcttcct ccacctgggt atgctccttg ttatgaagct 4740

gttctgccaa aagaaaagtt gaaagtggaa cacagccgag agtacaagca agaaagaact 4800

tacacacgcg acctgctggg ccccacagtt tccctgacgc aagctgcctt cacacccatc 4860

cctgtggata ccagccagat cgtgttgcct cctcatctag aaagaataag agaaaaactg 4920

gcagagaata tccatgaact ctgggttatg aataaaattg agcttggctg gcagtatggt 4980

ccggttagag atgacaacaa gagacaacac ccatgcctgg tggagttctc caagctgcct 5040

gaacaggagc gcaattacaa cttacaaatg tcgcttgaga ccctgaagac tttgttggca 5100

ttaggatgtc atgtgggtat atcagatgaa catgctgaag acaaggtgaa aaaaatgaag 5160

ctacccaaga attaccagct gacaagtgga tacaagcctg cccctatgga cctgagcttt 5220

atcaaactca ccccatcgca agaagcaatg gtggacaagt tggcagaaaa tgcacataat 5280

gtgtgggcgc gggatcgaat ccggcagggc tggacttatg gcatccaaca ggacgtaaag 5340

aacagaagaa atcctcgcct tgttccctac actcttctgg atgaccgaac caagaaatcc 5400

aacaaggaca gcctccgcga ggctgtgcgc acgctgctgg ggtacggcta caacttggaa 5460

gcaccagatc aagatcatgc agccagagcc gaagtgtgca gcggcaccgg ggaaaggttc 5520

cgaatcttcc gtgccgagaa gacctatgca gtgaaggccg gacggtggta ttttgaattt 5580

gagacggtca ctgctggaga catgagggtt ggttggagtc gtcctggttg tcaaccggat 5640

caggagcttg gctcagatga acgtgccttt gcctttgatg gcttcaaggc ccagcggtgg 5700

catcagggca atgaacacta tgggcgctct tggcaagcag gcgatgtcgt ggggtgtatg 5760

gttgacatga acgaacacac catgatgttc acactgaatg gtgaaatcct tcttgatgat 5820

tcaggctcag aactggcttt caaggacttt gatgttggcg atggattcat acctgtgtgt 5880

agccttggag tggctcaagt gggtaggatg aactttggaa aggatgtcag caccttgaaa 5940

tatttcacca tctgtggctt acaagagggc tatgaaccat ttgccgttaa tacaaacagg 6000

gatattacca tgtggctgag caagaggctt cctcagtttc ttcaagttcc atcaaaccat 6060

gaacatatag aggtgaccag aatagacggc accatagaca gttccccatg tttaaaggtc 6120

actcagaagt cttttggttc tcagaacagc aacactgata tcatgtttta tcgcctgagc 6180

atgccgatcg agtgcgcgga ggtcttctcc aagacggtgg ctggagggct ccctggggct 6240

ggcctttttg ggcccaagaa tgacttggaa gattatgatg ctgattctga ctttgaggtt 6300

ctgatgaaga cagctcatgg ccatctagtg cccgatcgtg ttgacaaaga caaagaagct 6360

actaaaccag agtttaacaa ccacaaagat tatgcccagg aaaagccctc tcgtctgaaa 6420

caaagatttt tgcttagaag aacaaagcca gattacagca caagccattc tgcaagactc 6480

accgaagatg tccttgctga tgatcgggat gactatgatt tcttgatgca aacgtccacg 6540

tactattact cagtgagaat ctttcctgga caagaacctg ctaatgtctg ggtgggctgg 6600

attacatcag atttccatca gtatgacaca ggctttgact tggacagagt tcgcacagta 6660

acagttactc taggagatga aaaaggaaaa gtgcatgaaa gcatcaaacg cagcaactgc 6720

tatatggtat gtgcgggtga gagcatgagc cccgggcaag gacgcaacaa taatggactg 6780

gagattggct gtgtggtgga tgctgccagc gggctgctca cattcattgc caatggcaag 6840

gaactgagca catactatca ggtggaaccg agtacaaaat tatttcctgc ggtttttgca 6900

caagctacaa gtcccaatgt tttccagttt gagttgggaa gaataaagaa tgtgatgcct 6960

ctctcggcgg gattattcaa gagtgagcac aagaaccccg tgccgcagtg ccccccgcgc 7020

ctccacgtgc agttcctgtc acacgtcctg tggagcagaa tgcccaacca gtttttgaag 7080

gtagatgtgt ctcgaataag tgaacgccaa ggctggttgg tgcagtgttt ggatcctctg 7140

cagttcatgt ctcttcatat ccctgaggaa aacagatctg ttgacatctt agagttgaca 7200

gagcaggagg aattgctgaa atttcactat cacactctcc ggctctactc agccgtctgt 7260

gctcttggga accaccgggt ggcccatgcc ctgtgcagcc atgtggatga acctcagctc 7320

ctctatgcca ttgagaacaa gtacatgcct ggtttgctgc gtgctggcta ctatgacctg 7380

ctgattgaca tccacctgag ctcctatgcc actgccaggc tcatgatgaa caacgagtac 7440

attgtcccca tgacggagga gacgaagagc atcaccctgt tccctgatga gaacaaaaaa 7500

cacggccttc cagggatcgg cctcagcacc tccctcaggc cacggatgca gttttcctcc 7560

cccagttttg taagcattag taatgaatgt taccagtaca gtccagagtt cccactggac 7620

atcctcaagt ccaaaaccat acagatgctg acagaagctg ttaaagaggg cagtcttcat 7680

gcccgggacc cagttggagg gactactgaa ttcctctttg tacctctcat caagcttttc 7740

tataccctgc tgatcatggg catctttcac aacgaggact tgaagcacat cttgcagttg 7800

attgagccca gtgtgtttaa agaagctgcc actccggagg aggagagtga cacgctggag 7860

aaagagctca gtgtggacga tgcaaagctg caaggagctg gtgaggaaga agccaagggg 7920

ggcaagcggc ccaaggaagg cctgctccaa atgaaactgc cagagccagt taaattgcag 7980

atgtgcctac tgcttcagta cctctgtgac tgccaggtcc ggcaccggat agaagccatt 8040

gtagcctttt cagatgattt tgtggctaag ctccaagaca atcaacgttt ccgatacaac 8100

gaagtcatgc aagccttaaa catgtcagct gcactcacag ccaggaagac aaaggaattt 8160

agatcaccac ctcaagaaca gatcaatatg cttctcaatt ttaaggatga caaaagtgaa 8220

tgtccatgtc cagaagaaat tcgtgaccaa ctattggatt tccatgaaga tttgatgaca 8280

cattgtggaa ttgagctgga tgaagatggg tctctggatg gaaacagtga tttaacaatt 8340

agagggcgtc tgctatccct ggtagaaaag gtgacatatc tgaagaagaa gcaagcagaa 8400

aaaccagttg agagtgactc caaaaagtcc tccactctgc agcagctgat ttctgagacc 8460

atggtccgat gggctcagga gtctgtcatt gaagaccccg agctggtgag ggccatgttt 8520

gtgttgctcc atcggcagta tgacggcatt gggggtcttg ttcgggccct gccaaagacc 8580

tacacgataa atggtgtgtc cgtggaggac accatcaacc tgctggcatc ccttggtcag 8640

attcggtccc tgctgagtgt gagaatgggc aaagaagaag agaagctcat gattcgtgga 8700

ttaggggata ttatgaataa caaagtgttt taccagcacc ctaatctcat gagggcactg 8760

gggatgcacg agactgtgat ggaggtcatg gtgaacgtcc ttggaggtgg agagtccaag 8820

gaaatcacct ttcccaagat ggtggccaac tgttgccgtt ttctctgtta cttctgtcgt 8880

ataagtaggc agaatcaaaa agctatgttt gatcatctca gttatttact ggaaaacagc 8940

agtgttggtc ttgcctcccc agctatgaga ggttcaacac cactggatgt ggctgcagct 9000

tcggtgatgg ataataatga actagcatta gctctgcgtg agccggatct agaaaaggta 9060

gttcgttatt tggctggttg tggactgcaa agttgccaga tgctggtgtc taagggctat 9120

ccagacattg ggtggaaccc agttgaagga gagagatatc ttgactttct cagatttgct 9180

gtcttctgta atggggagag tgtggaggaa aatgcaaatg tcgtggtgag attgctcatt 9240

cggaggcctg agtgttttgg tcctgctttg agaggagaag gtgggaatgg gcttcttgca 9300

gcaatggaag aagccatcaa aatcgccgag gatccttccc gagatggtcc ctcaccaaat 9360

agcggatcca gtaaaacact tgacacagag gaggaggaag atgacactat ccacatgggg 9420

aacgcgatca tgaccttcta ttcagctttg attgacctct tgggacgctg tgctcctgag 9480

atgcatttga ttcatgccgg gaagggagaa gccatcagaa ttaggtccat tttgagatcc 9540

ctcattcccc tgggagattt ggtgggcgtt atcagcatcg cttttcagat gccaacaata 9600

gccaaagatg ggaatgtggt ggaacctgac atgtctgcgg ggttttgccc agatcacaag 9660

gcagccatgg ttttattcct tgacagggtc tatgggattg aggttcaaga cttcctcctc 9720

catcttcttg aggttggctt tctgccagat ctccgggcgg ctgcttcttt agatacggca 9780

gctttgagtg ctacagacat ggccttggcc ctcaatcggt acctttgcac agccgtcttg 9840

ccattgttaa caagatgtgc tcctctcttt gctggcacag agcaccacgc ttctctcatt 9900

gactcattac ttcatactgt gtatagactt tctaagggct gttcacttac caaagctcag 9960

cgggattcca tagaagtttg tttactctct atttgtggac aactgagacc ttctatgatg 10020

cagcacttac tcagaagatt agtatttgat gttccattat taaatgaaca cgcaaagatg 10080

cctcttaaac tgctgacaaa tcattatgaa agatgctgga aatattactg cctgcctgga 10140

gggtggggaa actttggtgc tgcctcagaa gaagaacttc atttatcaag aaagttgttc 10200

tggggcattt ttgatgccct gtctcaaaag aaatatgaac aagaactttt caaactggca 10260

ctgccttgcc tgagtgcagt tgcgggagct ttgcctccag actacatgga gtcaaattat 10320

gtcagtatga tggaaaaaca gtcatcaatg gattctgaag ggaactttaa cccacaacct 10380

gttgatacct caaatattac aattcctgag aaattggaat acttcattaa caaatatgca 10440

gaacactccc atgacaaatg gtcaatggac aagttggcaa atggatggat ttatggagaa 10500

atatattcag actcttctaa ggttcagcca ttaatgaagc catataagct attgtctgaa 10560

aaggaaaaag aaatttatcg ctggccaatc aaagaatctt taaaaactat gctggcttgg 10620

ggctggagaa ttgaaagaac tcgggaggga gacagcatgg ccctttacaa ccggactcgt 10680

cgtatttctc agacaagcca ggtttctgtg gacgctgccc atggttacag tccccgggcc 10740

attgacatga gcaatgttac actatctaga gacctgcatg ctatggcaga aatgatggct 10800

gaaaactacc ataatatatg ggcaaagaaa aagaaaatgg agttggagtc caaaggagga 10860

ggaaaccatc ctctgctggt gccctatgat acactgacag ccaaagagaa agccaaggat 10920

agagaaaaag cacaggacat cctcaagttc ttgcagatca atggatatgc tgtatccaga 10980

ggatttaagg acctggaact ggacacgcct tctattgaga aacgatttgc ctatagtttc 11040

ctccaacaac tcattcgcta tgtggatgaa gcccatcagt atatcctgga gtttgatggt 11100

ggcagcagag gcaaaggaga acatttccct tatgaacaag aaatcaagtt ctttgcaaaa 11160

gtcgttcttc ctttaattga tcagtatttc aaaaaccatc gtttatactt cttatctgca 11220

gcaagcagac ctctctgctc tggaggacat gcttccaaca aagagaaaga aatggtgact 11280

agcctattct gcaaacttgg agttcttgtc aggcatagga tttcactatt tggcaatgat 11340

gcaacatcaa ttgtcaactg tcttcatatt ttgggtcaga ctttggatgc aaggacagtg 11400

atgaagactg gcctggagag tgttaaaagt gcactcagag cttttctgga caacgctgca 11460

gaggatctgg agaagaccat ggaaaacctc aagcagggcc agttcactca cacccgaaac 11520

cagcccaaag gggttactca gattatcaat tacaccacag tggccctgct gccaatgctg 11580

tcgtcattat ttgaacatat tggccagcat cagttcggag aagacctaat attggaagat 11640

gtccaggtgt cttgttatag aattctgact agcttatatg ctttgggaac cagcaagagt 11700

atttacgtgg agaggcaacg ttctgcatta ggagaatgtc tagctgcctt tgctggtgct 11760

tttcctgtag catttttgga aactcatctg gacaaacata atatttactc catctacaat 11820

accaagtctt cacgagaaag agcagctctc agtttgccaa ctaatgtgga agatgtttgt 11880

ccaaacatac cgtctttgga gaaactcatg gaagaaatcg tggaattagc cgagtccggc 11940

attcgctaca ctcaaatgcc acatgtcatg gaagtcatac tgcccatgct ttgcagctac 12000

atgtctcgtt ggtgggagca tggacctgag aacaatccag aacgggccga gatgtgctgc 12060

acagccctga actcagagca catgaacaca cttctaggga acatattgaa aatcatatat 12120

aataacttgg ggattgatga gggagcctgg atgaagaggc tagcagtgtt ttcccagcct 12180

ataataaata aagtgaaacc tcagctcttg aaaactcatt tcttgccgtt aatggagaaa 12240

ctcaagaaaa aggcagctac ggtggtgtct gaggaagacc acctgaaagc tgaggccagg 12300

ggggacatgt cggaggcaga actcctcatc ctagatgagt tcaccacact ggccagagat 12360

ctctatgcct tctaccctct cttgattaga tttgtggact ataacagggc aaagtggcta 12420

aaggagccta acccagaagc agaggagctc ttccgcatgg tggctgaagt gtttatctac 12480

tggtcgaagt cccataattt caaaagagaa gagcagaact tcgttgtaca gaatgaaatc 12540

aacaatatgt ctttccttat tactgatacc aagtcaaaga tgtcaaaggc agctgtttct 12600

gatcaggaaa ggaagaaaat gaagcgcaaa ggagatcggt attccatgca gacctctctg 12660

attgtagcag ctctgaagcg gttactgccc attgggttga acatctgtgc ccctggggac 12720

caggagctca ttgctctggc caaaaatcga tttagcctga aagatactga ggatgaagta 12780

cgagatataa tccgcagcaa tattcattta caaggcaagt tggaggatcc tgctattaga 12840

tggcaaatgg ctctttacaa agacttacca aacaggactg atgatacctc agatccagag 12900

aagacggtag aaagagtatt ggatatagca aatgtgcttt ttcatcttga acagaagtct 12960

aaacgtgtgg gtcggagaca ttactgtctg gtggaacatc ctcagagatc taaaaaggct 13020

gtatggcata aactactgtc taagcagagg aaaagggctg ttgtagcctg cttccggatg 13080

gcccccttat ataatctgcc aaggcatcgg gctgtcaatc tctttcttca gggatatgaa 13140

aagtcttgga ttgaaacaga agaacattac tttgaagata aactgataga agatttagca 13200

aaacctgggg ctgaacctcc agaagaagat gaaggcacta agagagttga tcctctacat 13260

cagctgatcc ttctgtttag tcggacagct ttaacagaga aatgcaaact ggaggaagat 13320

tttttatata tggcctatgc agatattatg gcaaagagtt gtcatgatga ggaagatgac 13380

gatggtgaag aggaagtgaa gagttttgaa gaaaaagaaa tggaaaagca aaagcttcta 13440

taccagcaag cccgactcca cgatcgtggc gcggctgaga tggtgctaca gacaatcagt 13500

gccagcaaag gtgaaactgg accaatggta gcagctactc tgaaacttgg aattgctatt 13560

ttaaatggtg ggaactccac agtacagcag aaaatgcttg actacctcaa ggagaaaaag 13620

gatgtgggct tctttcagag cctggccggc ctgatgcagt catgtagtgt ccttgaccta 13680

aatgcatttg agcgacaaaa caaagctgaa ggtcttggga tggtgacaga ggaaggatca 13740

ggagaaaagg ttctgcagga cgatgagttc acctgtgacc tcttccgatt cctgcaacta 13800

ctctgtgagg gacacaactc agattttcag aattatctga gaactcagac tggcaataat 13860

acaactgtca acataattat ctccactgta gactacctac tgagagttca ggaatcaatt 13920

agtgactttt attggtatta ctctgggaaa gatgttattg atgaacaagg acaacggaat 13980

ttctccaaag ctatccaagt ggcaaaacaa gtctttaaca ctcttacaga gtatattcag 14040

ggtccttgca ctgggaatca acagagtttg gcacacagca ggctgtggga tgctgtggtc 14100

ggctttcttc atgtgtttgc ccatatgcag atgaagctgt cgcaggattc cagtcaaatt 14160

gagctattaa aagaattaat ggatctgcag aaggatatgg tggtcatgtt gctgtccatg 14220

ttagaaggta atgttgttaa tggaacgatt ggcaaacaga tggtggatat gcttgtggaa 14280

tcttccaaca acgtggagat gattctcaaa ttttttgaca tgttcttaaa actaaaggat 14340

ttgacgtcgt ctgatacttt taaagaatat gaccccgatg gcaagggagt catttccaag 14400

agggacttcc acaaagcgat ggagagccat aagcactaca cgcagtcaga aacggaattt 14460

cttttgtctt gtgcggagac ggatgagaat gaaaccctcg actacgaaga gttcgtcaaa 14520

cgcttccacg aacctgcgaa ggacatcggc ttcaacgtcg ccgtccttct gacaaacctc 14580

tctgagcaca tgcccaacga tacccgactt cagacttttc tggaattagc agagagcgtc 14640

ctgaattatt tccagccctt tctgggccgc atcgaaatca tgggaagcgc caaacgcatc 14700

gagagggtct attttgaaat cagtgagtcc agccgaaccc agtgggagaa gccccaggtc 14760

aaggagtcca aaagacagtt catatttgac gtggtcaacg aaggcggaga gaaagagaag 14820

atggaactct ttgtgaactt ctgcgaggac accatctttg aaatgcagct ggcggctcag 14880

atctcggagt cggacttgaa cgagaggtca gcgaataagg aagaaagcga gaaggagagg 14940

ccggaagagc aggggccgag gatggctttc ttctccattc tgacggtcag gtcggccctg 15000

tttgcgctca ggtacaatat cttgaccctt atgcgaatgc tcagtctgaa gagcctgaag 15060

aagcagatga aaaaagtaaa aaagatgacc gtgaaggaca tggtcacggc cttcttttca 15120

tcctactgga gtattttcat gaccctcttg cacttcgtgg ccagcgtttt cagaggcttt 15180

ttccgcatca tttgcagcct gctgcttggg ggaagcctcg tcgaaggtgc taaaaagatc 15240

aaagttgcag aactgttagc caacatgcca gaccccactc aggatgaggt tagaggagat 15300

ggggaggagg gagagaggaa acccctggaa gccgccctgc cctccgagga tctgaccgac 15360

ttaaaggagc tgacagagga aagtgacctt ctttcggaca tctttggcct ggatctgaag 15420

agagaaggag gacagtacaa actgattcct cataatccaa atgctgggct cagtgacctc 15480

atgagcaacc cagtccccat gcctgaggtg caggaaaaat ttcaggaaca gaaggcaaaa 15540

gaagaagaaa aggaagaaaa agaagaaacc aaatctgaac ctgaaaaagc cgagggagaa 15600

gatggagaaa aagaagagaa agccaaggaa gacaaggcca aacaaaagtt gaggcagctt 15660

cacacacaca gatacggaga accagaagtg ccagagtcag cattctggaa gaaaatcata 15720

gcatatcaac agaaacttct aaactatttt gctcgcaact tttacaacat gagaatgtta 15780

gccttatttg tcgcatttgc tatcaatttc atcttgctct tttataaggt ctccacttct 15840

tctgtggttg aaggaaagga gctccccacg agaagttcaa gtgaaaatgc caaagtgaca 15900

agcctggaca gcagctccca tagaatcatc gcagttcact atgtactaga ggagagcagc 15960

ggctacatgg agcccacgtt gcgtatctta gctattctgc acacggtcat ttctttcttc 16020

tgcatcattg gatactactg cttgaaagtc ccattggtta tttttaagcg agaaaaggaa 16080

gtggcacgga aattggaatt tgatggcttt tatattacag aacagccttc agaagatgat 16140

attaaaggcc agtgggatag actcgtaatc aacacacagt catttcccaa caactactgg 16200

gacaaatttg ttaaaagaaa ggttatggat aaatatggag agttctacgg ccgagacaga 16260

atcagtgaat tacttggcat ggacaaggca gctctggact tcagtgatgc cagagaaaag 16320

aagaagccaa agaaagacag ctccttatca gctgtactga actccattga tgtgaagtat 16380

cagatgtgga aactaggagt cgttttcact gacaactcct tcctctacct agcctggtat 16440

atgactatgt ctgttcttgg acactataac aacttttttt ttgccgctca ccttctcgac 16500

attgctatgg gattcaagac attaagaacc atcttgtcct cagtaactca caatggcaaa 16560

cagctcgtat taaccgttgg cttattagct gttgttgtat acctatacac tgtggtggca 16620

ttcaattttt tccgaaaatt ctacaataaa agtgaagatg gtgatacacc agatatgaaa 16680

tgtgacgata tgctaacatg ctatatgttc cacatgtatg ttggagttcg tgctggagga 16740

gggatcgggg atgaaatcga agacccagca ggagatgaat atgagatcta tcgaatcatc 16800

tttgacatca ctttcttctt ctttgttatt gtcattctct tggccataat acaaggtcta 16860

attattgatg cttttggaga actaagagac caacaggaac aagtcaaaga agacatggag 16920

accaaatgct tcatctgtgg gataggcaat gattacttcg acacagtgcc acatggcttt 16980

gaaacccaca ctttacagga gcacaacttg gctaattact tgttttttct gatgtatctt 17040

ataaacaaag atgaaacaga acacacagga caggaatctt atgtctggaa gatgtatcaa 17100

gaaaggtgtt gggaattttt cccagcaggg gattgcttcc ggaaacagta tgaagaccag 17160

ctaaattaa 17169

<210> 7
<211> 5483
<212> PRT
<213> artificial

<220>
<223> OpthoRyR2 no EYFP

<400> 7

Met Asp Tyr Gly Gly Ala Leu Ser Ala Val Gly Arg Glu Leu Leu Phe
1               5                   10                  15

Val Thr Asn Pro Val Val Val Asn Gly Ser Val Leu Val Pro Glu Asp
            20                  25                  30

Gln Cys Tyr Cys Ala Gly Trp Ile Glu Ser Arg Gly Thr Asn Gly Ala
        35                  40                  45

Gln Thr Ala Ser Asn Val Leu Gln Trp Leu Ala Ala Gly Phe Ser Ile
    50                  55                  60

Leu Leu Leu Met Phe Tyr Ala Tyr Gln Thr Trp Lys Ser Thr Cys Gly
65                  70                  75                  80

Trp Glu Glu Ile Tyr Val Cys Ala Ile Glu Met Val Lys Val Ile Leu
                85                  90                  95

Glu Phe Phe Phe Glu Phe Lys Asn Pro Ser Met Leu Tyr Leu Ala Thr
            100                 105                 110

Gly His Arg Val Gln Trp Leu Arg Tyr Ala Glu Trp Leu Leu Thr Cys
        115                 120                 125

Pro Val Ile Cys Ile Arg Leu Ser Asn Leu Thr Gly Leu Ser Asn Asp
    130                 135                 140

Tyr Ser Arg Arg Thr Met Gly Leu Leu Val Ser Asp Ile Gly Cys Ile
145                 150                 155                 160

Val Trp Gly Ala Thr Ser Ala Met Ala Thr Gly Tyr Val Lys Val Ile

                165                 170                 175

Phe Phe Cys Leu Gly Leu Cys Tyr Gly Ala Asn Thr Phe Phe His Ala
            180                 185                 190

Ala Lys Ala Tyr Ile Glu Gly Tyr His Thr Val Pro Lys Gly Arg Cys
        195                 200                 205

Arg Gln Val Val Thr Gly Met Ala Trp Leu Phe Phe Val Ser Trp Gly
    210                 215                 220

Met Phe Pro Ile Leu Phe Ile Leu Gly Pro Glu Gly Phe Gly Val Leu
225                 230                 235                 240

Ser Val Tyr Gly Ser Thr Val Gly His Thr Ile Ile Asp Leu Met Ser
                245                 250                 255

Lys Asn Cys Trp Gly Leu Leu Gly His Tyr Leu Arg Val Leu Ile His
            260                 265                 270

Glu His Ile Leu Ile His Gly Asp Ile Arg Lys Thr Thr Lys Leu Asn
        275                 280                 285

Ile Gly Gly Thr Glu Ile Glu Val Glu Thr Leu Val Glu Asp Glu Ala
    290                 295                 300

Glu Ala Gly Ala Val Pro Ala Ala Ala Thr Met Pro Asn Trp Gly Gly
305                 310                 315                 320

Gly Lys Lys Cys Gly Val Cys Gln Lys Ala Val Tyr Phe Ala Glu Glu
                325                 330                 335

Val Gln Cys Glu Gly Ser Ser Phe His Lys Ser Cys Phe Leu Cys Met
            340                 345                 350

Val Cys Lys Lys Asn Leu Asp Ser Thr Thr Val Ala Val His Gly Asp
        355                 360                 365

Glu Ile Tyr Cys Lys Ser Cys Tyr Gly Lys Lys Tyr Gly Pro Lys Gly
    370                 375                 380

Lys Gly Lys Gly Met Gly Ala Gly Thr Leu Ser Thr Asp Lys Gly Glu
385                 390                 395                 400

Ser Leu Gly Ile Lys Tyr Glu Glu Gly Gln Ser His Arg Pro Thr Asn
                405                 410                 415

Pro Asn Ala Ser Arg Met Ala Gln Lys Val Gly Gly Ser Asp Gly Cys
            420                 425                 430

Pro Arg Cys Gly Gln Ala Val Tyr Ala Ala Glu Lys Val Ile Gly Ala
        435                 440                 445

Gly Lys Ser Trp His Lys Ser Cys Phe Arg Cys Ala Lys Cys Gly Lys
    450                 455                 460

Ser Leu Glu Ser Thr Thr Leu Ala Asp Lys Asp Gly Glu Ile Tyr Cys
465                 470                 475                 480

Lys Gly Cys Tyr Ala Lys Asn Phe Gly Pro Lys Gly Phe Gly Phe Gly
                485                 490                 495

Gln Gly Ala Gly Ala Leu Ile His Ser Gln Ser Ser Gly Arg Ser Arg
            500                 505                 510

Thr Ser Gly Thr Met Ala Asp Gly Gly Glu Gly Glu Asp Glu Ile Gln
        515                 520                 525

Phe Leu Arg Thr Asp Asp Glu Val Val Leu Gln Cys Thr Ala Thr Ile
    530                 535                 540

His Lys Glu Gln Gln Lys Leu Cys Leu Ala Ala Glu Gly Phe Gly Asn
545                 550                 555                 560

Arg Leu Cys Phe Leu Glu Ser Thr Ser Asn Ser Lys Asn Val Pro Pro
                565                 570                 575

Asp Leu Ser Ile Cys Thr Phe Val Leu Glu Gln Ser Leu Ser Val Arg
            580                 585                 590

Ala Leu Gln Glu Met Leu Ala Asn Thr Val Glu Lys Ser Glu Gly Gln
        595                 600                 605

Val Asp Val Glu Lys Trp Lys Phe Met Met Lys Thr Ala Gln Gly Gly
    610                 615                 620

Gly His Arg Thr Leu Leu Tyr Gly His Ala Ile Leu Leu Arg His Ser
625                 630                 635                 640

Tyr Ser Gly Met Tyr Leu Cys Cys Leu Ser Thr Ser Arg Ser Ser Thr
                645                 650                 655

Asp Lys Leu Ala Phe Asp Val Gly Leu Gln Glu Asp Thr Thr Gly Glu
            660                 665                 670

Ala Cys Trp Trp Thr Ile His Pro Ala Ser Lys Gln Arg Ser Glu Gly
        675                 680                 685

Glu Lys Val Arg Val Gly Asp Asp Leu Ile Leu Val Ser Val Ser Ser
    690                 695                 700

Glu Arg Tyr Leu His Leu Ser Tyr Gly Asn Gly Ser Leu His Val Asp
705                 710                 715                 720

Ala Ala Phe Gln Gln Thr Leu Trp Ser Val Ala Pro Ile Ser Ser Gly
                725                 730                 735

Ser Glu Ala Ala Gln Gly Tyr Leu Ile Gly Gly Asp Val Leu Arg Leu
            740                 745                 750

Leu His Gly His Met Asp Glu Cys Leu Thr Val Pro Ser Gly Glu His
        755                 760                 765

Gly Glu Glu Gln Arg Arg Thr Val His Tyr Glu Gly Gly Ala Val Ser
    770                 775                 780

Val His Ala Arg Ser Leu Trp Arg Leu Glu Thr Leu Arg Val Ala Trp
785                 790                 795                 800

Ser Gly Ser His Ile Arg Trp Gly Gln Pro Phe Arg Leu Arg His Val
                805                 810                 815

Thr Thr Gly Lys Tyr Leu Ser Leu Met Glu Asp Lys Asn Leu Leu Leu
            820                 825                 830

Met Asp Lys Glu Lys Ala Asp Val Lys Ser Thr Ala Phe Thr Phe Arg
        835                 840                 845

Ser Ser Lys Glu Lys Leu Asp Val Gly Val Arg Lys Glu Val Asp Gly
    850                 855                 860

Met Gly Thr Ser Glu Ile Lys Tyr Gly Asp Ser Val Cys Tyr Ile Gln
865                 870                 875                 880

His Val Asp Thr Gly Leu Trp Leu Thr Tyr Gln Ser Val Asp Val Lys
                885                 890                 895

Ser Val Arg Met Gly Ser Ile Gln Arg Lys Ala Ile Met His His Glu
            900                 905                 910

Gly His Met Asp Asp Gly Ile Ser Leu Ser Arg Ser Gln His Glu Glu
        915                 920                 925

Ser Arg Thr Ala Arg Val Ile Arg Ser Thr Val Phe Leu Phe Asn Arg
    930                 935                 940

Phe Ile Arg Gly Leu Asp Ala Leu Ser Lys Lys Ala Lys Ala Ser Thr
945                 950                 955                 960

Val Asp Leu Pro Ile Glu Ser Val Ser Leu Ser Leu Gln Asp Leu Ile
                965                 970                 975

Gly Tyr Phe His Pro Pro Asp Glu His Leu Glu His Glu Asp Lys Gln
            980                 985                 990

Asn Arg Leu Arg Ala Leu Lys Asn  Arg Gln Asn Leu Phe  Gln Glu Glu
        995                 1000                 1005

Gly Met  Ile Asn Leu Val Leu  Glu Cys Ile Asp Arg  Leu His Val
    1010                 1015                 1020

Tyr Ser  Ser Ala Ala His Phe  Ala Asp Val Ala Gly  Arg Glu Ala
    1025                 1030                 1035

Gly Glu  Ser Trp Lys Ser Ile  Leu Asn Ser Leu Tyr  Glu Leu Leu
    1040                 1045                 1050

Ala Ala  Leu Ile Arg Gly Asn  Arg Lys Asn Cys Ala  Gln Phe Ser
    1055                 1060                 1065

Gly Ser  Leu Asp Trp Leu Ile  Ser Arg Leu Glu Arg  Leu Glu Ala
    1070                 1075                 1080

Ser Ser  Gly Ile Leu Glu Val  Leu His Cys Val Leu  Val Glu Ser
    1085                 1090                 1095

Pro Glu  Ala Leu Asn Ile Ile  Lys Glu Gly His Ile  Lys Ser Ile
    1100                 1105                 1110

Ile Ser  Leu Leu Asp Lys His  Gly Arg Asn His Lys  Val Leu Asp
    1115                 1120                 1125

Val Leu  Cys Ser Leu Cys Val  Cys His Gly Val Ala  Val Arg Ser
    1130                 1135                 1140

Asn Gln  His Leu Ile Cys Asp  Asn Leu Leu Pro Gly  Arg Asp Leu
    1145                 1150                 1155

Leu Leu  Gln Thr Arg Leu Val  Asn His Val Ser Ser  Met Arg Pro

1160                1165                1170

Asn Ile Phe Leu Gly Val Ser Glu Gly Ser Ala Gln Tyr Lys Lys
    1175                1180                1185

Trp Tyr Tyr Glu Leu Met Val Asp His Thr Glu Pro Phe Val Thr
    1190                1195                1200

Ala Glu Ala Thr His Leu Arg Val Gly Trp Ala Ser Thr Glu Gly
    1205                1210                1215

Tyr Ser Pro Tyr Pro Gly Gly Gly Glu Glu Trp Gly Gly Asn Gly
    1220                1225                1230

Val Gly Asp Asp Leu Phe Ser Tyr Gly Phe Asp Gly Leu His Leu
    1235                1240                1245

Trp Ser Gly Cys Ile Ala Arg Thr Val Ser Ser Pro Asn Gln His
    1250                1255                1260

Leu Leu Arg Thr Asp Asp Val Ile Ser Cys Cys Leu Asp Leu Ser
    1265                1270                1275

Ala Pro Ser Ile Ser Phe Arg Ile Asn Gly Gln Pro Val Gln Gly
    1280                1285                1290

Met Phe Glu Asn Phe Asn Ile Asp Gly Leu Phe Phe Pro Val Val
    1295                1300                1305

Ser Phe Ser Ala Gly Ile Lys Val Arg Phe Leu Leu Gly Gly Arg
    1310                1315                1320

His Gly Glu Phe Lys Phe Leu Pro Pro Pro Gly Tyr Ala Pro Cys
    1325                1330                1335

Tyr Glu Ala Val Leu Pro Lys Glu Lys Leu Lys Val Glu His Ser
    1340                1345                1350

Arg Glu Tyr Lys Gln Glu Arg Thr Tyr Thr Arg Asp Leu Leu Gly
    1355                1360                1365

Pro Thr Val Ser Leu Thr Gln Ala Ala Phe Thr Pro Ile Pro Val
    1370                1375                1380

Asp Thr Ser Gln Ile Val Leu Pro Pro His Leu Glu Arg Ile Arg
    1385                1390                1395

Glu Lys Leu Ala Glu Asn Ile His Glu Leu Trp Val Met Asn Lys
    1400                1405                1410

Ile Glu Leu Gly Trp Gln Tyr Gly Pro Val Arg Asp Asp Asn Lys
    1415                1420                1425

Arg Gln His Pro Cys Leu Val Glu Phe Ser Lys Leu Pro Glu Gln
    1430                1435                1440

Glu Arg Asn Tyr Asn Leu Gln Met Ser Leu Glu Thr Leu Lys Thr
    1445                1450                1455

Leu Leu Ala Leu Gly Cys His Val Gly Ile Ser Asp Glu His Ala
    1460                1465                1470

Glu Asp Lys Val Lys Lys Met Lys Leu Pro Lys Asn Tyr Gln Leu
    1475                1480                1485

Thr Ser Gly Tyr Lys Pro Ala Pro Met Asp Leu Ser Phe Ile Lys
    1490                1495                1500

Leu Thr Pro Ser Gln Glu Ala Met Val Asp Lys Leu Ala Glu Asn
    1505                1510                1515

Ala His Asn Val Trp Ala Arg Asp Arg Ile Arg Gln Gly Trp Thr
    1520                1525                1530

Tyr Gly Ile Gln Gln Asp Val Lys Asn Arg Arg Asn Pro Arg Leu
    1535                1540                1545

Val Pro Tyr Thr Leu Leu Asp Asp Arg Thr Lys Lys Ser Asn Lys
    1550                1555                1560

Asp Ser Leu Arg Glu Ala Val Arg Thr Leu Leu Gly Tyr Gly Tyr
    1565                1570                1575

Asn Leu Glu Ala Pro Asp Gln Asp His Ala Ala Arg Ala Glu Val
    1580                1585                1590

Cys Ser Gly Thr Gly Glu Arg Phe Arg Ile Phe Arg Ala Glu Lys
    1595                1600                1605

Thr Tyr Ala Val Lys Ala Gly Arg Trp Tyr Phe Glu Phe Glu Thr
    1610                1615                1620

Val Thr Ala Gly Asp Met Arg Val Gly Trp Ser Arg Pro Gly Cys
    1625                1630                1635

Gln Pro Asp Gln Glu Leu Gly Ser Asp Glu Arg Ala Phe Ala Phe
    1640                1645                1650

Asp Gly Phe Lys Ala Gln Arg Trp His Gln Gly Asn Glu His Tyr
    1655                1660                1665

Gly Arg Ser Trp Gln Ala Gly Asp Val Val Gly Cys Met Val Asp
    1670                1675                1680

Met Asn Glu His Thr Met Met Phe Thr Leu Asn Gly Glu Ile Leu
    1685                1690                1695

Leu Asp Asp Ser Gly Ser Glu Leu Ala Phe Lys Asp Phe Asp Val
    1700                1705                1710

Gly Asp Gly Phe Ile Pro Val Cys Ser Leu Gly Val Ala Gln Val
    1715                1720                1725

Gly Arg Met Asn Phe Gly Lys Asp Val Ser Thr Leu Lys Tyr Phe
    1730                1735                1740

Thr Ile Cys Gly Leu Gln Glu Gly Tyr Glu Pro Phe Ala Val Asn
    1745                1750                1755

Thr Asn Arg Asp Ile Thr Met Trp Leu Ser Lys Arg Leu Pro Gln
    1760                1765                1770

Phe Leu Gln Val Pro Ser Asn His Glu His Ile Glu Val Thr Arg
    1775                1780                1785

Ile Asp Gly Thr Ile Asp Ser Ser Pro Cys Leu Lys Val Thr Gln
    1790                1795                1800

Lys Ser Phe Gly Ser Gln Asn Ser Asn Thr Asp Ile Met Phe Tyr
    1805                1810                1815

Arg Leu Ser Met Pro Ile Glu Cys Ala Glu Val Phe Ser Lys Thr
    1820                1825                1830

Val Ala Gly Gly Leu Pro Gly Ala Gly Leu Phe Gly Pro Lys Asn
    1835                1840                1845

Asp Leu Glu Asp Tyr Asp Ala Asp Ser Asp Phe Glu Val Leu Met
    1850                1855                1860

Lys Thr Ala His Gly His Leu Val Pro Asp Arg Val Asp Lys Asp
    1865                1870                1875

Lys Glu Ala Thr Lys Pro Glu Phe Asn Asn His Lys Asp Tyr Ala
    1880                1885                1890

Gln Glu Lys Pro Ser Arg Leu Lys Gln Arg Phe Leu Leu Arg Arg
    1895                1900                1905

Thr Lys Pro Asp Tyr Ser Thr Ser His Ser Ala Arg Leu Thr Glu
    1910                1915                1920

Asp Val Leu Ala Asp Asp Arg Asp Asp Tyr Asp Phe Leu Met Gln
    1925                1930                1935

Thr Ser Thr Tyr Tyr Tyr Ser Val Arg Ile Phe Pro Gly Gln Glu
    1940                1945                1950

Pro Ala Asn Val Trp Val Gly Trp Ile Thr Ser Asp Phe His Gln
    1955                1960                1965

Tyr Asp Thr Gly Phe Asp Leu Asp Arg Val Arg Thr Val Thr Val
    1970                1975                1980

Thr Leu Gly Asp Glu Lys Gly Lys Val His Glu Ser Ile Lys Arg
    1985                1990                1995

Ser Asn Cys Tyr Met Val Cys Ala Gly Glu Ser Met Ser Pro Gly
    2000                2005                2010

Gln Gly Arg Asn Asn Asn Gly Leu Glu Ile Gly Cys Val Val Asp
    2015                2020                2025

Ala Ala Ser Gly Leu Leu Thr Phe Ile Ala Asn Gly Lys Glu Leu
    2030                2035                2040

Ser Thr Tyr Tyr Gln Val Glu Pro Ser Thr Lys Leu Phe Pro Ala
    2045                2050                2055

Val Phe Ala Gln Ala Thr Ser Pro Asn Val Phe Gln Phe Glu Leu
    2060                2065                2070

Gly Arg Ile Lys Asn Val Met Pro Leu Ser Ala Gly Leu Phe Lys
    2075                2080                2085

Ser Glu His Lys Asn Pro Val Pro Gln Cys Pro Pro Arg Leu His
    2090                2095                2100

Val Gln Phe Leu Ser His Val Leu Trp Ser Arg Met Pro Asn Gln

2105                2110                2115

Phe Leu Lys Val Asp Val Ser Arg Ile Ser Glu Arg Gln Gly Trp
    2120                2125                2130

Leu Val Gln Cys Leu Asp Pro Leu Gln Phe Met Ser Leu His Ile
    2135                2140                2145

Pro Glu Glu Asn Arg Ser Val Asp Ile Leu Glu Leu Thr Glu Gln
    2150                2155                2160

Glu Glu Leu Leu Lys Phe His Tyr His Thr Leu Arg Leu Tyr Ser
    2165                2170                2175

Ala Val Cys Ala Leu Gly Asn His Arg Val Ala His Ala Leu Cys
    2180                2185                2190

Ser His Val Asp Glu Pro Gln Leu Leu Tyr Ala Ile Glu Asn Lys
    2195                2200                2205

Tyr Met Pro Gly Leu Leu Arg Ala Gly Tyr Tyr Asp Leu Leu Ile
    2210                2215                2220

Asp Ile His Leu Ser Ser Tyr Ala Thr Ala Arg Leu Met Met Asn
    2225                2230                2235

Asn Glu Tyr Ile Val Pro Met Thr Glu Glu Thr Lys Ser Ile Thr
    2240                2245                2250

Leu Phe Pro Asp Glu Asn Lys Lys His Gly Leu Pro Gly Ile Gly
    2255                2260                2265

Leu Ser Thr Ser Leu Arg Pro Arg Met Gln Phe Ser Ser Pro Ser
    2270                2275                2280

Phe Val Ser Ile Ser Asn Glu Cys Tyr Gln Tyr Ser Pro Glu Phe
    2285                2290                2295

Pro Leu Asp Ile Leu Lys Ser Lys Thr Ile Gln Met Leu Thr Glu
    2300                2305                2310

Ala Val Lys Glu Gly Ser Leu His Ala Arg Asp Pro Val Gly Gly
    2315                2320                2325

Thr Thr Glu Phe Leu Phe Val Pro Leu Ile Lys Leu Phe Tyr Thr
    2330                2335                2340

Leu Leu Ile Met Gly Ile Phe His Asn Glu Asp Leu Lys His Ile
    2345                2350                2355

Leu Gln Leu Ile Glu Pro Ser Val Phe Lys Glu Ala Ala Thr Pro
    2360                2365                2370

Glu Glu Glu Ser Asp Thr Leu Glu Lys Glu Leu Ser Val Asp Asp
    2375                2380                2385

Ala Lys Leu Gln Gly Ala Gly Glu Glu Glu Ala Lys Gly Gly Lys
    2390                2395                2400

Arg Pro Lys Glu Gly Leu Leu Gln Met Lys Leu Pro Glu Pro Val
    2405                2410                2415

Lys Leu Gln Met Cys Leu Leu Leu Gln Tyr Leu Cys Asp Cys Gln
    2420                2425                2430

Val Arg His Arg Ile Glu Ala Ile Val Ala Phe Ser Asp Asp Phe
    2435                2440                2445

Val Ala Lys Leu Gln Asp Asn Gln Arg Phe Arg Tyr Asn Glu Val
    2450                2455                2460

Met Gln Ala Leu Asn Met Ser Ala Ala Leu Thr Ala Arg Lys Thr
    2465                2470                2475

Lys Glu Phe Arg Ser Pro Pro Gln Glu Gln Ile Asn Met Leu Leu
    2480                2485                2490

Asn Phe Lys Asp Asp Lys Ser Glu Cys Pro Cys Pro Glu Glu Ile
    2495                2500                2505

Arg Asp Gln Leu Leu Asp Phe His Glu Asp Leu Met Thr His Cys
    2510                2515                2520

Gly Ile Glu Leu Asp Glu Asp Gly Ser Leu Asp Gly Asn Ser Asp
    2525                2530                2535

Leu Thr Ile Arg Gly Arg Leu Leu Ser Leu Val Glu Lys Val Thr
    2540                2545                2550

Tyr Leu Lys Lys Lys Gln Ala Glu Lys Pro Val Glu Ser Asp Ser
    2555                2560                2565

Lys Lys Ser Ser Thr Leu Gln Gln Leu Ile Ser Glu Thr Met Val
    2570                2575                2580

Arg Trp Ala Gln Glu Ser Val Ile Glu Asp Pro Glu Leu Val Arg
    2585                2590                2595

Ala Met Phe Val Leu Leu His Arg Gln Tyr Asp Gly Ile Gly Gly
    2600                2605                2610

Leu Val Arg Ala Leu Pro Lys Thr Tyr Thr Ile Asn Gly Val Ser
    2615                2620                2625

Val Glu Asp Thr Ile Asn Leu Leu Ala Ser Leu Gly Gln Ile Arg
    2630                2635                2640

Ser Leu Leu Ser Val Arg Met Gly Lys Glu Glu Glu Lys Leu Met
    2645                2650                2655

Ile Arg Gly Leu Gly Asp Ile Met Asn Asn Lys Val Phe Tyr Gln
    2660                2665                2670

His Pro Asn Leu Met Arg Ala Leu Gly Met His Glu Thr Val Met
    2675                2680                2685

Glu Val Met Val Asn Val Leu Gly Gly Gly Glu Ser Lys Glu Ile
    2690                2695                2700

Thr Phe Pro Lys Met Val Ala Asn Cys Cys Arg Phe Leu Cys Tyr
    2705                2710                2715

Phe Cys Arg Ile Ser Arg Gln Asn Gln Lys Ala Met Phe Asp His
    2720                2725                2730

Leu Ser Tyr Leu Leu Glu Asn Ser Ser Val Gly Leu Ala Ser Pro
    2735                2740                2745

Ala Met Arg Gly Ser Thr Pro Leu Asp Val Ala Ala Ala Ser Val
    2750                2755                2760

Met Asp Asn Asn Glu Leu Ala Leu Ala Leu Arg Glu Pro Asp Leu
    2765                2770                2775

Glu Lys Val Val Arg Tyr Leu Ala Gly Cys Gly Leu Gln Ser Cys
    2780                2785                2790

Gln Met Leu Val Ser Lys Gly Tyr Pro Asp Ile Gly Trp Asn Pro
    2795                2800                2805

Val Glu Gly Glu Arg Tyr Leu Asp Phe Leu Arg Phe Ala Val Phe
    2810                2815                2820

Cys Asn Gly Glu Ser Val Glu Glu Asn Ala Asn Val Val Val Arg
    2825                2830                2835

Leu Leu Ile Arg Arg Pro Glu Cys Phe Gly Pro Ala Leu Arg Gly
    2840                2845                2850

Glu Gly Gly Asn Gly Leu Leu Ala Ala Met Glu Glu Ala Ile Lys
    2855                2860                2865

Ile Ala Glu Asp Pro Ser Arg Asp Gly Pro Ser Pro Asn Ser Gly
    2870                2875                2880

Ser Ser Lys Thr Leu Asp Thr Glu Glu Glu Glu Asp Asp Thr Ile
    2885                2890                2895

His Met Gly Asn Ala Ile Met Thr Phe Tyr Ser Ala Leu Ile Asp
    2900                2905                2910

Leu Leu Gly Arg Cys Ala Pro Glu Met His Leu Ile His Ala Gly
    2915                2920                2925

Lys Gly Glu Ala Ile Arg Ile Arg Ser Ile Leu Arg Ser Leu Ile
    2930                2935                2940

Pro Leu Gly Asp Leu Val Gly Val Ile Ser Ile Ala Phe Gln Met
    2945                2950                2955

Pro Thr Ile Ala Lys Asp Gly Asn Val Val Glu Pro Asp Met Ser
    2960                2965                2970

Ala Gly Phe Cys Pro Asp His Lys Ala Ala Met Val Leu Phe Leu
    2975                2980                2985

Asp Arg Val Tyr Gly Ile Glu Val Gln Asp Phe Leu Leu His Leu
    2990                2995                3000

Leu Glu Val Gly Phe Leu Pro Asp Leu Arg Ala Ala Ala Ser Leu
    3005                3010                3015

Asp Thr Ala Ala Leu Ser Ala Thr Asp Met Ala Leu Ala Leu Asn
    3020                3025                3030

Arg Tyr Leu Cys Thr Ala Val Leu Pro Leu Leu Thr Arg Cys Ala
    3035                3040                3045

Pro Leu Phe Ala Gly Thr Glu His His Ala Ser Leu Ile Asp Ser

3050                3055                3060

Leu Leu His Thr Val Tyr Arg Leu Ser Lys Gly Cys Ser Leu Thr
    3065                3070                3075

Lys Ala Gln Arg Asp Ser Ile Glu Val Cys Leu Leu Ser Ile Cys
    3080                3085                3090

Gly Gln Leu Arg Pro Ser Met Met Gln His Leu Leu Arg Arg Leu
    3095                3100                3105

Val Phe Asp Val Pro Leu Leu Asn Glu His Ala Lys Met Pro Leu
    3110                3115                3120

Lys Leu Leu Thr Asn His Tyr Glu Arg Cys Trp Lys Tyr Tyr Cys
    3125                3130                3135

Leu Pro Gly Gly Trp Gly Asn Phe Gly Ala Ala Ser Glu Glu Glu
    3140                3145                3150

Leu His Leu Ser Arg Lys Leu Phe Trp Gly Ile Phe Asp Ala Leu
    3155                3160                3165

Ser Gln Lys Lys Tyr Glu Gln Glu Leu Phe Lys Leu Ala Leu Pro
    3170                3175                3180

Cys Leu Ser Ala Val Ala Gly Ala Leu Pro Pro Asp Tyr Met Glu
    3185                3190                3195

Ser Asn Tyr Val Ser Met Met Glu Lys Gln Ser Ser Met Asp Ser
    3200                3205                3210

Glu Gly Asn Phe Asn Pro Gln Pro Val Asp Thr Ser Asn Ile Thr
    3215                3220                3225

Ile Pro Glu Lys Leu Glu Tyr Phe Ile Asn Lys Tyr Ala Glu His
    3230                3235                3240

Ser His Asp Lys Trp Ser Met Asp Lys Leu Ala Asn Gly Trp Ile
    3245                3250                3255

Tyr Gly Glu Ile Tyr Ser Asp Ser Ser Lys Val Gln Pro Leu Met
    3260                3265                3270

Lys Pro Tyr Lys Leu Leu Ser Glu Lys Glu Lys Glu Ile Tyr Arg
    3275                3280                3285

Trp Pro Ile Lys Glu Ser Leu Lys Thr Met Leu Ala Trp Gly Trp
    3290                3295                3300

Arg Ile Glu Arg Thr Arg Glu Gly Asp Ser Met Ala Leu Tyr Asn
    3305                3310                3315

Arg Thr Arg Arg Ile Ser Gln Thr Ser Gln Val Ser Val Asp Ala
    3320                3325                3330

Ala His Gly Tyr Ser Pro Arg Ala Ile Asp Met Ser Asn Val Thr
    3335                3340                3345

Leu Ser Arg Asp Leu His Ala Met Ala Glu Met Met Ala Glu Asn
    3350                3355                3360

Tyr His Asn Ile Trp Ala Lys Lys Lys Lys Met Glu Leu Glu Ser
    3365                3370                3375

Lys Gly Gly Gly Asn His Pro Leu Leu Val Pro Tyr Asp Thr Leu
    3380                3385                3390

Thr Ala Lys Glu Lys Ala Lys Asp Arg Glu Lys Ala Gln Asp Ile
    3395                3400                3405

Leu Lys Phe Leu Gln Ile Asn Gly Tyr Ala Val Ser Arg Gly Phe
    3410                3415                3420

Lys Asp Leu Glu Leu Asp Thr Pro Ser Ile Glu Lys Arg Phe Ala
    3425                3430                3435

Tyr Ser Phe Leu Gln Gln Leu Ile Arg Tyr Val Asp Glu Ala His
    3440                3445                3450

Gln Tyr Ile Leu Glu Phe Asp Gly Gly Ser Arg Gly Lys Gly Glu
    3455                3460                3465

His Phe Pro Tyr Glu Gln Glu Ile Lys Phe Phe Ala Lys Val Val
    3470                3475                3480

Leu Pro Leu Ile Asp Gln Tyr Phe Lys Asn His Arg Leu Tyr Phe
    3485                3490                3495

Leu Ser Ala Ala Ser Arg Pro Leu Cys Ser Gly Gly His Ala Ser
    3500                3505                3510

Asn Lys Glu Lys Glu Met Val Thr Ser Leu Phe Cys Lys Leu Gly
    3515                3520                3525

Val Leu Val Arg His Arg Ile Ser Leu Phe Gly Asn Asp Ala Thr
    3530                3535                3540

Ser Ile Val Asn Cys Leu His Ile Leu Gly Gln Thr Leu Asp Ala
    3545                3550                3555

Arg Thr Val Met Lys Thr Gly Leu Glu Ser Val Lys Ser Ala Leu
    3560                3565                3570

Arg Ala Phe Leu Asp Asn Ala Ala Glu Asp Leu Glu Lys Thr Met
    3575                3580                3585

Glu Asn Leu Lys Gln Gly Gln Phe Thr His Thr Arg Asn Gln Pro
    3590                3595                3600

Lys Gly Val Thr Gln Ile Ile Asn Tyr Thr Thr Val Ala Leu Leu
    3605                3610                3615

Pro Met Leu Ser Ser Leu Phe Glu His Ile Gly Gln His Gln Phe
    3620                3625                3630

Gly Glu Asp Leu Ile Leu Glu Asp Val Gln Val Ser Cys Tyr Arg
    3635                3640                3645

Ile Leu Thr Ser Leu Tyr Ala Leu Gly Thr Ser Lys Ser Ile Tyr
    3650                3655                3660

Val Glu Arg Gln Arg Ser Ala Leu Gly Glu Cys Leu Ala Ala Phe
    3665                3670                3675

Ala Gly Ala Phe Pro Val Ala Phe Leu Glu Thr His Leu Asp Lys
    3680                3685                3690

His Asn Ile Tyr Ser Ile Tyr Asn Thr Lys Ser Ser Arg Glu Arg
    3695                3700                3705

Ala Ala Leu Ser Leu Pro Thr Asn Val Glu Asp Val Cys Pro Asn
    3710                3715                3720

Ile Pro Ser Leu Glu Lys Leu Met Glu Glu Ile Val Glu Leu Ala
    3725                3730                3735

Glu Ser Gly Ile Arg Tyr Thr Gln Met Pro His Val Met Glu Val
    3740                3745                3750

Ile Leu Pro Met Leu Cys Ser Tyr Met Ser Arg Trp Trp Glu His
    3755                3760                3765

Gly Pro Glu Asn Asn Pro Glu Arg Ala Glu Met Cys Cys Thr Ala
    3770                3775                3780

Leu Asn Ser Glu His Met Asn Thr Leu Leu Gly Asn Ile Leu Lys
    3785                3790                3795

Ile Ile Tyr Asn Asn Leu Gly Ile Asp Glu Gly Ala Trp Met Lys
    3800                3805                3810

Arg Leu Ala Val Phe Ser Gln Pro Ile Ile Asn Lys Val Lys Pro
    3815                3820                3825

Gln Leu Leu Lys Thr His Phe Leu Pro Leu Met Glu Lys Leu Lys
    3830                3835                3840

Lys Lys Ala Ala Thr Val Val Ser Glu Glu Asp His Leu Lys Ala
    3845                3850                3855

Glu Ala Arg Gly Asp Met Ser Glu Ala Glu Leu Leu Ile Leu Asp
    3860                3865                3870

Glu Phe Thr Thr Leu Ala Arg Asp Leu Tyr Ala Phe Tyr Pro Leu
    3875                3880                3885

Leu Ile Arg Phe Val Asp Tyr Asn Arg Ala Lys Trp Leu Lys Glu
    3890                3895                3900

Pro Asn Pro Glu Ala Glu Glu Leu Phe Arg Met Val Ala Glu Val
    3905                3910                3915

Phe Ile Tyr Trp Ser Lys Ser His Asn Phe Lys Arg Glu Glu Gln
    3920                3925                3930

Asn Phe Val Val Gln Asn Glu Ile Asn Asn Met Ser Phe Leu Ile
    3935                3940                3945

Thr Asp Thr Lys Ser Lys Met Ser Lys Ala Ala Val Ser Asp Gln
    3950                3955                3960

Glu Arg Lys Lys Met Lys Arg Lys Gly Asp Arg Tyr Ser Met Gln
    3965                3970                3975

Thr Ser Leu Ile Val Ala Ala Leu Lys Arg Leu Leu Pro Ile Gly
    3980                3985                3990

Leu Asn Ile Cys Ala Pro Gly Asp Gln Glu Leu Ile Ala Leu Ala

3995                4000                4005

Lys Asn Arg Phe Ser Leu Lys Asp Thr Glu Asp Glu Val Arg Asp
    4010                4015                4020

Ile Ile Arg Ser Asn Ile His Leu Gln Gly Lys Leu Glu Asp Pro
    4025                4030                4035

Ala Ile Arg Trp Gln Met Ala Leu Tyr Lys Asp Leu Pro Asn Arg
    4040                4045                4050

Thr Asp Asp Thr Ser Asp Pro Glu Lys Thr Val Glu Arg Val Leu
    4055                4060                4065

Asp Ile Ala Asn Val Leu Phe His Leu Glu Gln Lys Ser Lys Arg
    4070                4075                4080

Val Gly Arg Arg His Tyr Cys Leu Val Glu His Pro Gln Arg Ser
    4085                4090                4095

Lys Lys Ala Val Trp His Lys Leu Leu Ser Lys Gln Arg Lys Arg
    4100                4105                4110

Ala Val Val Ala Cys Phe Arg Met Ala Pro Leu Tyr Asn Leu Pro
    4115                4120                4125

Arg His Arg Ala Val Asn Leu Phe Leu Gln Gly Tyr Glu Lys Ser
    4130                4135                4140

Trp Ile Glu Thr Glu Glu His Tyr Phe Glu Asp Lys Leu Ile Glu
    4145                4150                4155

Asp Leu Ala Lys Pro Gly Ala Glu Pro Pro Glu Glu Asp Glu Gly
    4160                4165                4170

Thr Lys Arg Val Asp Pro Leu His Gln Leu Ile Leu Leu Phe Ser
    4175                4180                4185

Arg Thr Ala Leu Thr Glu Lys Cys Lys Leu Glu Glu Asp Phe Leu
    4190                4195                4200

Tyr Met Ala Tyr Ala Asp Ile Met Ala Lys Ser Cys His Asp Glu
    4205                4210                4215

Glu Asp Asp Asp Gly Glu Glu Glu Val Lys Ser Phe Glu Glu Lys
    4220                4225                4230

Glu Met Glu Lys Gln Lys Leu Leu Tyr Gln Gln Ala Arg Leu His
    4235                4240                4245

Asp Arg Gly Ala Ala Glu Met Val Leu Gln Thr Ile Ser Ala Ser
    4250                4255                4260

Lys Gly Glu Thr Gly Pro Met Val Ala Ala Thr Leu Lys Leu Gly
    4265                4270                4275

Ile Ala Ile Leu Asn Gly Gly Asn Ser Thr Val Gln Gln Lys Met
    4280                4285                4290

Leu Asp Tyr Leu Lys Glu Lys Lys Asp Val Gly Phe Phe Gln Ser
    4295                4300                4305

Leu Ala Gly Leu Met Gln Ser Cys Ser Val Leu Asp Leu Asn Ala
    4310                4315                4320

Phe Glu Arg Gln Asn Lys Ala Glu Gly Leu Gly Met Val Thr Glu
    4325                4330                4335

Glu Gly Ser Gly Glu Lys Val Leu Gln Asp Asp Glu Phe Thr Cys
    4340                4345                4350

Asp Leu Phe Arg Phe Leu Gln Leu Leu Cys Glu Gly His Asn Ser
    4355                4360                4365

Asp Phe Gln Asn Tyr Leu Arg Thr Gln Thr Gly Asn Asn Thr Thr
    4370                4375                4380

Val Asn Ile Ile Ile Ser Thr Val Asp Tyr Leu Leu Arg Val Gln
    4385                4390                4395

Glu Ser Ile Ser Asp Phe Tyr Trp Tyr Tyr Ser Gly Lys Asp Val
    4400                4405                4410

Ile Asp Glu Gln Gly Gln Arg Asn Phe Ser Lys Ala Ile Gln Val
    4415                4420                4425

Ala Lys Gln Val Phe Asn Thr Leu Thr Glu Tyr Ile Gln Gly Pro
    4430                4435                4440

Cys Thr Gly Asn Gln Gln Ser Leu Ala His Ser Arg Leu Trp Asp
    4445                4450                4455

Ala Val Val Gly Phe Leu His Val Phe Ala His Met Gln Met Lys
    4460                4465                4470

Leu Ser Gln Asp Ser Ser Gln Ile Glu Leu Leu Lys Glu Leu Met
    4475                4480                4485

Asp Leu Gln Lys Asp Met Val Val Met Leu Leu Ser Met Leu Glu
    4490                4495                4500

Gly Asn Val Val Asn Gly Thr Ile Gly Lys Gln Met Val Asp Met
    4505                4510                4515

Leu Val Glu Ser Ser Asn Asn Val Glu Met Ile Leu Lys Phe Phe
    4520                4525                4530

Asp Met Phe Leu Lys Leu Lys Asp Leu Thr Ser Ser Asp Thr Phe
    4535                4540                4545

Lys Glu Tyr Asp Pro Asp Gly Lys Gly Val Ile Ser Lys Arg Asp
    4550                4555                4560

Phe His Lys Ala Met Glu Ser His Lys His Tyr Thr Gln Ser Glu
    4565                4570                4575

Thr Glu Phe Leu Leu Ser Cys Ala Glu Thr Asp Glu Asn Glu Thr
    4580                4585                4590

Leu Asp Tyr Glu Glu Phe Val Lys Arg Phe His Glu Pro Ala Lys
    4595                4600                4605

Asp Ile Gly Phe Asn Val Ala Val Leu Leu Thr Asn Leu Ser Glu
    4610                4615                4620

His Met Pro Asn Asp Thr Arg Leu Gln Thr Phe Leu Glu Leu Ala
    4625                4630                4635

Glu Ser Val Leu Asn Tyr Phe Gln Pro Phe Leu Gly Arg Ile Glu
    4640                4645                4650

Ile Met Gly Ser Ala Lys Arg Ile Glu Arg Val Tyr Phe Glu Ile
    4655                4660                4665

Ser Glu Ser Ser Arg Thr Gln Trp Glu Lys Pro Gln Val Lys Glu
    4670                4675                4680

Ser Lys Arg Gln Phe Ile Phe Asp Val Val Asn Glu Gly Gly Glu
    4685                4690                4695

Lys Glu Lys Met Glu Leu Phe Val Asn Phe Cys Glu Asp Thr Ile
    4700                4705                4710

Phe Glu Met Gln Leu Ala Ala Gln Ile Ser Glu Ser Asp Leu Asn
    4715                4720                4725

Glu Arg Ser Ala Asn Lys Glu Glu Ser Glu Lys Glu Arg Pro Glu
    4730                4735                4740

Glu Gln Gly Pro Arg Met Ala Phe Phe Ser Ile Leu Thr Val Arg
    4745                4750                4755

Ser Ala Leu Phe Ala Leu Arg Tyr Asn Ile Leu Thr Leu Met Arg
    4760                4765                4770

Met Leu Ser Leu Lys Ser Leu Lys Lys Gln Met Lys Lys Val Lys
    4775                4780                4785

Lys Met Thr Val Lys Asp Met Val Thr Ala Phe Phe Ser Ser Tyr
    4790                4795                4800

Trp Ser Ile Phe Met Thr Leu Leu His Phe Val Ala Ser Val Phe
    4805                4810                4815

Arg Gly Phe Phe Arg Ile Ile Cys Ser Leu Leu Leu Gly Gly Ser
    4820                4825                4830

Leu Val Glu Gly Ala Lys Lys Ile Lys Val Ala Glu Leu Leu Ala
    4835                4840                4845

Asn Met Pro Asp Pro Thr Gln Asp Glu Val Arg Gly Asp Gly Glu
    4850                4855                4860

Glu Gly Glu Arg Lys Pro Leu Glu Ala Ala Leu Pro Ser Glu Asp
    4865                4870                4875

Leu Thr Asp Leu Lys Glu Leu Thr Glu Glu Ser Asp Leu Leu Ser
    4880                4885                4890

Asp Ile Phe Gly Leu Asp Leu Lys Arg Glu Gly Gly Gln Tyr Lys
    4895                4900                4905

Leu Ile Pro His Asn Pro Asn Ala Gly Leu Ser Asp Leu Met Ser
    4910                4915                4920

Asn Pro Val Pro Met Pro Glu Val Gln Glu Lys Phe Gln Glu Gln
    4925                4930                4935

Lys Ala Lys Glu Glu Glu Lys Glu Glu Lys Glu Glu Thr Lys Ser

4940                4945                4950

Glu Pro Glu Lys Ala Glu Gly Glu Asp Gly Glu Lys Glu Glu Lys
    4955                4960                4965

Ala Lys Glu Asp Lys Gly Lys Gln Lys Leu Arg Gln Leu His Thr
    4970                4975                4980

His Arg Tyr Gly Glu Pro Glu Val Pro Glu Ser Ala Phe Trp Lys
    4985                4990                4995

Lys Ile Ile Ala Tyr Gln Gln Lys Leu Leu Asn Tyr Phe Ala Arg
    5000                5005                5010

Asn Phe Tyr Asn Met Arg Met Leu Ala Leu Phe Val Ala Phe Ala
    5015                5020                5025

Ile Asn Phe Ile Leu Leu Phe Tyr Lys Val Ser Thr Ser Ser Val
    5030                5035                5040

Val Glu Gly Lys Glu Leu Pro Thr Arg Ser Ser Ser Glu Asn Ala
    5045                5050                5055

Lys Val Thr Ser Leu Asp Ser Ser Ser His Arg Ile Ile Ala Val
    5060                5065                5070

His Tyr Val Leu Glu Glu Ser Ser Gly Tyr Met Glu Pro Thr Leu
    5075                5080                5085

Arg Ile Leu Ala Ile Leu His Thr Val Ile Ser Phe Phe Cys Ile
    5090                5095                5100

Ile Gly Tyr Tyr Cys Leu Lys Val Pro Leu Val Ile Phe Lys Arg
    5105                5110                5115

Glu Lys Glu Val Ala Arg Lys Leu Glu Phe Asp Gly Leu Tyr Ile
    5120                5125                5130

Thr Glu Gln Pro Ser Glu Asp Asp Ile Lys Gly Gln Trp Asp Arg
    5135                5140                5145

Leu Val Ile Asn Thr Gln Ser Phe Pro Asn Asn Tyr Trp Asp Lys
    5150                5155                5160

Phe Val Lys Arg Lys Val Met Asp Lys Tyr Gly Glu Phe Tyr Gly
    5165                5170                5175

Arg Asp Arg Ile Ser Glu Leu Leu Gly Met Asp Lys Ala Ala Leu
    5180                5185                5190

Asp Phe Ser Asp Ala Arg Glu Lys Lys Lys Pro Lys Lys Asp Ser
    5195                5200                5205

Ser Leu Ser Ala Val Leu Asn Ser Ile Asp Val Lys Tyr Gln Met
    5210                5215                5220

Trp Lys Leu Gly Val Val Phe Thr Asp Asn Ser Phe Leu Tyr Leu
    5225                5230                5235

Ala Trp Tyr Met Thr Met Ser Val Leu Gly His Tyr Asn Asn Phe
    5240                5245                5250

Phe Phe Ala Ala His Leu Leu Asp Ile Ala Met Gly Phe Lys Thr
    5255                5260                5265

Leu Arg Thr Ile Leu Ser Ser Val Thr His Asn Gly Lys Gln Leu
    5270                5275                5280

Val Leu Thr Val Gly Leu Leu Ala Val Val Val Tyr Leu Tyr Thr
    5285                5290                5295

Val Val Ala Phe Asn Phe Phe Arg Lys Phe Tyr Asn Lys Ser Glu
    5300                5305                5310

Asp Gly Asp Thr Pro Asp Met Lys Cys Asp Asp Met Leu Thr Cys
    5315                5320                5325

Tyr Met Phe His Met Tyr Val Gly Val Arg Ala Gly Gly Gly Ile
    5330                5335                5340

Gly Asp Glu Ile Glu Asp Pro Ala Gly Asp Glu Tyr Glu Ile Tyr
    5345                5350                5355

Arg Ile Ile Phe Asp Ile Thr Phe Phe Phe Phe Val Ile Val Ile
    5360                5365                5370

Leu Leu Ala Ile Ile Gln Gly Leu Ile Ile Asp Ala Phe Gly Glu
    5375                5380                5385

Leu Arg Asp Gln Gln Glu Gln Val Lys Glu Asp Met Glu Thr Lys
    5390                5395                5400

Cys Phe Ile Cys Gly Ile Gly Asn Asp Tyr Phe Asp Thr Val Pro
    5405                5410                5415

His Gly Phe Glu Thr His Thr Leu Gln Glu His Asn Leu Ala Asn
    5420                5425                5430

Tyr Leu Phe Phe Leu Met Tyr Leu Ile Asn Lys Asp Glu Thr Glu
    5435                5440                5445

His Thr Gly Gln Glu Ser Tyr Val Trp Lys Met Tyr Gln Glu Arg
    5450                5455                5460

Cys Trp Glu Phe Phe Pro Ala Gly Asp Cys Phe Arg Lys Gln Tyr
    5465                5470                5475

Glu Asp Gln Leu Asn
    5480

<210>  8
<211>  35
<212>  DNA
<213>  artificial

<220>
<223>  primer

<400>  8
gagacccaag ctggctagcg tttaaacggg ccctc                                35

<210>  9
<211>  39
<212>  DNA
<213>  srtificial

<400>  9
cgtttaaacg ggccctctag accatggatt atggaggcg                            39

<210>  10
<211>  36
<212>  DNA
<213>  artificial

<220>
<223>  primer

<400>  10
cctccgcccc agttgggcat cttgtacagc tcgtcc                               36

## Description

The present invention relates in a first aspect to a polypeptide comprising a channelrhodopsin moiety, a linker moiety and a moiety of a Ca²⁺ sensitive protein. In particular, the polypeptide is a polypeptide composed of the channelrhodopsin-2 of *Chlamydomonas reinhardtii,* a linker and RyR2 whereby the linker is designed to establish a distance of from 100 to 200 Angstrom between the channelrhodopsin moiety and the RyR2 moiety when present in a cellular membrane of eukaryotes. In addition, nucleic acid molecules encoding the polypeptide according to the present invention as well as vector or vector systems containing said nucleic acid molecule. Further, nucleic acid constructs comprising the nucleic acid encoding the polypeptide of the channelrhodopsin as well as the linker is provided. In addition, isolated cells, cell lines or host cells containing the vector or vector system according to the present invention or expressing the polypeptide according to the present invention are provided. Further, the *C. elegans* strains containing the genomic sequence according to the present invention or expressing the polypeptide according to the present invention are provided. Method for determining the activity of potential principal actives on Ca²⁺ sensitive proteins are provided based on determining the activity of these molecules on said Ca²⁺ sensitive protein.

### Prior art

The intracellular Ca²⁺ concentration is the most important signaling mechanism in all cells of all organisms to control a broad range of physiological and pathological effects including gene expression, ion channel function, synaptic transmission, cell metabolism and muscle contraction. Alteration of intracellular Ca²⁺ levels can result in cellular damage, apoptosis, altered gene expression and generally failure of proper cell function and have been implicated in several disease conditions. Thus, the control of intracellular Ca²⁺ concentrations must be very tightly regulated. Intracellular Ca²⁺ levels are affecting cellular function by modulating Ca²⁺ sensitive proteins including enzymes, ion channel and many others though various mechanisms such EF-hand proteins, annexins, and C2 domain proteins (Bagur R, et al. Mol Cell. 2017 Jun 15;66(6):780-788)

The ryanodine receptor (RyR) Ca²⁺ release channel resides in the largest membrane organelle, the sarco-/endoplasmic reticulum (SR/ER), where it plays an important role in Ca²⁺-Induced Ca²⁺ Release (CICR). CICR is an essential signaling process for all excitable cells, particularly neurons and cardiomyocytes to maintain physiological functions without which life is not possible. Importantly, abnormally increased RyR channel activity, for example during the resting phase of cardiomyocytes, contributes to a "leak" flux of Ca²⁺ ions from the SR/ER organelle and to intracellular Ca²⁺ overload. Consequently, increased metabolic and proteomic stress can lead to cell injury, for example neurological disorders like seizures and dementia. Likewise, in the heart, in cardiomyocytes abnormal Ca²⁺ leak from SR/ER through RyR type 2 (RyR2) channels for example due to inherited mutations in the isoform RyR2 as well as pathologically increased RyR2 phosphorylation in patients with acquired heart failure can cause lethal arrhythmias and cardiac degeneration.

Given the vital importance of normal RyR channel function, a growing number of common as well as rare genetic organotypic diseases were associated with SR/ER increased Ca²⁺ leak. Accordingly, there is a growing need for reproducible and scalable screening systems, ideally based on human cell types, to identify and optimize therapeutically active RyR channel modifying substances. Within this context, it is important to activate the RyR Ca²⁺ release channel physiologically as much as possible via CICR. However, precise control of CICR for drug screening is challenging as cellular models for analysis of RyR function are currently not suited for high throughput assays. The gold-standard for analysis of RyR function are single-channel experiments in artificial lipid bilayers, which allow for quasi-physiological control of RyR activation through increasing cytosolic (cis chamber) Ca²⁺ concentrations. However, reconstitution and analysis of RyR channels in lipid bilayers is extremely time consuming and, ultimately, not representative of endogenous RyR activation in the physiological context of subcellular Ca²⁺ nanodomains.

Therefore, low-throughput confocal Ca²⁺ imaging experiments in isolated cardiomyocytes are employed presently as standard approach to study RyR channel function in its physiological context. Moreover, in this native cardiomyocyte system, increases in intracellular Ca²⁺ concentration to activate RyR channel opening can be manipulated by cell membrane permeabilization with a Ca²⁺ ionophore. Alternative membrane-intact live-cell assays involving a non-physiological elevation of extracellular Ca²⁺ to secondarily increase Ca²⁺ levels in the SR/ER store to trigger RyR channel opening from the luminal channel side has been reported (Jiang et al. PNAS 2004;101(35):13062). However, this "store-overload-induced Ca²⁺ release" does not represent a physiological screening method despite other claims (US Patent US8703804), because RyR channels are also activated through increased cytosolic Ca²⁺ levels, and thus uncontrolled CICR occurs, which is difficult to impossible to analyze and interpret data properly.

The RyR represents a target for various active principals since it plays an important role in various diseases and conditions, e.g. pathological intracellular Ca²⁺ leakage. Kushnir A., and MarxA.R., Recent Path. Biotechnol., 2012; 6(3): 157-166 disclose RyR patents discussing the development of novel drugs, screening tools and research methods in this field. It is speculated that in view of the central role that SR/ER Ca²⁺ leakage play in human physiology, there will be an increasing number of compounds and patents in this field. However, suitable methods under physiological conditions for screening suitable target compounds leading eventually to drugs inhibiting pathological intracellular Ca²⁺ leakages are still an issue. Namely, these limitations described above both in scaling of the methods and the non-physiological activation of RyR channel opening requires to conceptualize new approaches to gain more precise spatial and temporal control of intracellular Ca²⁺ release using light and targeting of suitable optogenetic tools in the immediate vicinity of RyR channels.

### Brief description of the present invention

There is a further need for providing tools and methods allowing to identify candidate compounds and physiological tests on Ca²⁺ sensitive proteins or altering Ca²⁺ release in cells and cellular compartments under physiological conditions. The present inventors aim in providing fusion polypeptides wherein direct fusion of an optogenetic tool with Ca²⁺ sensitive protein allow to control effectively the Ca²⁺ sensitive protein upon light application followed e. g. by Ca²⁺ release. For example, in case of the RyR channel system, the optogenetic tool effectively controls the RyR channel activation upon light application followed by Ca²⁺ release from the SR/ER store in any intact living cell. The present approach specifically addresses the physiologically relevant activation of RyR channels by increasing Ca²⁺ selectively at the cytosolic site to mimic the CICR mechanism. The present inventors recognized that by combining optogenetic tools that use light control as a primary CICR activation mechanism, it is compatible with high throughput screening assays as repetitive Ca²⁺ pipetting steps to activate RyR channel opening are dispensable and replaced by a light controlled CICR mechanism.

Hence, in a first aspect, the present invention relates to a polypeptide comprising from the N-terminus to the C-terminus i) a Channelrhodopsin (ChR) moiety, ii) optionally a linker moiety and iii) a moiety of a Ca²⁺ sensitive protein. In an embodiment of said polypeptide, the polypeptide is composed of the Channelrhodopsin 2 (ChR2) of *Chlamydomonas reinhardtii* in combination with a linker and the human RyR2 moiety.

In a further aspect, a nucleic acid molecule comprising a nucleic acid sequence encoding the polypeptide according to the present invention is provided.

Moreover, a nucleic acid construct is disclosed herein. Said construct comprise a nucleic acid encoding a fusion polypeptide wherein a moiety encoding a Channelrhodopsin as described herein and a linker as defined herein is present vector in combination with at least a part of at least one exon of the DNA encoding the moiety of the Ca²⁺ sensitive protein. Further, the present invention relates to a vector containing a nucleic acid or a nucleic acid construct according to the present invention. Moreover, isolated cells, cell lines or host cells are described containing the vector according to the present invention, or the nucleic acid according to the present invention or expressing the polypeptide according to the present invention.

Further, *C. elegans* strains are provided containing the nucleic acid according to the present invention or expressing the polypeptide according to the present invention.

Finally, a method for determining the activity of a candidate compounds on Ca²⁺ sensitive proteins is provided, said method comprises the steps of providing a cell, cell line or host cell according to the present invention or a *C. elegans* strain according to the present invention;
exposing the cell, cell line or host cell or the *C. elegans* strain with the candidate compound; and
determining the activity of said candidate compound on the Ca²⁺ sensitive proteins.

### Brief description of the figures

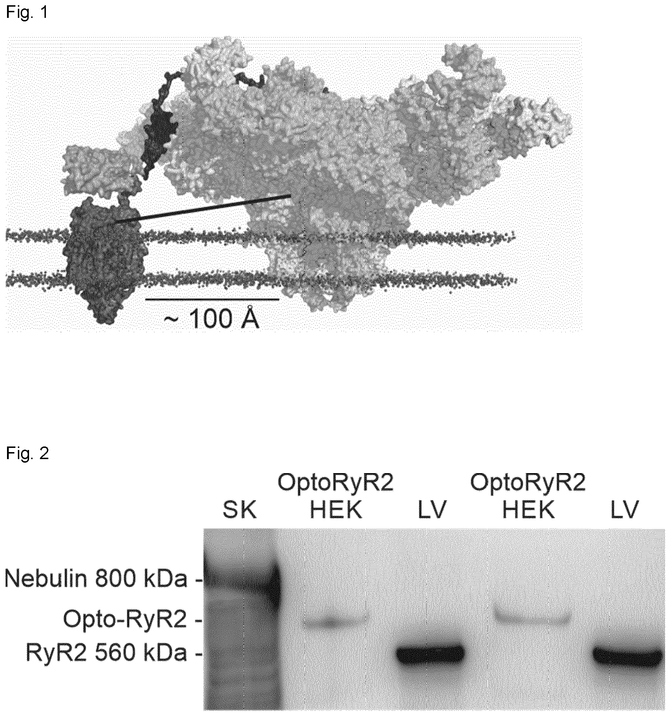

Fig. 1: OptoRyR2 structure, predicted with MODELLER, restraining known structures of ChR2 monomer (grey, PDB: 3UG9), EYFP (light grey, PDB: 1EMA), CRP1 linker (black PDB: 1B8T) and RyR2 (large light grey structure on the right, shown as tetramer, PDB: 5GOA). For clarity only one EYFP-linker structure is shown at one protomer of the RyR2 tetramer. Distance between CatChUP Ca²⁺ release site and RyR Ca²⁺ sensor indicated in black line (∼100 Ǻ)
Fig. 2: Western blot from a 2.3% SDS-PAGE of OptoRyR2 HEK293 cells, rat left ventricle (LV) and rat skeletal muscle (SK) tested for RyR2 and Nebulin showing the expected ∼84 kDa size increase of OptoRyR2 due to CatChUP-EYFP-CRP1 addition to RyR2 (560 kDa).

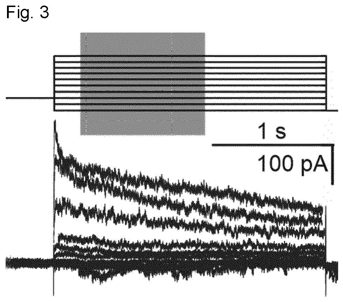

Fig. 3: Current traces (lower) in voltage clamp mode of a HEK293 cell stably expressing OptoRyR2, at potentials ranging from -60 mV to +30 mV in 10 mV steps (protocol indicated above). Blue light was applied for 1 s (grey box, 470 nm, 1.4 mW/mm²) and did not induce currents at all holding potentials indication lack of OptoRyR2 plasma membrane expression,

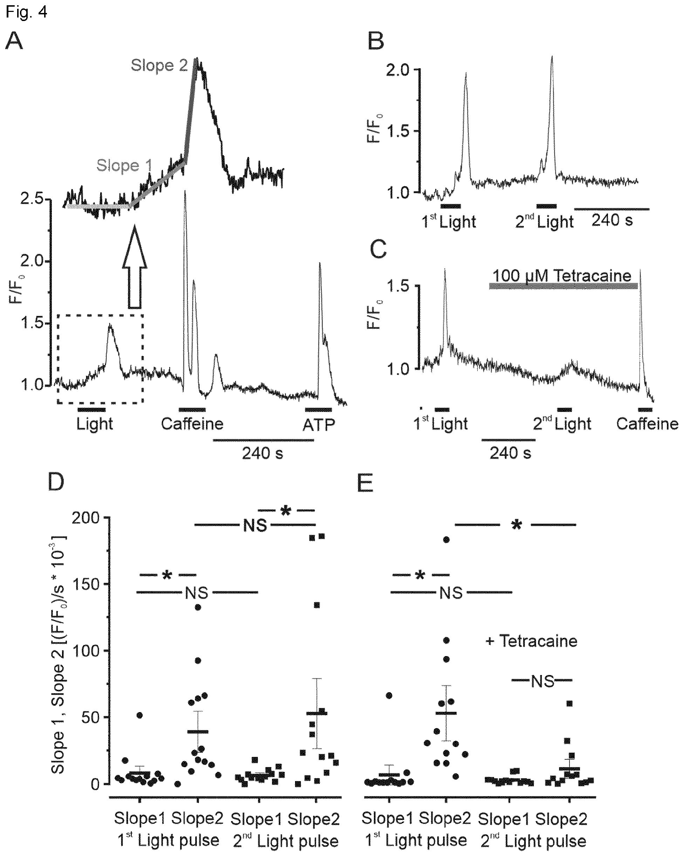

Fig. 4: A) Ca²⁺ imaging (X-Rhod-1) with light, caffeine (10 mM) and ATP (1 mM) induced Ca²⁺ releases. Note the biphasic effect of light (upper insert: slope 1 (light grey) and slope 2 (dark grey) analyzed by linear fits). B-E) Two light pulse protocol revealed similar effects (B, statistic in D) however application of Tetracaine before the 2^{nd} light pulse (C, statistics in E) almost completely blocked the fast release phase (slope 2). *: p < 0.05 (Paired Student's t-test). Light pulses at 1.4 mW/mm².

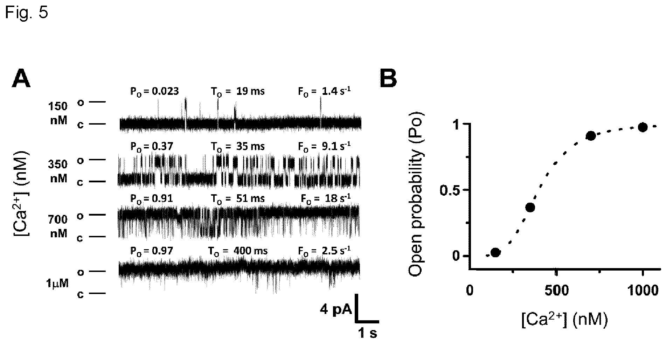

Fig 5: Single-channel OptoRyR2 current traces in planar lipid bilayer and Ca²⁺-induced Ca²⁺ activation via activation of the cytosolic Ca²⁺ binding site. A) Representative single-channel optoRyR2 current traces at four different cytosolic Ca²⁺ concentrations (150 nM to 1 µM). Each trace shows 10 s of OptoRyR2 channel activity. Single-channel openings are plotted upward (o) versus closed (c) states to the left of each trace. Single-channel open probability (Pₒ), mean open time (Tₒ), and frequency of openings (Fₒ) are depicted above each trace and represent average values from 1-2 minutes of continuous recording. B) Corresponding Pₒ-Ca²⁺ concentration plot fitted with a Hill sigmoidal function (k=4) confirming a Ca²⁺ concentration-dependent activation of OptoRyR2.

### Detailed description of the present invention

The present invention relates in a first aspect to a polypeptide comprising from the N-terminus to the C-terminus a channelrhodopsin (ChR) moiety, optionally a linker moiety, and a moiety of a Ca²⁺ sensitive protein.

The present inventors recognized that the fusion polypeptide comprising the channelrhodopsin moiety and a moiety of a Ca²⁺ sensitive protein whereby both moieties are optionally spaced apart by a linker moiety located between the channelrhodopsin moiety and a moiety of a Ca²⁺ sensitive protein, allows to analyze compounds able to control the Ca²⁺ sensitive protein activation. The polypeptide according to the present invention allows to establish reproducible and scalable screening systems. Namely, it is possible to conduct methods and screening assays with higher specificity and sensitivity with a fast and reliable screening system based on the polypeptide according to the present invention.

Generally, single amino acids or 1 to 15, like 1 to 10 amino acids may be present between the different moieties, e.g. located between the ChR moiety and the linker, if present, the linker and the Ca²⁺ sensitive protein, like the RyR2 protein.

In an aspect of the present invention, the polypeptide is a fusion protein or fusion polypeptide wherein the moiety of a Ca²⁺ sensitive protein is stemming from a Ca²⁺ sensitive transmembrane protein in particular, wherein the moiety is stemming from a Ca²⁺ sensitive transmembrane protein located at the sarco/endoplasmatic reticulum Ca²⁺ store.

As used herein, the term "polypeptide" or "protein" refers to a polypeptide composed of amino acids. Typically, the polypeptide or protein according to the present invention is an isolated polypeptide or protein unless otherwise indicated. Namely, the polypeptide or protein according to the present invention is a recombinant polypeptide or protein. The polypeptide or protein is a fusion polypeptide or fusion protein which means that the polypeptide or protein contain at least two different moieties stemming from at least two different genus.

The term "Ca²⁺ sensitive protein" as used herein refers to a protein, which is directly regulated in its function by the local Ca²⁺ concentration.

Further, the term "moiety" as used herein refers to at least a functional part or the full protein. That is, the moiety may comprise the mature protein or may comprise the protein including the leader sequence. In addition, the moiety may be a functional part of the full protein. For example, in case of the ChR moiety, typically the C-terminus is absent, e.g. as shown in SEQ ID No.2 or 3. The linker, if present, may be the full protein, namely, encoded by the respective cDNA. The Ca²⁺ sensitive protein may be present as mature protein, or may also contain the leader sequence.

In another aspect, the present invention refers to a polypeptide wherein the moiety of a Ca²⁺ sensitive transmembrane protein is stemming from a moiety of a human inositol triphosphate receptor or a moiety of a ryanodine receptor (RyR).

In this connection, the term "stemming from" or "derived from" are used interchangeably herein. This term identifies that the peptide has the amino acid sequence of the respective initial protein whereby said protein may contain additional one or more substitutions, insertions or deletions without deteriorating the function thereof.

The ryanodine receptor may be the human RyR2 moiety. The full sequence of the RyR2 moiety is shown in SEQ ID No. 1.

In case of using *C. elegans* as a model system for screening purposes the moiety of a Ca²⁺ sensitive transmembrane moiety is stemming or derived from the UNC-68 protein of *C. elegans.* In a broader sense, the Ca²⁺ sensitive protein is typically stemming from the host cell, cell line or isolated cell, the polypeptide according to the present invention is introduced. That is, in case of human cells, the Ca²⁺ sensitive moiety is stemming from a human source while in case of *C. elegans,* the moiety is stemming from *C. elegans,* like the UNC-68 protein accordingly.

In another embodiment of the present invention, the polypeptide according to the present invention is a polypeptide wherein the channelrhodopsin moiety is stemming from *Chlamydomonas reinhardtii* or *Volvox carteri* or combinations thereof. In an embodiment, a combination of these two channelrhodopsin moieties with the ryanodine receptor moiety from human proteins is disclosed.

In another embodiment, the channelrhodopsin moiety is channelrhodopsin-2 (ChR2) stemming from *Chlamydomonas reinhardtii.* For example, this ChR2 is a ChR2 molecule peptide which may contain mutations, like substitutions. For example, for the ChR2 from *Chlamydomonas reinhardtii* mutations are known improving Ca²⁺ permeability as well as overall permeability and membrane targeting. The sequence of the ChR2 is shown in SEQ ID No. 2 while the mutated ChR2 is shown in SEQ ID No. 3. This construct termed CatChUP is described as ChR2 (L132C;H134R;T159C) in Bergs et al., 2018, PLOS One 13(2):e0191802.

In another embodiment, the polypeptide according to the present invention is a polypeptide wherein the linker is selected to establish a distance of from 100 to 200 Angstrom between the ChR moiety and the Ca²⁺ sensitive protein moiety, like the RyR moiety when present in the cell membrane of a eukaryotic cell.

In an embodiment, the linker allows to establish a suitable distance between the two moieties to enable proper protein folding of the moieties and defined distance of Ca2+ diffusion between the ChR2 moiety and the moiety of the Ca²⁺ sensitive protein.

In this connection, the term "linker" refers to a protein that in can be used to establish a physical distance between two proteins without disturbing their function. Depending on the type of membrane wherein the polypeptide according to the present invention should be integrated, the linker is selected to build up the desired physical distance between the two proteins, namely the Channelrhodopsin moiety and the Ca²⁺ sensitive protein. Typically, the distances are in between 10 Angstrom to 500 Angstrom, e. g. in case of transmembrane polypeptides the distance is in between 50 to 300 Angstrom, like 100 to 200 Angstrom. In an embodiment, the linker is selected from the chicken cysteine-rich protein (CRP1). A suitable linker is given in SEQ ID No. 4.

An embodiment of the polypeptide of the present invention is a polypeptide of SEQ ID No. 5 with EYFP or SEQ ID No. 7 without EYFP. The nucleic acid sequence encoding the polypeptide of SEQ ID No. 5 is shown in SEQ ID No. 6 accordingly.

The nucleic acid according to the present invention or the nucleic acid construct according to the present invention comprise a nucleic acid sequence coding for the polypeptide according to the present invention or a nucleic acid encoding a polypeptide comprising a Channelrhodopsin, in particular, a Channelrhodopsin-2, optionally a linker as defined herein, and at the 3' end of the nucleic acid, at least a part of at least one exon of the DNA encoding the moiety of the Ca²⁺ sensitive protein, like the RyR molecule, in particular, a RyR2 molecule.

To ensure optimal expression, the coding nucleic acid can also be suitably modified. For example, by adding suitable regulatory sequences and/or targeting sequences and/or by matching of the coding DNA sequence to the preferred codon usage of the chosen host, modification may be affected. The targeting sequence may encode a C-terminal extension targeting the polypeptide to a particular site or compartment within the cell.

Generally, the nucleic acid construct according to the present invention is suitable for homology directed integration in a host cell DNA. Various genome editing methods are known to the skilled person allowing homology directed integration accordingly, suitable methods include: genome editing with homology directed repair (HDR) using meganucleases, zinc finger nucleases (ZFNs), transcription activator-like effector-based nucleases (TALEN) or clustered regularly interspaced short palindromic repeats (CRISPR/CAS), as well as classical homologous recombination methods.

In one aspect, the nucleic acid construct is suitable for CRISP/CAS mediated homology directed repair integration into a host cell DNA. The skilled person is well aware of suitable constructs allowing the same. In addition, the skilled person is well aware of suitable vector systems necessary for effecting the homology directed repair based on the CRISP/CAS technology.

The nucleic acid may be combined with further elements, e.g., a promoter, and a transcription start and stop signal and a translation start and stop signal and a polyadenylation signal in order to provide for expression of the sequence of the polypeptide according to the present invention. The promoter can be inducible or constitutive, general host cells specific promoter. Examples are known to the skilled person. Selection of promoters, vectors and other elements is a matter of routine and the skilled person is well aware of the suitable promoters, vectors and other elements depending on the host cell.

In an embodiment, the nucleic acid construct is a construct comprising a nucleic acid encoding the channelrhodopsin-2 polypeptide as defined herein in combination with the linker as defined herein. In a further embodiment, the nucleic acid construct is construct comprising further at the 3' end prime at least one exon of the DNA encoding the Ca²⁺ sensitive protein or the moiety of said Ca²⁺ sensitive protein as defined herein. In an embodiment, the moiety of the Ca²⁺ sensitive protein is a moiety of the RyR molecule, in particular, a RyR2 molecule of human origin or a UNC-68 molecule of *C. elegans.*

It has been recognized that when introducing the light gated channelrhodopsin moiety with the linker moiety into the cells already containing the Ca²⁺ sensitive protein in the genome, the efficacy and efficiency of transcription and translation is improved when introducing also the at least first exon encoding said Ca²⁺ sensitive protein and integrating the nucleic acid construct according to the present invention at the 5' end of the next exon or intron following the sequence of the Ca²⁺ sensitive protein. For example, for effecting HDR, the nucleic acid construct contains at least a part of the exon of the Ca²⁺ sensitive protein to allow integration in the reading frame of the Ca²⁺ sensitive protein accordingly.

In an embodiment, introduction of the nucleic acid construct is effected by known methods. An embodiment of the method for introduction is the CRISP/CAS mediated homology direct repair integration into the host cell DNA. Construction of the suitable construct is known to the skilled person.

Further, the present invention provides a vector containing a nucleic acid or a nucleic acid construct according to the present invention. The vector can be any vector suitable for the cells to be transfected. For example, the vector may be suitable for virus mediated gen transfer and, thus can be delivered packed in a virus to the site or the cells of interest. Suitable vectors are known to the skilled person. Alternatively, the vector may be introduced into the cells by other means for transfections including e.g. by chemical transfection, electroporation or coprecitation.

The vector is selected based on the method applied for genomic integration of the nucleic acid or nucleic acid construct according to the present invention.

For example, in case of homology directed repair (HDR), the nucleic acid or nucleic acid construct according to the present invention may be introduced as single stranded DNA, ssDNA, into the respective host to enable HDR in order to obtain cells, cell lines or host cells containing a nucleic acid or nucleic acid construct as defined herein. Also, for obtaining *C. elegans* strains, single stranded DNA may be used.

In another aspect, isolated cells, cell lines or host cells containing the vector according to the present invention, a nucleic acid or nucleic acid construct according to the present invention or expressing the polypeptide according to the present invention is provided.

Suitable cells to be transfected with the vector or the nucleic acid molecule or nucleic acid construct according to the present invention or containing the nucleic acid according to the present invention or expressing the polypeptide according to the present invention include human embryonic kidney 293 cell, mouse embryonic stem cells as well as human pluripotent cells, like embryonic stem cells or human induced pluripotent cells (hiPSC). Of course, derivatives of the hiPSC are included. In this connection, the term "derivative" refers to hiPSC differentiated into specific subtypes, specifically cardiomyocytes. Finally, hiPSC can be differentiated in excitable cell types, for example cardiomyocytes or neurons, as well as engineered in multicellular tissue constructs and organoids presently under development. Thus, suitable cells include neurons and precursors thereof.

The isolated cells, cell lines or host cells according to the present invention will be selected on the basis of the target Ca²⁺ sensitive protein to be influenced. Namely, depending on the target protein, the cell, cell line or host cell is selected. Based on the selected cell type the nucleic acid molecule or nucleic acid construct according to the present invention is elected. For example, in case of pluripotent stem cells, like embryonic stem cells or of human induced pluripotent cells as well as derivatives thereof, a nucleic acid construct containing at least a part of the first exon of the Ca²⁺ sensitive protein. In case of cells wherein the DNA is methylated to silence transcription, the nucleic acid construct contains more than one exons, thus, substituting the methylated exons to allow transcription. The methods for obtaining isolated cells for obtaining cell lines or host cells transfected with the respective nucleic acid sequences are known to the skilled person.

In another aspect, *C. elegans* strains are provided containing a genomic insert according to the present invention or a nucleic acid or a nucleic acid construct according to the present invention, said *C. elegans* strains expressing the polypeptide according to the present invention. Hence, in another embodiment, the present invention relates to *C. elegans* strains containing and expressing the polypeptide accordingly.

In addition, the present invention relates to a method for determining the activity of candidate compounds on Ca²⁺ sensitive proteins. In an embodiment, the Ca²⁺ sensitive proteins are as defined herein. The method is based on the use of the polypeptide, in particular, expression of the polypeptide according to the present invention in a model system as described herein including the *C. elegans* strains or expressed in the host cell, cell lines or isolated cells as described herein. These cells or strains are exposed to the candidate compound in media suitable for determining under physiological conditions the effect of said candidate compounds on processes involving the Ca²⁺ sensitive protein. Namely, the candidate compounds may affect various functionalities and properties of the Ca²⁺ sensitive protein including enzymatic activity, conformational changes, Ca² transport, opening / closing of ion channels besides the control of Ca²⁺ transport and Ca²⁺ release as well as Ca²⁺ storage, in particular, from intracellular SR/ER organelle stores. Namely, the effect of the candidate compound on Ca²⁺ release from stores in the cells under the control of the Ca²⁺ sensitive proteins, in particular, the Ca²⁺ sensitive proteins as defined herein is examined. This method is particularly helpful for determining any therapeutic or toxic effect of said candidate compounds based on the Ca²⁺ release from intercellular stores of the cells. The method allows an economic and fast but also reliable test of a number of compounds overcoming the problems of the tests available so far, which are time consuming and costly with low reliability. The method is particularly suitable for high throughput screening of compound libraries.

The read-out system suitable in the method for determining the activity of candidate compounds on Ca²⁺ sensitive proteins include:
- Ca²⁺ imaging,
- determining the enzymatic activity of Ca²⁺ sensitive proteins or enzymes downstream of the metabolic pathways;
- electrophysiologic analysis of cells by known methods, includingpatch clamp, planar patch clamp, automatic patch clamp, field potential measurements, impedance measurements,
- electrophysiological methods to assess C. elegans pharyngeal action potentials in microfluidic chips,
- videomicroscopy of beating cardiomyocytes or engineered cardiac tissue,
- videomicroscopy for quantification of C. elegans locomotion or pharyngeal pumping.

The present invention will be described further by way of examples without limiting the present invention thereto.

### Materials and Methods

### Generation a ChR2 mutant with an enhanced Ca²⁺ permeability and photo-current, CatChUP

Based on the original ChR2 subclone construct for expression *Caenorhabditis elegans* muscle cells (pmyo-3::ChR2(1-314; H134R)::eYFP; Nagel et al., 2005, Curr. Biol. 2005;15(24):2279-84), a version with enhanced Ca²⁺ permeability and overall permeability and membrane targeting was generated, by introducing the point mutations L132C (based on Kleinlogel et al., 2011, Nat. Neurosci.; 14 (4):513-8) and T159C (based on Berndt et al., 2011, PNAS 108(18):7595-600). The resulting construct pmyo-3::ChR2(1-314; L132C; H134R; T159C)::eYFP was termed CatChUP (published as ChR2(L132C;H134R;T159C) in Bergs et al., 2018, PLOS One 13(2):e0191802.).

### Construction of the OptoRyR2 expression plasmids

The novel hybrid light-activated ryanodine receptor 2 (RyR2) named OptoRyR2 is a fusion chimeric protein, from N-terminus to the C-terminus, consists of a new variant of Ca²⁺-permeable light-gated ion channel channelrhodopsin-2 (ChR2) named CatChUP, the enhanced yellow fluorescent protein (EYFP), the full-length chicken cysteine-rich protein 1 (CRP1) and the human RyR2. To construct the OptoRyR2 expression vector, we employed a DNA fragment assembly method using in-vitro homologous recombination reaction to fuse together DNA fragments that share terminal end-homology (GeneArt^{®} Seamless Cloning Kit, Invitrogen^{™} Life Technologies^{™}). In detail, a vector pcDNA3-hRyR2 (kindly provided by Dr. Andrew Marks, Columbia University, NY, USA) containing the entire cDNA sequence (∼15 kb) of the human cardiac muscle ryanodine receptor 2 (hRyR2) was linearized with the restriction enzyme Xhol and subsequently blunted with Klenow fragment (both ThermoFisher Scientific). A DNA fragment 1) encoding for the chicken CRP1 of SEQ ID No. 4 (RefSeq accession no. NP_990579.1) was engineered for mammalian codon usage and synthesized as double-stranded linear DNA fragments (Invitrogen^{™} GeneArt^{™} Strings^{™}, ThermoFisher). A DNA fragment 2) encoding for CatChUP-EYFP was obtained by polymerase chain reaction (PCR) using 100 pg of pmyo-3::ChR2(1-314; H134R)::eYFP as template DNA, 10 pM each of these primers: CatChUP-EYFP_FW1: GAGACCCAAGCTGGCTAGCGTTTAAACGGGCCCTC (SEQ ID No 8), CatChUP-EYFP_FW2: CGTTTAAACGGGCCCTCTAGACCATGGATTATGGAGGCG (SEQ ID No 9), CatChUP-EYFP_RV: CCTCCGCCCCAGTTGGGCATCTTGTACAGCTCGTCC (SEQ ID No 10), 25 µl of 2x PrimeSTAR Max Premix (TaKaRa) in a final volume of 50 µl and a PCR program of initial 30 s 98°C, 35 cycles of 10 s 98°C, 5 s 55°C, 20 s 72°C. The PCR product was purified using QIAquick PCR Purification Kit (QIAGEN). Finally, purified DNA fragments 1) and 2) were assembled with linearized pcDNA3-hRyR2 using GeneArt^{®} Seamless Cloning Kit (Invitrogen^{™} Life Technologies^{™}, ThermoFisher Scientific) following the manufacturer's instructions. The new construct pcDNA3-CatChUP-EYFP-linkerCRP1-hRyR2 was named shortly pcDNA3-OptoRyR2 and was verified by sanger sequencing (MWG Eurofins), SEQ ID No. 6.

### Generation of stable HEK293 cell line expressing OptoRyR2

A stable HEK293 cell line expressing OptoRyR2 was generated from AD-293 cells (Agilent) grown at 37°C and 5% CO₂ in DMEM medium supplemented with 10% fetal calf serum, 100 U/ml penicillin, and 100 µg/ml streptomycin. For stable transfection, the pcDNA3-OptoRyR2 plasmid was linearized with ScaI (ThermoFisher Scientific), purified by QAEX II Gel Extraction Kit (QIAGEN) and introduced into cells using the FuGENE^{®} HD transfection reagent (Promega) according to the manufacturer's instructions. Transfected cells were selected with 900 mg/ml G418 ∼48 h post-transfection. From the polyclonal population, single cell clones were generated by serial dilution in 96 well plates. The clones with correct EYFP expression were examined, The OptoRyR2 protein expression was tested using fluorescence microscopy and western blot analysis. Whole-cell patch clamp recording was performed as previously reported (Bruegmann et al. Nat Methods. 2010 7(11):897-900.

### Sodium dodecyl sulfate-polyacrylamide gel electrophoresis (SDS-PAGE) and Western blot analysis

Samples were solubilized in modified Laemmli buffer containing 8 M urea, 2 M thiourea, 3% SDS (wt/vol), 75 mM DTT, 0.03% bromophenol blue, 10% glycerol, and 0.05 M Tris -HCI, pH 6.8. After incubation for 5 min on ice samples were heated for 5 min at 95°C, followed by centrifugation. Protein lysates were loaded on agarose-stabilized (1.5%) SDS-polyacrylamide-gels (with 3 % acrylamide/bisacrylamide). Separated Proteins were transferred to a PVDF (polyvinylidene difluoride) membrane using a semi-dry blot system. Membranes were incubated with anti-RyR2 monoclonal antibody (clone C3-33, ThermoFisher). Anti-rabbit IgG conjugated with horseradish peroxidase served as secondary antibody. Bands were visualized using a Fusion SOLO imaging system (Vilber Lourmat).

### Fluorescent imaging of intracellular Ca²⁺

Intracellular Ca²⁺ transients in HEK293 cells expressing OptoRyR2 were measured by using Ca²⁺ imaging technique. Briefly, cells were loaded with 1.5 µM X-Rhod-1 acetoxymethyl ester (ThermoFisher Scientific) plus 0.02% pluronic F-127 (ThermoFisher Scientific) in Tyrode solution (composition in mmol/L: 142 NaCl, 5.4 KCI, 1.8 CaCI2, 2 MgCI2, 10 glucose and 10 Hepes; pH 7.4, NaOH) for 20 min at room temperature and subsequently incubated for another 20 min at 37°C for deesterification. A Ca²⁺ imaging system (Till Photonics, ThermoFisher Scientific) equipped Axiovert 200 microscope (Zeiss) and optical fibers were employed to perform the intracellular Ca²⁺ imaging in cells continuously superfused with Tyrode solution containing various test substances: Caffeine 10 mM, ATP 1 mM and Tetracaine 100 µM at 37°C. Cells were imaged every 500 ms with monochromic excitation light (15 ms, 570 nM, bandwidth 10 nm). Simultaneous OptoRyR2 stimulation with blue-light during Ca²⁺ imaging was performed with a LED (see below) using a dual port condenser (Till Photonics, ThermoFisher Scientific) and a 505 nm DCXR dichroic filter (all filters AHF Analysetechnik) for combining OptoRyR2 stimulation and X-Rhod-1 excitation light. Upon OptoRyR2 stimulation by light, blue-light illumination was paused for 15 ms for each 500 ms during image acquisition. Origin8 (OriginLab Corporation) was used for off-line data analysis.

### Blue-light illumination

Blue-light stimulation of OptoRyR2 expressing HEK293 cells during Ca²⁺ imaging was performed with a temperature-controlled LED module (Omicron LEDMOD LAB, 470nm, Omicron Laserage) that was coupled to an Axiovert 200 microscope (Zeiss) with an optical fiber. Low light intensities were obtained by internal pulse-width modulation of the LED (1% output, 5000 Hz). Stimulation light passed through a neutral density filter with 1% transmission (ND 2) and was directed to a 20x Fluar objective (numerical aperture 0.75, Zeiss). Light intensity was measured at the objective with a powermeter and appropriate wavelength correction (PM 100 and S130A sensor, Thorlabs).

### Isolation of OptoRyR2-containing microsomes and analysis in lipid bilayers

OptoRyR2-containing microsomes were prepared as follows: HEK293T/17 cells (ATCC Cat. No. CRL-11268) were cultured according to manufacturer's recommendations with suggested medium (ATCC 30-2002) and transiently transfected with endotoxin-free pcDNA3-OptoRyR2 plasmid (Qiagen EndoFree Plasmid Kit) using Lipofectamin 3000 (Invitrogen) according to manufacturer's recommendations. 48 h after transfection, cells were collected, resuspended in homogenization buffer (10 mM Tris-Maleate, 1 mM EDTA, 1 mM TCEP, pH 6.8) at a concentration of ∼25x10⁶ cells/ml of buffer, supplemented with protease inhibitors (Complete protease inhibitors, Roche), and homogenized on ice using a glass-teflon douncer (50-70 passages at 500 rpm). The cell nuclei and plasma membrane fractions were removed by centrifugation at 4,000g for 10 min at 4°C, and the supernatant containing the SR/ER vesicles subjected to centrifugation at ∼40,000 g (19,300 rpm; Beckman Optima MAX-XP MLS-50 rotor) for 30 min at 4°C. The pellet containing the crude microsomal fraction was resuspended in homogenization buffer (10 mM MOPS, 250 mM sucrose, 1 mM EDTA, 1 mM TCEP, pH 7.3), snap-frozen in liquid nitrogen, and stored at -80°C for subsequent single-channel bilayer analysis. Fusion of optoRyR containing microsomal vesicles into artificial bilayer membranes, single-channel current measurement and analysis were performed based on protocols described previously (Meli A.C. et al., Circ Res. 2011 109(3): 281-290).

### Results

To realize a light-controlled CICR approach the light-gated channel Channelrhodopsin-2 (ChR2) to achieve a higher Ca²⁺ permeability (CatChUP) was genetically engineered. Next, CatChUP was fused to the human RyR2 channel protein, the most abundant form in the brain and heart, to generate a polypeptide according to the present invention, namely, a novel optogenetic tool (OptoRyR2). OptoRyR2 is able to 1) release Ca²⁺ from the SR/ER upon light activation in close vicinity to the cytosolic Ca²⁺ sensor of RyR2, which 2) subsequently triggers physiological RyR2 activation through CICR. Because of the molecular crystal and cryoEM structures of RyR channel showed that the C-terminus is not accessible, we fused CatChUP together with EYFP to the N-terminus of the RyR2 protomer using an approximately 160 A long flexible linker chicken cysteine-rich protein (CRP1), which resulted in the successful development of OptoRyR2 **(****Fig. 1****).** Based on previously established molecular structures of all involved proteins, we estimated ∼100 A as the diffusion distance from the cytosolic site of CatChUP Ca²⁺ release to the Ca²⁺ sensor that activates the RyR2 channel (**Fig. 1****,** black line), providing a realistic model geometry for CICR.

Cloning of the plasmid DNA that encodes OptoRyR2 for stable expression in human embryonic kidney (HEK) 293 cells was successful and Western blot analysis showed that OptoRyR2 was, as predicted, ∼84 kDa larger than the native RyR2 protein **(****Fig. 2****).** Whole-cell patch clamp recordings excluded ectopic plasma membrane photocurrents in OptoRyR2-expressing HEK293 cells, further confirming correct CatChUP localization, exclusively in the ER organelle membranes **(****Fig. 3****).** Ca²⁺ imaging experiments using X-Rhod-1 as Ca²⁺ indicator showed fast Ca²⁺ release events in ∼80% of OptoRyR2 expressing HEK293 cells upon application of the RyR2 sensitizing agent caffeine (10 mM) but not in non-transfected wild-type HEK293 control cells, suggesting proper folding and function of OptoRyR2 **(****Fig. 4A****).** Importantly, blue-light illumination (1.4 mW/mm², 60 s) led to Ca²⁺ release in ∼20% of OptoRyR2-HEK293 cells, and with similar magnitudes compared to caffeine-induced Ca²⁺ release. The latter effect was specific for Ca²⁺ release via OptoRyR2 as it was not seen in non-transfected wild-type or human RyR2 expressing HEK293 cells, thus, excluding thermal heat effects or phototoxic effects as unspecific mechanisms. Detailed analysis of the light-induced Ca²⁺ release kinetics identified a biphasic response upon illumination **(****Fig. 4A****)** with an initial linear increase (slope 1: 5.1±1.4 x10⁻³ (F/Fo)/s) followed (after 49.0±3.3 s) by a second, much faster linear release (slope 2: 38.9±5.2 x10⁻³ (F/Fo)/s) until the peak concentration of [Ca²⁺] release was reached (1.48±0.04 F/Fo, all values n=57). Dual light pulse protocols showed similar slope values for the 2^{nd} light pulse (**Fig. 4B****,D**), however, application of the fast-acting drug Tetracaine (100 µM), which decreases RyR open probability by stabilizing a long-lived closed state, completely abolished the fast component of light-induced Ca²⁺ release (slope 2) without affecting the initial phase (slope 1) **(****Fig. 4C, E).**

Activity of OptoRyR2 on the single-channel level was examined in planar lipid bilayer system using a new protocol for isolation of OptoRyR2-containing microsomes. OptoRyR2-containing microsomes were added to the cytosolic (cis) solution of the bilayer chamber. After successful fusion and incorporation of OptoRyR2 channel into the bilayer, the physiological channel activation (CICR) was confirmed by addition of Ca²⁺ to the cytosolic (cis) side, which induced stochastic openings of OptoRyR2 (Fig. 5) similar to those of wild-type RyR2 channels. Importantly, OptoRyR2 channels were blocked by ryanodine, a high-affinity alkaloid blocker, which molecularly proves the RyR2 channel identity and its correct orientation following reconstitution in a lipid bilayer.

The cellular and single-channel data clearly show that the OptoRyR2 strategy allows for light-induced Ca²⁺ release from the SR/ER via CatChUP (slope 1) that eventually activates RyR2 opening through CICR (slope 2) due to the close spatial vicinity (100 A) of CatChUP and RyR2 engineered in the OptoRyR2 fusion protein. Specifically, we could already prove that OptoRyR2 is a valuable tool for screening of RyR modulating pharmacological substances such as the well-known RyR-blocker Tetracaine with a specific mode of action during the second phase of Ca²⁺ release via the RyR2-component (slope 2), but, as expected, not blocking Ca²⁺ release via CatChUP (slope 1). Potentially, OptoRyR2s could be a very important tool for high throughput screening to test therapeutic and toxic effects of commercial and novel class of drugs that modulate RyR channels.

Finally, the here developed strategy for light-induced generation of Ca²⁺ microdomains by fusion of optogenetic proteins (here CatChUP) with linkers of defined lengths could be a universal concept to control endogenous function of Ca²⁺ sensitive proteins (here RyR2) and to aid development of and screening for novel therapeutic drugs.

## Claims

1. A polypeptide comprising from the N-terminus to the C-terminus a channelrhodopsin (ChR) moiety, optionally a linker moiety, and a moiety of a Ca²⁺ sensitive protein.

2. The polypeptide according to claim 1 wherein the moiety of a Ca²⁺ sensitive protein is stemming from a Ca²⁺ sensitive transmembrane protein, in particular, wherein the moiety is stemming from a Ca²⁺ sensitive transmembrane protein located at the sarco/endoplasmatic reticulum Ca²⁺ store, and the linker moiety is present.

3. The polypeptide according to claim 2 wherein the moiety of a Ca²⁺ sensitive transmembrane protein is stemming from a moiety of a human inositol triphosphate receptor or a moiety of a ryanodine receptor (RyR), preferably, wherein the Ca²⁺ sensitive protein is a human RyR2 moiety, in particular of SEQ ID No. 1.

4. The polypeptide according to claim 2 wherein the moiety of a Ca²⁺ sensitive transmembrane moiety is stemming from the UNC-68 protein of *Caenorhabditis elegans.*

5. The polypeptide according to any one of the preceding claims wherein the channelrhodopsin moiety is stemming from *Chlamydomonas reinhardtii* or *Volvox carteri* or combinations thereof and the ryanodine receptor moiety is stemming from human proteins.

6. The polypeptide according to any one of the preceding claims wherein the channelrhodopsin moiety is channelrhodopsin-2 (ChR2) stemming from *Chlamydomonas reinhardtii,* preferably, wherein the sequence encoding ChR2 is the sequence of SEQ ID No. 2 or SEQ ID No.3.

7. The polypeptide according to any one of the preceding claims wherein the linker is selected to establish a distance of from 100 to 200 Angstrom between the ChR moiety and the Ca²⁺ sensitive protein moiety, like the RyR moiety when the said polypeptide is present in the cell membrane of a eukaryotic cell.

8. The polypeptide according to any one of the preceding claims wherein the linker is from chicken cysteine-rich protein (CRP1), preferably of SEQ ID No. 4.

9. A nucleic acid molecule comprising a nucleic acid sequence encoding the polypeptide according to any one of claims 1 to 8.

10. A nucleic acid construct comprising a nucleic acid encoding a polypeptide comprising a channelrhodopsin, in particular, a channelrhodopsin-2, optionally a linker as defined in any one of claims 1 to 8, and at the 3' end at least one exon of the DNA encoding moiety of a Ca²⁺ sensitive protein, like the RyR molecule, in particular, a RyR2 molecule.

11. A nucleic acid construct according to claim 10 suitable for homology directed integration into a host cell DNA, like by CRISP/CAS technology.

12. A vector containing a nucleic acid or a nucleic acid construct according to any one of claims 9 to 11.

13. Isolated cell, cell line or host cell containing a vector according to claim 15 or a nucleic acid according to any one of claims 9 to 11 expressing the polypeptide according to any one of claims 1 to 8.

14. *C. elegans* strains containing a vector according to claim 12, or a nucleic acid according to any one of claims 9 to 11 expressing the polypeptide according to any one of claims 1 to 8.

15. A method for determining the activity of candidate compounds on Ca²⁺ sensitive proteins, in particular, Ca²⁺ sensitive proteins as defined in any one of claims 1 to 8 comprising the step of
- Providing a cell, cell line or host cell according to claim 13 or *C. elegans according to claim 14;*
- Contacting the cell, cell line or host cell, or the *C. elegans* strain with the candidate compound;
- Determining activity of said candidate compound on the Ca²⁺ sensitive proteins, in particular, as defined in any one of claims 1 to 8, like on the Ca²⁺ release involving the Ca²⁺ sensitive proteins, preferably for determining therapeutic or toxic effects of candidate compounds based on Ca²⁺ release from intracellular stores of the cells.
